# EUROPEAN PATENT APPLICATION

(11) **EP 1 088 831 A1**
(43) Date of publication of application: **04.04.2001**
(21) Application number: 99926775.0
(22) Date of filing: 23.06.1999
(51) Int. Cl.: C07K 14/715, C07K 19/00, C12N 15/12, C12N 5/10, C12P 21/02, G01N 33/50, C07K 16/16, C07K 16/28, G01N 33/53, C12Q 1/68

(54) **NOVEL HEMOPOIETIN RECEPTOR PROTEINS**

(30) Priority: 24.06.1998 JP 21472098; 19.10.1998 JP 29740998
(71) Applicant: Chugai Research Institute for Molecular Medicine Inc., Niihari-gun, Ibaraki 300-4101 (JP)
(72) Inventor: NOMURA, Hitoshi, Chugai Res. Inst. for Molecular, Niikari-gun, Ibaraki 300-4101 (JP); MAEDA, Masatsugu, Chugai Res. Inst. for Molecular, Niikari-gun, Ibaraki 300-4101 (JP)
(74) Representative: VOSSIUS & PARTNER
(86) International application number: JP9903351
(87) International publication number: WO9967290

(57) **Abstract**

The present invention provides novel hemopoietin receptor proteins (proteins comprising the amino acid sequence of SEQ ID NOs: 1, 3, 5, 7, 19, or 21), proteins comprising a modified amino acid sequence of the amino acid sequence of the above protein in which one or more amino acids have been deleted, added, and/or replaced with another amino acid, genes encoding these proteins, methods of producing the proteins, as well as uses of these proteins and genes.

SEQUENCE LISTING

<110> CHUGAI RESEARCH INSTITUTE FOR MOLECULAR MEDICINE, INC.

<120> NOVEL HEMOPOIETIN RECEPTOR PROTEINS

<130> C2-004PCT

<150> JP 10-214720
<151> 1998-6-24

<160> 30

<170> PatentIn version 2.0

<210> 1
<211> 361
<212> PRT
<213> Homo sapiens

<400> 1
Met Pro Arg Gly Trp Ala Ala Pro Leu Leu Leu
1               5                   10

Leu Leu Leu Gln Gly Gly Trp Gly Cys Pro Asp Leu Val Cys Tyr Thr
            15                  20                  25

Asp Tyr Leu Gln Thr Val Ile Cys Ile Leu Glu Met Trp Asn Leu His
        30                  35                  40

Pro Ser Thr Leu Thr Leu Thr Trp Gln Asp Gln Tyr Glu Glu Leu Lys
45 50 55.

Asp Glu Ala Thr Ser Cys Ser Leu His Arg Ser Ala His Asn Ala Thr
60 65 70 75

His Ala Thr Tyr Thr Cys His Met Asp Val Phe His Phe Met Ala Asp
80 85 90

Asp Ile Phe Ser Val Asn Ile Thr Asp Gln Ser Gly Asn Tyr Ser Gln

95 100 105

Glu Cys Gly Ser Phe Leu Leu Ala Glu Ser Ile Lys Pro Ala Pro Pro
110 115 120

Phe Asn Val Thr Val Thr Phe Ser Gly Gln Tyr Asn Ile Ser Trp Arg
125 130 135

Ser Asp Tyr Glu Asp Pro Ala Phe Tyr Met Leu Lys Gly Lys Leu Gln
140 145 150 155

Tyr Glu Leu Gln Tyr Arg Asn Arg Gly Asp Pro Trp Ala Val Ser Pro
160 165 170

Arg Arg Lys Leu Ile Ser Val Asp Ser Arg Ser Val Ser Leu Leu Pro
175 180 185

Leu Glu Phe Arg Lys Asp Ser Ser Tyr Glu Leu Gln Val Arg Ala Gly
190 195 200

Pro Met Pro Gly Ser Ser Tyr Gln Gly Thr Trp Ser Glu Trp Ser Asp
205 210 215

Pro Val Ile Phe Gln Thr Gln Ser Glu Glu Leu Lys Glu Gly Trp Asn
220 225 230 235

Pro His Leu Leu Leu Leu Leu Leu Leu Val Ile Val Phe Ile Pro Ala
240 245 250

Phe Trp Ser Leu Lys Thr His Pro Leu Trp Arg Leu Trp Lys Lys Ile
255 260 265

Trp Ala Val Pro Ser Pro Glu Arg Phe Phe Met Pro Leu Tyr Lys Gly
270 275 280

Cys Ser Gly Asp Phe Lys Lys Trp Val Gly Ala Pro Phe Thr Gly Ser
285 290 295

Ser Leu Glu Leu Gly Pro Trp Ser Pro Glu Val Pro Ser Thr Leu Glu
300 305 310 315

Val Tyr Ser Cys His Pro Pro Ser Ser Pro Val Glu Cys Asp Phe Thr
                320                 325                 330

Ser Pro Gly Asp Glu Gly Pro Pro Arg Ser Tyr Leu Arg Gln Trp Val
            335                 340                 345

Val Ile Pro Pro Pro Leu Ser Ser Pro Gly Pro Gln Ala Ser
        350                 355                 360

<210> 2
<211> 1884
<212> DNA
<213> Homo sapiens

<220>
<221> CDS
<222> (441)..(1523)

<400> 2
   ggcagccagc ggcctcagac agacccactg gcgtctctct gctgagtgac cgtaagctcg      60
   gcgtctggcc ctctgcctgc ctctccctga gtgtggctga cagccacgca gctgtgtctg     120
   tctgtctgcg gcccgtgcat ccctgctgcg gccgcctggt accttccttg ccgtctcttt     180
   cctctgtctg ctgctctgtg ggacacctgc ctggaggccc agctgcccgt catcagagtg     240
   acaggtctta tgacagcctg attggtgact cgggctgggt gtggattctc accccaggcc     300
   tctgcctgct ttctcagacc ctcatctgtc acccccacgc tgaacccagc tgccaccccc     360
   agaagcccat cagactgccc ccagcacacg gaatggattt ctgagaaaga agccgaaaca     420
   gaaggcccgt gggagtcagc atg ccg cgt ggc tgg gcc gcc ccc ttg ctc ctg     473
                         Met Pro Arg Gly Trp Ala Ala Pro Leu Leu Leu
                         1               5                   10
   ctg ctg ctc cag gga ggc tgg ggc tgc ccc gac ctc gtc tgc tac acc     521
   Leu Leu Leu Gln Gly Gly Trp Gly Cys Pro Asp Leu Val Cys Tyr Thr
           15                  20                  25
   gat tac ctc cag acg gtc atc tgc atc ctg gaa atg tgg aac ctc cac 569
   Asp Tyr Leu Gln Thr Val Ile Cys Ile Leu Glu Met Trp Asn Leu His
   30 35 40
   ccc agc acg ctc acc ctt acc tgg caa gac cag tat gaa gag ctg aag 617
   Pro Ser Thr Leu Thr Leu Thr Trp Gln Asp Gln Tyr Glu Glu Leu Lys
   45 50 55
   gac gag gcc acc tcc tgc agc ctc cac agg tcg gcc cac aat gcc acg 665
   Asp Glu Ala Thr Ser Cys Ser Leu His Arg Ser Ala His Asn Ala Thr
   60 65 70 75
   cat gcc acc tac acc tgc cac atg gat gta ttc cac ttc atg gcc gac 713
   His Ala Thr Tyr Thr Cys His Met Asp Val Phe His Phe Met Ala Asp
   80 85 90
   gac att ttc agt gtc aac atc aca gac cag tct ggc aac tac tcc cag 761
   Asp Ile Phe Ser Val Asn Ile Thr Asp Gln Ser Gly Asn Tyr Ser Gln
   95 100 105
   gag tgt ggc agc ttt ctc ctg gct gag agc atc aag ccg gct ccc cct 809
   Glu Cys Gly Ser Phe Leu Leu Ala Glu Ser Ile Lys Pro Ala Pro Pro
   110 115 120
   ttc aac gtg act gtg acc ttc tca gga cag tat aat atc tcc tgg cgc 857
   Phe Asn Val Thr Val Thr Phe Ser Gly Gln Tyr Asn Ile Ser Trp Arg
   125 130 135
   tca gat tac gaa gac cct gcc ttc tac atg ctg aag ggc aag ctt cag 905
   Ser Asp Tyr Glu Asp Pro Ala Phe Tyr Met Leu Lys Gly Lys Leu Gln
   140 145 150 155
   tat gag ctg cag tac agg aac cgg gga gac ccc tgg gct gtg agt ccg 953
   Tyr Glu Leu Gln Tyr Arg Asn Arg Gly Asp Pro Trp Ala Val Ser Pro
   160 165 170
   agg aga aag ctg atc tca gtg gac tca aga agt gtc tcc ctc ctc ccc 1001
   Arg Arg Lys Leu Ile Ser Val Asp Ser Arg Ser Val Ser Leu Leu Pro
   175 180 185
   ctg gag ttc cgc aaa gac tcg agc tat gag ctg cag gtg cgg gca ggg 1049
   Leu Glu Phe Arg Lys Asp Ser Ser Tyr Glu Leu Gln Val Arg Ala Gly
   190 195 200
   ccc atg cct ggc tcc tcc tac cag ggg acc tgg agt gaa tgg agt gac 1097
   Pro Met Pro Gly Ser Ser Tyr Gln Gly Thr Trp Ser Glu Trp Ser Asp
   205 210 215
   ccg gtc atc ttt cag acc cag tca gag gag tta aag gaa ggc tgg aac 1145
   Pro Val Ile Phe Gln Thr Gln Ser Glu Glu Leu Lys Glu Gly Trp Asn
   220 225 230 235
   cct cac ctg ctg ctt ctc ctc ctg ctt gtc ata gtc ttc att cct gcc 1193
   Pro His Leu Leu Leu Leu Leu Leu Leu Val Ile Val Phe Ile Pro Ala
   240 245 250
   ttc tgg agc ctg aag acc cat cca ttg tgg agg cta tgg aag aag ata 1241
   Phe Trp Ser Leu Lys Thr His Pro Leu Trp Arg Leu Trp Lys Lys Ile
   255 260 265
   tgg gcc gtc ccc agc cct gag cgg ttc ttc atg ccc ctg tac aag ggc 1289
   Trp Ala Val Pro Ser Pro Glu Arg Phe Phe Met Pro Leu Tyr Lys Gly
   270 275 280
   tgc agc gga gac ttc aag aaa tgg gtg ggt gca ccc ttc act ggc tcc 1337
   Cys Ser Gly Asp Phe Lys Lys Trp Val Gly Ala Pro Phe Thr Gly Ser
   285 290 295
   agc ctg gag ctg gga ccc tgg agc cca gag gtg ccc tcc acc ctg gag 1385
   Ser Leu Glu Leu Gly Pro Trp Ser Pro Glu Val Pro Ser Thr Leu Glu
   300 305 310 315
   gtg tac agc tgc cac cca ccc agc agc cct gtg gag tgt gac ttc acc 1433
   Val Tyr Ser Cys His Pro Pro Ser Ser Pro Val Glu Cys Asp Phe Thr
   320 325 330
   agc ccc ggg gac gaa gga ccc ccc cgg agc tac ctc cgc cag tgg gtg 1481
   Ser Pro Gly Asp Glu Gly Pro Pro Arg Ser Tyr Leu Arg Gln Trp Val
   335 340 345
   gtc att cct ccg cca ctt tcg agc cct gga ccc cag gcc agc taa 1526
   Val Ile Pro Pro Pro Leu Ser Ser Pro Gly Pro Gln Ala Ser
           350                 355                 360
   tgaggctgac tggatgtcca gagctggcca ggccactggg ccctgagcca gagacaaggt    1586
   cacctgggct gtgatgtgaa gacacctgca gcctttggtc tcctggatgg gcctttgagc    1646
   ctgatgttta cagtgtctgt gtgtgtgtgc atatgtgtgt gtgtgcatat gcatgtgtgt    1706
   gtgtgtgtgt gtcttaggtg cgcagtggca tgtccacgtg tgtgtgattg cacgtgcctg    1766
   tgggcctggg ataatgccca tggtactcca tgcattcacc tgccctgtgc atgtctggac    1826
   tcacggagct cacccatgtg cacaagtgtg cacagtaaac gtgtttgtgg tcaacaga      1884

<210> 3
<211> 144
<212> PRT
<213> Homo sapiens

<400> 3
                        Met Pro Arg Gly Trp Ala Ala Pro Leu Leu Leu
                         1               5                  10

Leu Leu Leu Gln Gly Gly Trp Gly Cys Pro Asp Leu Val Cys Tyr Thr
            15                  20                  25

Asp Tyr Leu Gln Thr Val Ile Cys Ile Leu Glu Met Trp Asn Leu His
        30                  35                  40

Pro Ser Thr Leu Thr Leu Thr Trp Gln Asp Gln Tyr Glu Glu Leu Lys
    45                  50                  55

Asp Glu Ala Thr Ser Cys Ser Leu His Arg Ser Ala His Asn Ala Thr
60                  65                  70                  75

His Ala Thr Tyr Thr Cys His Met Asp Val Phe His Phe Met Ala Asp
                80                  85                  90

Asp Ile Phe Ser Val Asn Ile Thr Asp Gln Ser Gly Asn Tyr Ser Gln

            95                  100                 105

Glu Cys Gly Ser Phe Leu Leu Ala Glu Ser Lys Ser Glu Glu Lys Ala
        110                 115                 120

Asp Leu Ser Gly Leu Lys Lys Cys Leu Pro Pro Pro Pro Gly Val Pro
    125                 130                 135

Gln Arg Leu Glu Leu
140

<210> 4
<211> 1729
<212> DNA
<213> Homo sapiens

<220>
<221> CDS
<222> (441)..(872)

<400> 4
ggcagccagc ggcctcagac agacccactg gcgtctctct gctgagtgac cgtaagctcg 60

gcgtctggcc ctctgcctgc ctctccctga gtgtggctga cagccacgca gctgtgtctg 120

tctgtctgcg gcccgtgcat ccctgctgcg gccgcctggt accttccttg ccgtctcttt 180

cctctgtctg ctgctctgtg ggacacctgc ctggaggccc agctgcccgt catcagagtg 240

acaggtctta tgacagcctg attggtgact cgggctgggt gtggattctc accccaggcc 300

tctgcctgct ttctcagacc ctcatctgtc acccccacgc tgaacccagc tgccaccccc 360

agaagcccat cagactgccc ccagcacacg gaatggattt ctgagaaaga agccgaaaca 420

gaaggcccgt gggagtcagc atg ccg cgt ggc tgg gcc gcc ccc ttg ctc ctg 473
Met Pro Arg Gly Trp Ala Ala Pro Leu Leu Leu
1               5                   10

ctg ctg ctc cag gga ggc tgg ggc tgc ccc gac ctc gtc tgc tac acc 521

Leu Leu Leu Gln Gly Gly Trp Gly Cys Pro Asp Leu Val Cys Tyr Thr
            15                  20                  25

gat tac ctc cag acg gtc atc tgc atc ctg gaa atg tgg aac ctc cac 569
Asp Tyr Leu Gln Thr Val Ile Cys Ile Leu Glu Met Trp Asn Leu His
        30                  35                  40

ccc agc acg ctc acc ctt acc tgg caa gac cag tat gaa gag ctg aag 617
Pro Ser Thr Leu Thr Leu Thr Trp Gln Asp Gln Tyr Glu Glu Leu Lys
    45                  50                  55

GAC GAG GCC ACC TCC TGC AGC CTC CAC AGG TCG GCC CAC AAT GCC ACG 665
Asp Glu Ala Thr Ser Cys Ser Leu His Arg Ser Ala His Asn Ala Thr
60                  65                  70                  75

CAT GCC ACC TAC ACC TGC CAC ATG GAT GTA TTC CAC TTC ATG GCC GAC 713
His Ala Thr Tyr Thr Cys His Met Asp Val Phe His Phe Met Ala Asp
                80                  85                  90

GAC ATT TTC AGT GTC AAC ATC ACA GAC CAG TCT GGC AAC TAC TCC CAG 761
Asp Ile Phe Ser Val Asn Ile Thr Asp Gln Ser Gly Asn Tyr Ser Gln
            95                  100                 105

GAG TGT GGC AGC TTT CTC CTG GCT GAG AGC AAG TCC GAG GAG AAA GCT 809
Glu Cys Gly Ser Phe Leu Leu Ala Glu Ser Lys Ser Glu Glu Lys Ala
        110                 115                 120

gat ctc agt gga ctc aag aag tgt ctc cct cct ccc cct gga gtt ccg 857
Asp Leu Ser Gly Leu Lys Lys Cys Leu Pro Pro Pro Pro Gly Val Pro
    125                 130                 135

caa aga ctc gag cta tgagctgcag gtgcgggcag ggcccatgcc tggctcctcc 912
Gln Arg Leu Glu Leu
140

taccagggga cctggagtga atggagtgac ccggtcatct ttcagaccca gtcagaggag 972

ttaaaggaag gctggaaccc tcacctgctg cttctcctcc tgcttgtcat agtcttcatt 1032

cctgccttct ggagcctgaa gacccatcca ttgtggaggc tatggaagaa gatatgggcc 1092

gtccccagcc ctgagcggtt cttcatgccc ctgtacaagg gctgcagcgg agacttcaag    1152

aaatgggtgg gtgcaccctt cactggctcc agcctggagc tgggaccctg gagcccagag    1212

gtgccctcca ccctggaggt gtacagctgc cacccaccca gcagccctgt ggagtgtgac    1272

ttcaccagcc ccggggacga aggacccccc cggagctacc tccgccagtg ggtggtcatt    1332

cctccgccac tttcgagccc tggaccccag gccagctaat gaggctgact ggatgtccag    1392

agctggccag gccactgggc cctgagccag agacaaggtc acctgggctg tgatgtgaag    1452

acacctgcag cctttggtct cctggatggg cctttgagcc tgatgtttac agtgtctgtg    1512

tgtgtgtgca tatgtgtgtg tgtgcatatg catgtgtgtg tgtgtgtgtg tcttaggtgc    1572

gcagtggcat gtccacgtgt gtgtgattgc acgtgcctgt gggcctggga taatgcccat    1632

ggtactccat gcattcacct gccctgtgca tgtctggact cacggagctc acccatgtgc    1692

acaagtgtgc acagtaaacg tgtttgtggt caacaga                             1729

<210> 5
<211> 237
<212> PRT
<213> Homo sapiens

<400> 5
Met Pro Arg Met Pro Pro Thr Pro Ala Thr Trp Met Tyr Ser Thr Ser
1               5                   10                  15

Trp Pro Thr Thr Phe Ser Val Ser Thr Ser Gln Thr Ser Leu Ala Thr
            20                  25                  30

Thr Pro Arg Ser Val Ala Ala Phe Ser Trp Leu Arg Ala Ser Pro Arg
        35                  40                  45

Arg Lys Leu Ile Ser Val Asp Ser Arg Ser Val Ser Leu Leu Pro Leu
    50                  55                  60

Glu Phe Arg Lys Asp Ser Ser Tyr Glu Leu Gln Val Arg Ala Gly Pro
65                  70                  75                  80

Met Pro Gly Ser Ser Tyr Gln Gly Thr Trp Ser Glu Trp Ser Asp Pro
                85                  90                  95

Val Ile Phe Gln Thr Gln Ser Glu Glu Leu Lys Glu Gly Trp Asn Pro
            100                 105                 110

His Leu Leu Leu Leu Leu Leu Leu Val Ile Val Phe Ile Pro Ala Phe
        115                 120                 125

Trp Ser Leu Lys Thr His Pro Leu Trp Arg Leu Trp Lys Lys Ile Trp
    130                 135                 140

Ala Val Pro Ser Pro Glu Arg Phe Phe Met Pro Leu Tyr Lys Gly Cys
145                 150                 155                 160

Ser Gly Asp Phe Lys Lys Trp Val Gly Ala Pro Phe Thr Gly Ser Ser
                165                 170                 175

Leu Glu Leu Gly Pro Trp Ser Pro Glu Val Pro Ser Thr Leu Glu Val
            180                 185                 190

Tyr Ser Cys His Pro Pro Ser Ser Pro Val Glu Cys Asp Phe Thr Ser
        195                 200                 205

Pro Gly Asp Glu Gly Pro Pro Arg Ser Tyr Leu Arg Gln Trp Val Val
    210                 215                 220

Ile Pro Pro Pro Leu Ser Ser Pro Gly Pro Gln Ala Ser
225                 230                 235

<210> 6
<211> 1729
<212> DNA
<213> Homo sapiens

<220>
<221> CDS

<222> (659)..(1368)

<400> 6
ggcagccagc ggcctcagac agacccactg gcgtctctct gctgagtgac cgtaagctcg 60

gcgtctggcc ctctgcctgc ctctccctga gtgtggctga cagccacgca gctgtgtctg 120

tctgtctgcg gcccgtgcat ccctgctgcg gccgcctggt accttccttg ccgtctcttt 180

cctctgtctg ctgctctgtg ggacacctgc ctggaggccc agctgcccgt catcagagtg 240

acaggtctta tgacagcctg attggtgact cgggctgggt gtggattctc accccaggcc 300

tctgcctgct ttctcagacc ctcatctgtc acccccacgc tgaacccagc tgccaccccc 360

agaagcccat cagactgccc ccagcacacg gaatggattt ctgagaaaga agccgaaaca 420

gaaggcccgt gggagtcagc atgccgcgtg gctgggccgc ccccttgctc ctgctgctgc 480

tccagggagg ctggggctgc cccgacctcg tctgctacac cgattacctc cagacggtca 540

tctgcatcct ggaaatgtgg aacctccacc ccagcacgct cacccttacc tggcaagacc 600

agtatgaaga gctgaaggac gaggccacct cctgcagcct ccacaggtcg gcccacaa 658

atg cca cgc atg cca cct aca cct gcc aca tgg atg tat tcc act tca 705
Met Pro Arg Met Pro Pro Thr Pro Ala Thr Trp Met Tyr Ser Thr Ser
1               5                   10                  15

tgg ccg acg aca ttt tca gtg tca aca tca cag acc agt ctg gca act 753
Trp Pro Thr Thr Phe Ser Val Ser Thr Ser Gln Thr Ser Leu Ala Thr
            20                  25                  30

act ccc agg agt gtg gca gct ttc tcc tgg ctg aga gca agt ccg agg 801
Thr Pro Arg Ser Val Ala Ala Phe Ser Trp Leu Arg Ala Ser Pro Arg
        35                  40                  45

aga aag ctg atc tca gtg gac tca aga agt gtc tcc ctc ctc ccc ctg 849
Arg Lys Leu Ile Ser Val Asp Ser Arg Ser Val Ser Leu Leu Pro Leu
    50                  55                  60

gag ttc cgc aaa gac tcg agc tat gag ctg cag gtg cgg gca ggg ccc 897
Glu Phe Arg Lys Asp Ser Ser Tyr Glu Leu Gln Val Arg Ala Gly Pro
65                  70                  75                  80

atg cct ggc tcc tcc tac cag ggg acc tgg agt gaa tgg agt gac ccg 945
Met Pro Gly Ser Ser Tyr Gln Gly Thr Trp Ser Glu Trp Ser Asp Pro
                85                  90                  95

gtc atc ttt cag acc cag tca gag gag tta aag gaa ggc tgg aac cct 993
Val Ile Phe Gln Thr Gln Ser Glu Glu Leu Lys Glu Gly Trp Asn Pro
            100                 105                 110

cac ctg ctg ctt ctc ctc ctg ctt gtc ata gtc ttc att cct gcc ttc 1041
His Leu Leu Leu Leu Leu Leu Leu Val Ile Val Phe Ile Pro Ala Phe
        115                 120                 125

tgg agc ctg aag acc cat cca ttg tgg agg cta tgg aag aag ata tgg 1089
Trp Ser Leu Lys Thr His Pro Leu Trp Arg Leu Trp Lys Lys Ile Trp
    130                 135                 140

gcc gtc ccc agc cct gag cgg ttc ttc atg ccc ctg tac aag ggc tgc 1137
Ala Val Pro Ser Pro Glu Arg Phe Phe Met Pro Leu Tyr Lys Gly Cys
145                 150                 155                 160

agc gga gac ttc aag aaa tgg gtg ggt gca ccc ttc act ggc tcc agc 1185
Ser Gly Asp Phe Lys Lys Trp Val Gly Ala Pro Phe Thr Gly Ser Ser
                165                 170                 175

ctg gag ctg gga ccc tgg agc cca gag gtg ccc tcc acc ctg gag gtg 1233
Leu Glu Leu Gly Pro Trp Ser Pro Glu Val Pro Ser Thr Leu Glu Val
            180                 185                 190

tac agc tgc cac cca ccc agc agc cct gtg gag tgt gac ttc acc agc 1281
Tyr Ser Cys His Pro Pro Ser Ser Pro Val Glu Cys Asp Phe Thr Ser
        195                 200                 205

ccc ggg gac gaa gga ccc ccc cgg agc tac ctc cgc cag tgg gtg gtc 1329
Pro Gly Asp Glu Gly Pro Pro Arg Ser Tyr Leu Arg Gln Trp Val Val
    210                 215                 220

att cct ccg cca ctt tcg agc cct gga ccc cag gcc agc taatgaggct 1378

Ile Pro Pro Pro Leu Ser Ser Pro Gly Pro Gln Ala Ser
225                 230                 235

gactggatgt ccagagctgg ccaggccact gggccctgag ccagagacaa ggtcacctgg    1438

gctgtgatgt gaagacacct gcagcctttg gtctcctgga tgggcctttg agcctgatgt    1498

ttacagtgtc tgtgtgtgtg tgcatatgtg tgtgtgtgca tatgcatgtg tgtgtgtgtg    1558

tgtgtcttag gtgcgcagtg gcatgtccac gtgtgtgtga ttgcacgtgc ctgtgggcct    1618

gggataatgc ccatggtact ccatgcattc acctgccctg tgcatgtctg gactcacgga    1678

gctcacccat gtgcacaagt gtgcacagta aacgtgtttg tggtcaacaga              1729

<210> 7
<211> 538
<212> PRT
<213> Homo sapiens

<400> 7
                    Met Pro Arg Gly Trp Ala Ala Pro Leu Leu Leu
                     1               5                  10

Leu Leu Leu Gln Gly Gly Trp Gly Cys Pro Asp Leu Val Cys Tyr Thr
            15                  20                  25

Asp Tyr Leu Gln Thr Val Ile Cys Ile Leu Glu Met Trp Asn Leu His
        30                  35                  40

Pro Ser Thr Leu Thr Leu Thr Trp Gln Asp Gln Tyr Glu Glu Leu Lys
    45                  50                  55

Asp Glu Ala Thr Ser Cys Ser Leu His Arg Ser Ala His Asn Ala Thr
60                  65                  70                  75

His Ala Thr Tyr Thr Cys His Met Asp Val Phe His Phe Met Ala Asp
                80                  85                  90

Asp Ile Phe Ser Val Asn Ile Thr Asp Gln Ser Gly Asn Tyr Ser Gln

            95                  100                 105

Glu Cys Gly Ser Phe Leu Leu Ala Glu Ser Ile Lys Pro Ala Pro Pro
        110                 115                 120

Phe Asn Val Thr Val Thr Phe Ser Gly Gln Tyr Asn Ile Ser Trp Arg
    125                 130                 135

Ser Asp Tyr Glu Asp Pro Ala Phe Tyr Met Leu Lys Gly Lys Leu Gln
140                 145                 150                 155

Tyr Glu Leu Gln Tyr Arg Asn Arg Gly Asp Pro Trp Ala Val Ser Pro
                160                 165                 170

Arg Arg Lys Leu Ile Ser Val Asp Ser Arg Ser Val Ser Leu Leu Pro
            175                 180                 185

Leu Glu Phe Arg Lys Asp Ser Ser Tyr Glu Leu Gln Val Arg Ala Gly
        190                 195                 200

Pro Met Pro Gly Ser Ser Tyr Gln Gly Thr Trp Ser Glu Trp Ser Asp
    205                 210                 215

Pro Val Ile Phe Gln Thr Gln Ser Glu Glu Leu Lys Glu Gly Trp Asn
220                 225                 230                 235

Pro His Leu Leu Leu Leu Leu Leu Leu Val Ile Val Phe Ile Pro Ala
                240                 245                 250

Phe Trp Ser Leu Lys Thr His Pro Leu Trp Arg Leu Trp Lys Lys Ile
            255                 260                 265

Trp Ala Val Pro Ser Pro Glu Arg Phe Phe Met Pro Leu Tyr Lys Gly
        270                 275                 280

Cys Ser Gly Asp Phe Lys Lys Trp Val Gly Ala Pro Phe Thr Gly Ser
    285                 290                 295

Ser Leu Glu Leu Gly Pro Trp Ser Pro Glu Val Pro Ser Thr Leu Glu
300                 305                 310                 315

Val Tyr Ser Cys His Pro Pro Arg Ser Pro Ala Lys Arg Leu Gln Leu
                320                 325                 330
Thr Glu Leu Gln Glu Pro Ala Glu Leu Val Glu Ser Asp Gly Val Pro
            335                 340                 345
Lys Pro Ser Phe Trp Pro Thr Ala Gln Asn Ser Gly Gly Ser Ala Tyr
        350                 355                 360
Ser Glu Glu Arg Asp Arg Pro Tyr Gly Leu Val Ser Ile Asp Thr Val
    365                 370                 375
Thr Val Leu Asp Ala Glu Gly Pro Cys Thr Trp Pro Cys Ser Cys Glu
380                 385                 390                 395
Asp Asp Gly Tyr Pro Ala Leu Asp Leu Asp Ala Gly Leu Glu Pro Ser
                400                 405                 410
Pro Gly Leu Glu Asp Pro Leu Leu Asp Ala Gly Thr Thr Val Leu Ser
            415                 420                 425
Cys Gly Cys Val Ser Ala Gly Ser Pro Gly Leu Gly Gly Pro Leu Gly
        430                 435                 440
Ser Leu Leu Asp Arg Leu Lys Pro Pro Leu Ala Asp Gly Glu Asp Trp
    445                 450                 455
Ala Gly Gly Leu Pro Trp Gly Gly Arg Ser Pro Gly Gly Val Ser Glu
460                 465                 470                 475
Ser Glu Ala Gly Ser Pro Leu Ala Gly Leu Asp Met Asp Thr Phe Asp
                480                 485                 490
Ser Gly Phe Val Gly Ser Asp Cys Ser Ser Pro Val Glu Cys Asp Phe
            495                 500                 505
Thr Ser Pro Gly Asp Glu Gly Pro Pro Arg Ser Tyr Leu Arg Gln Trp
        510                 515                 520
Val Val Ile Pro Pro Pro Leu Ser Ser Pro Gly Pro Gln Ala Ser
    525                 530                 535

<210> 8
<211> 2415
<212> DNA
<213> Homo sapiens

<220>
<221> CDS
<222> (441)..(2054)

<400> 8
ggcagccagc ggcctcagac agacccactg gcgtctctct gctgagtgac cgtaagctcg      60

gcgtctggcc ctctgcctgc ctctccctga gtgtggctga cagccacgca gctgtgtctg     120

tctgtctgcg gcccgtgcat ccctgctgcg gccgcctggt accttccttg ccgtctcttt     180

cctctgtctg ctgctctgtg ggacacctgc ctggaggccc agctgcccgt catcagagtg     240

acaggtctta tgacagcctg attggtgact cgggctgggt gtggattctc accccaggcc     300

tctgcctgct ttctcagacc ctcatctgtc acccccacgc tgaacccagc tgccaccccc     360

agaagcccat cagactgccc ccagcacacg gaatggattt ctgagaaaga agccgaaaca     420

gaaggcccgt gggagtcagc atg ccg cgt ggc tgg gcc gcc ccc ttg ctc ctg     473
                      Met Pro Arg Gly Trp Ala Ala Pro Leu Leu Leu
                      1               5                   10

ctg ctg ctc cag gga ggc tgg ggc tgc ccc gac ctc gtc tgc tac acc     521
Leu Leu Leu Gln Gly Gly Trp Gly Cys Pro Asp Leu Val Cys Tyr Thr
            15                  20                  25

gat tac ctc cag acg gtc atc tgc atc ctg gaa atg tgg aac ctc cac     569
Asp Tyr Leu Gln Thr Val Ile Cys Ile Leu Glu Met Trp Asn Leu His
        30                  35                  40

ccc agc acg ctc acc ctt acc tgg caa gac cag tat gaa gag ctg aag     617
Pro Ser Thr Leu Thr Leu Thr Trp Gln Asp Gln Tyr Glu Glu Leu Lys
    45                  50                  55

gac gag gcc acc tcc tgc agc ctc cac agg tcg gcc cac aat gcc acg 665
Asp Glu Ala Thr Ser Cys Ser Leu His Arg Ser Ala His Asn Ala Thr
60                  65                  70                  75

cat gcc acc tac acc tgc cac atg gat gta ttc cac ttc atg gcc gac 713
His Ala Thr Tyr Thr Cys His Met Asp Val Phe His Phe Met Ala Asp
                80                  85                  90

gac att ttc agt gtc aac atc aca gac cag tct ggc aac tac tcc cag 761
Asp Ile Phe Ser Val Asn Ile Thr Asp Gln Ser Gly Asn Tyr Ser Gln
            95                  100                 105

gag tgt ggc agc ttt ctc ctg gct gag agc atc aag ccg gct ccc cct 809
Glu Cys Gly Ser Phe Leu Leu Ala Glu Ser Ile Lys Pro Ala Pro Pro
        110                 115                 120

ttc aac gtg act gtg acc ttc tca gga cag tat aat atc tcc tgg cgc 857
Phe Asn Val Thr Val Thr Phe Ser Gly Gln Tyr Asn Ile Ser Trp Arg
    125                 130                 135

tca gat tac gaa gac cct gcc ttc tac atg ctg aag ggc aag ctt cag 905
Ser Asp Tyr Glu Asp Pro Ala Phe Tyr Met Leu Lys Gly Lys Leu Gln
140                 145                 150                 155

tat gag ctg cag tac agg aac cgg gga gac ccc tgg gct gtg agt ccg 953
Tyr Glu Leu Gln Tyr Arg Asn Arg Gly Asp Pro Trp Ala Val Ser Pro
                160                 165                 170

agg aga aag ctg atc tca gtg gac tca aga agt gtc tcc ctc ctc ccc 1001
Arg Arg Lys Leu Ile Ser Val Asp Ser Arg Ser Val Ser Leu Leu Pro
            175                 180                 185

ctg gag ttc cgc aaa gac tcg agc tat gag ctg cag gtg cgg gca ggg 1049
Leu Glu Phe Arg Lys Asp Ser Ser Tyr Glu Leu Gln Val Arg Ala Gly
        190                 195                 200

ccc atg cct ggc tcc tcc tac cag ggg acc tgg agt gaa tgg agt gac 1097
Pro Met Pro Gly Ser Ser Tyr Gln Gly Thr Trp Ser Glu Trp Ser Asp
    205                 210                 215

ccg gtc atc ttt cag acc cag tca gag gag tta aag gaa ggc tgg aac 1145
Pro Val Ile Phe Gln Thr Gln Ser Glu Glu Leu Lys Glu Gly Trp Asn
220                 225                 230                 235

cct cac ctg ctg ctt ctc ctc ctg ctt gtc ata gtc ttc att cct gcc 1193
Pro His Leu Leu Leu Leu Leu Leu Leu Val Ile Val Phe Ile Pro Ala
                240                 245                 250

ttc tgg agc ctg aag acc cat cca ttg tgg agg cta tgg aag aag ata 1241
Phe Trp Ser Leu Lys Thr His Pro Leu Trp Arg Leu Trp Lys Lys Ile
            255                 260                 265

tgg gcc gtc ccc agc cct gag cgg ttc ttc atg ccc ctg tac aag ggc 1289
Trp Ala Val Pro Ser Pro Glu Arg Phe Phe Met Pro Leu Tyr Lys Gly
        270                 275                 280

tgc agc gga gac ttc aag aaa tgg gtg ggt gca ccc ttc act ggc tcc 1337
Cys Ser Gly Asp Phe Lys Lys Trp Val Gly Ala Pro Phe Thr Gly Ser
    285                 290                 295

agc ctg gag ctg gga ccc tgg agc cca gag gtg ccc tcc acc ctg gag 1385
Ser Leu Glu Leu Gly Pro Trp Ser Pro Glu Val Pro Ser Thr Leu Glu
300                 305                 310                 315

gtg tac agc tgc cac cca cca cgg agc ccg gcc aag agg ctg cag ctc 1433
Val Tyr Ser Cys His Pro Pro Arg Ser Pro Ala Lys Arg Leu Gln Leu
                320                 325                 330

acg gag cta caa gaa cca gca gag ctg gtg gag tct gac ggt gtg ccc 1481
Thr Glu Leu Gln Glu Pro Ala Glu Leu Val Glu Ser Asp Gly Val Pro
            335                 340                 345

aag ccc agc ttc tgg ccg aca gcc cag aac tcg ggg ggc tca gct tac 1529
Lys Pro Ser Phe Trp Pro Thr Ala Gln Asn Ser Gly Gly Ser Ala Tyr
        350                 355                 360

agt gag gag agg gat cgg cca tac ggc ctg gtg tcc att gac aca gtg 1577
Ser Glu Glu Arg Asp Arg Pro Tyr Gly Leu Val Ser Ile Asp Thr Val
    365                 370                 375

act gtg cta gat gca gag ggg cca tgc acc tgg ccc tgc agc tgt gag 1625

Thr Val Leu Asp Ala Glu Gly Pro Cys Thr Trp Pro Cys Ser Cys Glu
380                 385                 390                 395

gat gac ggc tac cca gcc ctg gac ctg gat gct ggc ctg gag ccc agc     1673
Asp Asp Gly Tyr Pro Ala Leu Asp Leu Asp Ala Gly Leu Glu Pro Ser
                400                 405                 410

cca ggc cta gag gac cca ctc ttg gat gca ggg acc aca gtc ctg tcc     1721
Pro Gly Leu Glu Asp Pro Leu Leu Asp Ala Gly Thr Thr Val Leu Ser
            415                 420                 425

tgt ggc tgt gtc tca gct ggc agc cct ggg cta gga ggg ccc ctg gga     1769
Cys Gly Cys Val Ser Ala Gly Ser Pro Gly Leu Gly Gly Pro Leu Gly
        430                 435                 440

agc ctc ctg gac aga cta aag cca ccc ctt gca gat ggg gag gac tgg     1817
Ser Leu Leu Asp Arg Leu Lys Pro Pro Leu Ala Asp Gly Glu Asp Trp
    445                 450                 455

gct ggg gga ctg ccc tgg ggt ggc cgg tca cct gga ggg gtc tca gag     1865
Ala Gly Gly Leu Pro Trp Gly Gly Arg Ser Pro Gly Gly Val Ser Glu
460                 465                 470                 475

agt gag gcg ggc tca ccc ctg gcc ggc ctg gat atg gac acg ttt gac     1913
Ser Glu Ala Gly Ser Pro Leu Ala Gly Leu Asp Met Asp Thr Phe Asp
                480                 485                 490

agt ggc ttt gtg ggc tct gac tgc agc agc cct gtg gag tgt gac ttc     1961
Ser Gly Phe Val Gly Ser Asp Cys Ser Ser Pro Val Glu Cys Asp Phe
            495                 500                 505

acc agc ccc ggg gac gaa gga ccc ccc cgg agc tac ctc cgc cag tgg     2009
Thr Ser Pro Gly Asp Glu Gly Pro Pro Arg Ser Tyr Leu Arg Gln Trp
        510                 515                 520

gtg gtc att cct ccg cca ctt tcg agc cct gga ccc cag gcc agc taa     2057
Val Val Ile Pro Pro Pro Leu Ser Ser Pro Gly Pro Gln Ala Ser
    525                 530                 535

tgaggctgac tggatgtcca gagctggcca ggccactggg ccctgagcca gagacaaggt   2117

cacctgggct gtgatgtgaa gacacctgca gcctttggtc tcctggatgg gcctttgagc 2177

ctgatgttta cagtgtctgt gtgtgtgtgc atatgtgtgt gtgtgcatat gcatgtgtgt 2237

gtgtgtgtgt gtcttaggtg cgcagtggca tgtccacgtg tgtgtgattg cacgtgcctg 2297

tgggcctggg ataatgccca tggtactcca tgcattcacc tgccctgtgc atgtctggac 2357

tcacggagct cacccatgtg cacaagtgtg cacagtaaac gtgtttgtgg tcaacaga 2415

<210> 9
<211> 30
<212> DNA
<213> Artificial Sequence

<220>
<223> Artificially Synthesized Primer Sequence

<400> 9
ccggctcccc ctttcaacgt gactgtgacc 30

<210> 10
<211> 30
<212> DNA
<213> Artificial Sequence

<220>
<223> Artificially Synthesized Primer Sequence

<400> 10
ggcaagcttc agtatgagct gcagtacagg 30

<210> 11
<211> 30
<212> DNA
<213> Artificial Sequence

<220>

<223> Artificially Synthesized Primer Sequence

<400> 11
accctctgac tgggtctgaa agatgaccgg                                        30


<210> 12
<211> 30
<212> DNA
<213> Artificial Sequence

<220>
<223> Artificially Synthesized Primer Sequence

<400> 12
catgggccct gcccgcacct gcagctcata                                        30


<210> 13
<211> 1128
<212> DNA
<213> Homo sapiens

<220>
<221> CDS
<222> (1)..(1125)

<400> 13
atg ccg cgt ggc tgg gcc gcc ccc ttg ctc ctg ctg ctg ctc cag gga     48
Met Pro Arg Gly Trp Ala Ala Pro Leu Leu Leu Leu Leu Leu Gln Gly
1               5                   10                  15

ggc tgg ggc tgc ccc gac ctc gtc tgc tac acc gat tac ctc cag acg     96
Gly Trp Gly Cys Pro Asp Leu Val Cys Tyr Thr Asp Tyr Leu Gln Thr
            20                  25                  30

gtc atc tgc atc ctg gaa atg tgg aac ctc cac ccc agc acg ctc acc     144
Val Ile Cys Ile Leu Glu Met Trp Asn Leu His Pro Ser Thr Leu Thr
        35                  40                  45

ctt acc tgg caa gac cag tat gaa gag ctg aag gac gag gcc acc tcc     192

Leu Thr Trp Gln Asp Gln Tyr Glu Glu Leu Lys Asp Glu Ala Thr Ser
    50                  55                  60

tgc agc ctc cac agg tcg gcc cac aat gcc acg cat gcc acc tac acc 240
Cys Ser Leu His Arg Ser Ala His Asn Ala Thr His Ala Thr Tyr Thr
65                  70                  75                  80

tgc cac atg gat gta ttc cac ttc atg gcc gac gac att ttc agt gtc 288
Cys His Met Asp Val Phe His Phe Met Ala Asp Asp Ile Phe Ser Val
                85                  90                  95

aac atc aca gac cag tct ggc aac tac tcc cag gag tgt ggc agc ttt 336
Asn Ile Thr Asp Gln Ser Gly Asn Tyr Ser Gln Glu Cys Gly Ser Phe
            100                 105                 110

ctc ctg gct gag agc atc aag ccg gct ccc cct ttc aac gtg act gtg 384
Leu Leu Ala Glu Ser Ile Lys Pro Ala Pro Pro Phe Asn Val Thr Val
        115                 120                 125

acc ttc tca gga cag tat aat atc tcc tgg cgc tca gat tac gaa gac 432
Thr Phe Ser Gly Gln Tyr Asn Ile Ser Trp Arg Ser Asp Tyr Glu Asp
    130                 135                 140

cct gcc ttc tac atg ctg aag ggc aag ctt cag tat gag ctg cag tac 480
Pro Ala Phe Tyr Met Leu Lys Gly Lys Leu Gln Tyr Glu Leu Gln Tyr
145                 150                 155                 160

agg aac cgg gga gac ccc tgg gct gtg agt ccg agg aga aag ctg atc 528
Arg Asn Arg Gly Asp Pro Trp Ala Val Ser Pro Arg Arg Lys Leu Ile
                165                 170                 175

tca gtg gac tca aga agt gtc tcc ctc ctc ccc ctg gag ttc cgc aaa 576
Ser Val Asp Ser Arg Ser Val Ser Leu Leu Pro Leu Glu Phe Arg Lys
            180                 185                 190

gac tcg agc tat gag ctg cag gtg cgg gca ggg ccc atg cct ggc tcc 624
Asp Ser Ser Tyr Glu Leu Gln Val Arg Ala Gly Pro Met Pro Gly Ser
        195                 200                 205

tcc tac cag ggg acc tgg agt gaa tgg agt gac ccg gtc atc ttt cag 672
Ser Tyr Gln Gly Thr Trp Ser Glu Trp Ser Asp Pro Val Ile Phe Gln

210                 215                 220

acc cag tca gag acc gcc tgg atc tcc ttg gtg acc gct ctg cat cta     720
Thr Gln Ser Glu Thr Ala Trp Ile Ser Leu Val Thr Ala Leu His Leu
225                 230                 235                 240

gtg ctg ggc ctc agc gcc gtc ctg ggc ctg ctg ctg ctg agg tgg cag     768
Val Leu Gly Leu Ser Ala Val Leu Gly Leu Leu Leu Leu Arg Trp Gln
                245                 250                 255

ttt cct gca cac tac agg aga ctg agg cat gcc ctg tgg ccc tca ctt     816
Phe Pro Ala His Tyr Arg Arg Leu Arg His Ala Leu Trp Pro Ser Leu
            260                 265                 270

cca gac ctg cac cgg gtc cta ggc cag tac ctt agg gac act gca gcc     864
Pro Asp Leu His Arg Val Leu Gly Gln Tyr Leu Arg Asp Thr Ala Ala
        275                 280                 285

ctg agc ccg ccc aag gcc aca gtc tca gat acc tgt gaa gaa gtg gaa     912
Leu Ser Pro Pro Lys Ala Thr Val Ser Asp Thr Cys Glu Glu Val Glu
    290                 295                 300

ccc agc ctc ctt gaa atc ctc ccc aag tcc tca gag agg act cct ttg     960
Pro Ser Leu Leu Glu Ile Leu Pro Lys Ser Ser Glu Arg Thr Pro Leu
305                 310                 315                 320

ccc ctg tgt tcc tcc cag gcc cag atg gac tac cga aga ttg cag cct     1008
Pro Leu Cys Ser Ser Gln Ala Gln Met Asp Tyr Arg Arg Leu Gln Pro
                325                 330                 335

tct tgc ctg ggg acc atg ccc ctg tct gtg tgc cca ccc atg gct gag     1056
Ser Cys Leu Gly Thr Met Pro Leu Ser Val Cys Pro Pro Met Ala Glu
            340                 345                 350

tca ggg tcc tgc tgt acc acc cac att gcc aac cat tcc tac cta cca     1104
Ser Gly Ser Cys Cys Thr Thr His Ile Ala Asn His Ser Tyr Leu Pro
        355                 360                 365

cta agc tat tgg cag cag cct tga                                     1128
Leu Ser Tyr Trp Gln Gln Pro
    370                 375

<210> 14
<211> 375
<212> PRT
<213> Homo sapiens

<400> 14
Met Pro Arg Gly Trp Ala Ala Pro Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Cys His Met Asp Val Phe His Phe Met Ala Asp Asp Ile Phe Ser Val
                85                  90                  95

Asn Ile Thr Asp Gln Ser Gly Asn Tyr Ser Gln Glu Cys Gly Ser Phe
            100                 105                 110

Leu Leu Ala Glu Ser Ile Lys Pro Ala Pro Pro Phe Asn Val Thr Val
        115                 120                 125

Thr Phe Ser Gly Gln Tyr Asn Ile Ser Trp Arg Ser Asp Tyr Glu Asp
    130                 135                 140

Pro Ala Phe Tyr Met Leu Lys Gly Lys Leu Gln Tyr Glu Leu Gln Tyr
145                 150                 155                 160

Arg Asn Arg Gly Asp Pro Trp Ala Val Ser Pro Arg Arg Lys Leu Ile
                165                 170                 175

Ser Val Asp Ser Arg Ser Val Ser Leu Leu Pro Leu Glu Phe Arg Lys
            180                 185                 190

Asp Ser Ser Tyr Glu Leu Gln Val Arg Ala Gly Pro Met Pro Gly Ser
        195                 200                 205

Ser Tyr Gln Gly Thr Trp Ser Glu Trp Ser Asp Pro Val Ile Phe Gln
    210                 215                 220

Thr Gln Ser Glu Thr Ala Trp Ile Ser Leu Val Thr Ala Leu His Leu
225                 230                 235                 240

Val Leu Gly Leu Ser Ala Val Leu Gly Leu Leu Leu Leu Arg Trp Gln
                245                 250                 255

Phe Pro Ala His Tyr Arg Arg Leu Arg His Ala Leu Trp Pro Ser Leu
            260                 265                 270

Pro Asp Leu His Arg Val Leu Gly Gln Tyr Leu Arg Asp Thr Ala Ala
        275                 280                 285

Leu Ser Pro Pro Lys Ala Thr Val Ser Asp Thr Cys Glu Glu Val Glu
    290                 295                 300

Pro Ser Leu Leu Glu Ile Leu Pro Lys Ser Ser Glu Arg Thr Pro Leu
305                 310                 315                 320

Pro Leu Cys Ser Ser Gln Ala Gln Met Asp Tyr Arg Arg Leu Gln Pro
                325                 330                 335

Ser Cys Leu Gly Thr Met Pro Leu Ser Val Cys Pro Pro Met Ala Glu
            340                 345                 350

Ser Gly Ser Cys Cys Thr Thr His Ile Ala Asn His Ser Tyr Leu Pro
        355                 360                 365

Leu Ser Tyr Trp Gln Gln Pro
    370                 375

<210> 15

<211> 1383
<212> DNA
<213> Homo sapiens

<220>
<221> CDS
<222> (1)..(1380)

<400> 15
atg ccg cgt ggc tgg gcc gcc ccc ttg ctc ctg ctg ctg ctc cag gga     48
Met Pro Arg Gly Trp Ala Ala Pro Leu Leu Leu Leu Leu Leu Gln Gly
1               5                   10                  15

ggc tgg ggc tgc ccc gac ctc gtc tgc tac acc gat tac ctc cag acg     96
Gly Trp Gly Cys Pro Asp Leu Val Cys Tyr Thr Asp Tyr Leu Gln Thr
            20                  25                  30

gtc atc tgc atc ctg gaa atg tgg aac ctc cac ccc agc acg ctc acc     144
Val Ile Cys Ile Leu Glu Met Trp Asn Leu His Pro Ser Thr Leu Thr
        35                  40                  45

ctt acc tgg caa gac cag tat gaa gag ctg aag gac gag gcc acc tcc     192
Leu Thr Trp Gln Asp Gln Tyr Glu Glu Leu Lys Asp Glu Ala Thr Ser
    50                  55                  60

tgc agc ctc cac agg tcg gcc cac aat gcc acg cat gcc acc tac acc     240
Cys Ser Leu His Arg Ser Ala His Asn Ala Thr His Ala Thr Tyr Thr
65                  70                  75                  80

tgc cac atg gat gta ttc cac ttc atg gcc gac gac att ttc agt gtc     288
Cys His Met Asp Val Phe His Phe Met Ala Asp Asp Ile Phe Ser Val
                85                  90                  95

aac atc aca gac cag tct ggc aac tac tcc cag gag tgt ggc agc ttt     336
Asn Ile Thr Asp Gln Ser Gly Asn Tyr Ser Gln Glu Cys Gly Ser Phe
            100                 105                 110

ctc ctg gct gag agc atc aag ccg gct ccc cct ttc aac gtg act gtg     384
Leu Leu Ala Glu Ser Ile Lys Pro Ala Pro Pro Phe Asn Val Thr Val
        115                 120                 125

acc ttc tca gga cag tat aat atc tcc tgg cgc tca gat tac gaa gac 432
Thr Phe Ser Gly Gln Tyr Asn Ile Ser Trp Arg Ser Asp Tyr Glu Asp
    130                 135                 140

cct gcc ttc tac atg ctg aag ggc aag ctt cag tat gag ctg cag tac 480
Pro Ala Phe Tyr Met Leu Lys Gly Lys Leu Gln Tyr Glu Leu Gln Tyr
145                 150                 155                 160

agg aac cgg gga gac ccc tgg gct gtg agt ccg agg aga aag ctg atc 528
Arg Asn Arg Gly Asp Pro Trp Ala Val Ser Pro Arg Arg Lys Leu Ile
                165                 170                 175

tca gtg gac tca aga agt gtc tcc ctc ctc ccc ctg gag ttc cgc aaa 576
Ser Val Asp Ser Arg Ser Val Ser Leu Leu Pro Leu Glu Phe Arg Lys
            180                 185                 190

gac tcg agc tat gag ctg cag gtg cgg gca ggg ccc atg cct ggc tcc 624
Asp Ser Ser Tyr Glu Leu Gln Val Arg Ala Gly Pro Met Pro Gly Ser
        195                 200                 205

tcc tac cag ggg acc tgg agt gaa tgg agt gac ccg gtc atc ttt cag 672
Ser Tyr Gln Gly Thr Trp Ser Glu Trp Ser Asp Pro Val Ile Phe Gln
    210                 215                 220

acc cag tca gag gag ccc aaa tct tgt gac aaa act cac aca tgc cca 720
Thr Gln Ser Glu Glu Pro Lys Ser Cys Asp Lys Thr His Thr Cys Pro
225                 230                 235                 240

ccg tgc cca gca cct gaa ctc ctg ggg gga ccg tca gtc ttc ctc ttc 768
Pro Cys Pro Ala Pro Glu Leu Leu Gly Gly Pro Ser Val Phe Leu Phe
                245                 250                 255

ccc cca aaa ccc aag gac acc ctc atg atc tcc cgg acc cct gag gtc 816
Pro Pro Lys Pro Lys Asp Thr Leu Met Ile Ser Arg Thr Pro Glu Val
            260                 265                 270

aca tgc gtg gtg gtg gac gtg agc cac gaa gac cct gag gtc aag ttc 864
Thr Cys Val Val Val Asp Val Ser His Glu Asp Pro Glu Val Lys Phe
        275                 280                 285

aac tgg tac gtg gac ggc gtg gag gtg cat aat gcc aag aca aag ccg 912

Asn Trp Tyr Val Asp Gly Val Glu Val His Asn Ala Lys Thr Lys Pro
    290                 295                 300

cgg gag gag cag tac aac agc acg tac cgg gtg gtc agc gtc ctc acc 960
Arg Glu Glu Gln Tyr Asn Ser Thr Tyr Arg Val Val Ser Val Leu Thr
305                 310                 315                 320

gtc ctg cac cag gac tgg ctg aat ggc aag gag tac aag tgc aag gtc 1008
Val Leu His Gln Asp Trp Leu Asn Gly Lys Glu Tyr Lys Cys Lys Val
                325                 330                 335

tcc aac aaa gcc ctc cca gcc ccc atc gag aaa acc atc tcc aaa gcc 1056
Ser Asn Lys Ala Leu Pro Ala Pro Ile Glu Lys Thr Ile Ser Lys Ala
            340                 345                 350

aaa ggg cag ccc cga gaa cca cag gtg tac acc ctg ccc cca tcc cgg 1104
Lys Gly Gln Pro Arg Glu Pro Gln Val Tyr Thr Leu Pro Pro Ser Arg
        355                 360                 365

gat gag ctg acc aag aac cag gtc agc ctg acc tgc ctg gtc aaa ggc 1152
Asp Glu Leu Thr Lys Asn Gln Val Ser Leu Thr Cys Leu Val Lys Gly
    370                 375                 380

ttc tat ccc agc gac atc gcc gtg gag tgg gag agc aat ggg cag ccg 1200
Phe Tyr Pro Ser Asp Ile Ala Val Glu Trp Glu Ser Asn Gly Gln Pro
385                 390                 395                 400

gag aac aac tac aag acc acg cct ccc gtg ctg gac tcc gac ggc tcc 1248
Glu Asn Asn Tyr Lys Thr Thr Pro Pro Val Leu Asp Ser Asp Gly Ser
                405                 410                 415

ttc ttc ctc tac agc aag ctc acc gtg gac aag agc agg tgg cag cag 1296
Phe Phe Leu Tyr Ser Lys Leu Thr Val Asp Lys Ser Arg Trp Gln Gln
            420                 425                 430

ggg aac gtc ttc tca tgc tcc gtg atg cat gag gct ctg cac aac cac 1344
Gly Asn Val Phe Ser Cys Ser Val Met His Glu Ala Leu His Asn His
        435                 440                 445

tac acg cag aag agc ctc tcc ctg tct ccg ggt aaa tga 1383
Tyr Thr Gln Lys Ser Leu Ser Leu Ser Pro Gly Lys

450                 455                 460

<210> 16
<211> 460
<212> PRT
<213> Homo sapiens

<400> 16
Met Pro Arg Gly Trp Ala Ala Pro Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Cys His Met Asp Val Phe His Phe Met Ala Asp Asp Ile Phe Ser Val
                85                  90                  95

Asn Ile Thr Asp Gln Ser Gly Asn Tyr Ser Gln Glu Cys Gly Ser Phe
            100                 105                 110

Leu Leu Ala Glu Ser Ile Lys Pro Ala Pro Pro Phe Asn Val Thr Val
        115                 120                 125

Thr Phe Ser Gly Gln Tyr Asn Ile Ser Trp Arg Ser Asp Tyr Glu Asp
    130                 135                 140

Pro Ala Phe Tyr Met Leu Lys Gly Lys Leu Gln Tyr Glu Leu Gln Tyr
145                 150                 155                 160

Arg Asn Arg Gly Asp Pro Trp Ala Val Ser Pro Arg Arg Lys Leu Ile
                165                 170                 175

Ser Val Asp Ser Arg Ser Val Ser Leu Leu Pro Leu Glu Phe Arg Lys
            180                 185                 190

Asp Ser Ser Tyr Glu Leu Gln Val Arg Ala Gly Pro Met Pro Gly Ser
        195                 200                 205

Ser Tyr Gln Gly Thr Trp Ser Glu Trp Ser Asp Pro Val Ile Phe Gln
    210                 215                 220

Thr Gln Ser Glu Glu Pro Lys Ser Cys Asp Lys Thr His Thr Cys Pro
225                 230                 235                 240

Pro Cys Pro Ala Pro Glu Leu Leu Gly Gly Pro Ser Val Phe Leu Phe
                245                 250                 255

Pro Pro Lys Pro Lys Asp Thr Leu Met Ile Ser Arg Thr Pro Glu Val
            260                 265                 270

Thr Cys Val Val Val Asp Val Ser His Glu Asp Pro Glu Val Lys Phe
        275                 280                 285

Asn Trp Tyr Val Asp Gly Val Glu Val His Asn Ala Lys Thr Lys Pro
    290                 295                 300

Arg Glu Glu Gln Tyr Asn Ser Thr Tyr Arg Val Val Ser Val Leu Thr
305                 310                 315                 320

Val Leu His Gln Asp Trp Leu Asn Gly Lys Glu Tyr Lys Cys Lys Val
                325                 330                 335

Ser Asn Lys Ala Leu Pro Ala Pro Ile Glu Lys Thr Ile Ser Lys Ala
            340                 345                 350

Lys Gly Gln Pro Arg Glu Pro Gln Val Tyr Thr Leu Pro Pro Ser Arg
        355                 360                 365

Asp Glu Leu Thr Lys Asn Gln Val Ser Leu Thr Cys Leu Val Lys Gly
    370                 375                 380

Phe Tyr Pro Ser Asp Ile Ala Val Glu Trp Glu Ser Asn Gly Gln Pro

385                 390                 395                 400

Glu Asn Asn Tyr Lys Thr Thr Pro Pro Val Leu Asp Ser Asp Gly Ser
                405                 410                 415

Phe Phe Leu Tyr Ser Lys Leu Thr Val Asp Lys Ser Arg Trp Gln Gln
            420                 425                 430

Gly Asn Val Phe Ser Cys Ser Val Met His Glu Ala Leu His Asn His
        435                 440                 445

Tyr Thr Gln Lys Ser Leu Ser Leu Ser Pro Gly Lys
    450                 455                 460

<210> 17
<211> 477
<212> DNA
<213> Homo sapiens

<220>
<221> CDS
<222> (1)..(474)

<400> 17
atg ccg cgt ggc tgg gcc gcc ccc ttg ctc ctg ctg ctg ctc cag gga      48
Met Pro Arg Gly Trp Ala Ala Pro Leu Leu Leu Leu Leu Leu Gln Gly
1               5                   10                  15

ggc tgg ggc tgc ccc gac ctc gtc tgc tac acc gat tac ctc cag acg      96
Gly Trp Gly Cys Pro Asp Leu Val Cys Tyr Thr Asp Tyr Leu Gln Thr
            20                  25                  30

gtc atc tgc atc ctg gaa atg tgg aac ctc cac ccc agc acg ctc acc      144
Val Ile Cys Ile Leu Glu Met Trp Asn Leu His Pro Ser Thr Leu Thr
        35                  40                  45

ctt acc tgg caa gac cag tat gaa gag ctg aag gac gag gcc acc tcc      192
Leu Thr Trp Gln Asp Gln Tyr Glu Glu Leu Lys Asp Glu Ala Thr Ser
    50                  55                  60

tgc agc ctc cac agg tcg gcc cac aat gcc acg cat gcc acc tac acc    240
Cys Ser Leu His Arg Ser Ala His Asn Ala Thr His Ala Thr Tyr Thr
65                  70                  75                  80

tgc cac atg gat gta ttc cac ttc atg gcc gac gac att ttc agt gtc    288
Cys His Met Asp Val Phe His Phe Met Ala Asp Asp Ile Phe Ser Val
                85                  90                  95

aac atc aca gac cag tct ggc aac tac tcc cag gag tgt ggc agc ttt    336
Asn Ile Thr Asp Gln Ser Gly Asn Tyr Ser Gln Glu Cys Gly Ser Phe
            100                 105                 110

ctc ctg gct gag agc aag tcc gag gag aaa gct gat ctc agt gga ctc    384
Leu Leu Ala Glu Ser Lys Ser Glu Glu Lys Ala Asp Leu Ser Gly Leu
        115                 120                 125

aag aag tgt ctc cct cct ccc cct gga gtt ccg caa aga ctc gag cta    432
Lys Lys Cys Leu Pro Pro Pro Pro Gly Val Pro Gln Arg Leu Glu Leu
    130                 135                 140

agg gcg cgc cag gac tac aag gac gac gat gac aag acg cgt taa        477
Arg Ala Arg Gln Asp Tyr Lys Asp Asp Asp Asp Lys Thr Arg
145                 150                 155


<210> 18
<211> 158
<212> PRT
<213> Homo sapiens

<400> 18
Met Pro Arg Gly Trp Ala Ala Pro Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser

50                  55                  60

Cys Ser Leu His Arg Ser Ala His Asn Ala Thr His Ala Thr Tyr Thr
65                  70                  75                  80

Cys His Met Asp Val Phe His Phe Met Ala Asp Asp Ile Phe Ser Val
                85                  90                  95

Asn Ile Thr Asp Gln Ser Gly Asn Tyr Ser Gln Glu Cys Gly Ser Phe
            100                 105                 110

Leu Leu Ala Glu Ser Lys Ser Glu Glu Lys Ala Asp Leu Ser Gly Leu
        115                 120                 125

Lys Lys Cys Leu Pro Pro Pro Pro Gly Val Pro Gln Arg Leu Glu Leu
    130                 135                 140

Arg Ala Arg Gln Asp Tyr Lys Asp Asp Asp Asp Lys Thr Arg
145                 150                 155

<210> 19
<211> 144
<212> PRT
<213> Mus musculus

<400> 19
Met Pro Arg Gly Trp Ala Ala Ser Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Ser His Met Asp Val Phe His Phe Met Ala Asp Asp Ile Phe Ser Val
                85                  90                  95

Asn Ile Thr Asp Gln Ser Gly Asn Tyr Phe Gln Glu Cys Gly Ser Phe
            100                 105                 110

Leu Arg Ala Glu Ser Lys Ser Glu Glu Lys Ala Asp Leu Ser Gly Leu
        115                 120                 125

Lys Lys Cys Leu Pro Pro Pro Pro Gly Val Pro Gln Arg Leu Glu Leu
    130                 135                 140

<210> 20
<211> 1960
<212> DNA
<213> Mus musculus

<400> 20
cagccagcgg cctcagacag acccactggc gtctctctgc tgagtgaccg taagctcggc 60

gtctggccct ctgcctgcct ctccctgagt gtggctgaca gccacgcagc tgtgtctgtc 120

tgtctgcggc ccgtgcatcc ctgctgcggc cgcctggtac cttccttgcc gtctctttcc 180

tctgtctgct gctctgtggg acacctgcct ggaggcccag ctgcccgtca tcagagtgac 240

aggtcttatg acagcctgat tggtgactcg ggctgggtgt ggattctcac cccaggcctc 300

tgcctgcttt ctcagaccct catcggtcac ccccacgctg aacccagctg ccacccccag 360

aagcccatca gactgccccc agcacacgga atggatttct gagaaagaag ccgaaacaga 420

aggcccgtgg gagtcagc atg ccg cgt ggc tgg gcc gcc tcc ttg ctc ctg    471
                    Met Pro Arg Gly Trp Ala Ala Ser Leu Leu Leu
                    1               5                   10

ctg ctg ctc cag gga ggc tgg ggc tgc ccc gac ctc gtc tgc tac acc    519
Leu Leu Leu Gln Gly Gly Trp Gly Cys Pro Asp Leu Val Cys Tyr Thr
            15                  20                  25

gat tac ctc cag acg gtc atc tgc atc ctg gaa atg tgg aac ctc cac 567
Asp Tyr Leu Gln Thr Val Ile Cys Ile Leu Glu Met Trp Asn Leu His
        30                  35                  40

ccc agc acg ctc acc ctt acc tgg caa gac cag tat gaa gag ctg aag 615
Pro Ser Thr Leu Thr Leu Thr Trp Gln Asp Gln Tyr Glu Glu Leu Lys
    45                  50                  55

gac gag gcc acc tcc tgc agc ctc cac agg tcg gcc cac aat gcc acg 663
Asp Glu Ala Thr Ser Cys Ser Leu His Arg Ser Ala His Asn Ala Thr
60                  65                  70                  75

cat gcc acc tac acc agc cac atg gat gta ttc cac ttc atg gcc gac 711
His Ala Thr Tyr Thr Ser His Met Asp Val Phe His Phe Met Ala Asp
                80                  85                  90

gac att ttc agt gtc aac atc aca gac cag tct ggc aac tac ttc cag 759
Asp Ile Phe Ser Val Asn Ile Thr Asp Gln Ser Gly Asn Tyr Phe Gln
            95                  100                 105

gag tgt ggc agc ttt ctc cgg gct gag agc aag tcc gag gag aaa gct 807
Glu Cys Gly Ser Phe Leu Arg Ala Glu Ser Lys Ser Glu Glu Lys Ala
        110                 115                 120

gat ctc agt gga ctc aag aag tgt ctc cct cct ccc cct gga gtt ccg 855
Asp Leu Ser Gly Leu Lys Lys Cys Leu Pro Pro Pro Pro Gly Val Pro
    125                 130                 135

caa aga ctc gag cta tgagctgcag gtgcgggcag ggcccatgcc tggctcctcc 910
Gln Arg Leu Glu Leu
140

taccagggga cctggagtga atggagtgac ccggtcatct ttcagaccca gtcagaggag 970

ttaaaggaag gctggaaccc tcacctgctg cttctcctcc tgcttgtcat agtcttcatt 1030

cctgccttct ggagcctgaa gacccatcca ttgtggaggc tatggaagaa gatatgggcc 1090

gtccccagcc ctgagcggtt cttcatgccc ctgtacaagg gctgcagcgg agacttcaag 1150

aaatgggtgg gtgcaccctt cactggctcc agcctggagc tgggaccctg gagcccagag 1210

gtgccctcca ccctggaggt gtacagctgc cacccaccac ggagcccggc caagaggctg 1270

cagctcacgg agctacaaga accagcagag ctggtggagt ctgacggtgt gcccaagccc 1330

agcttctggc cgacagccca gaactcgggg ggctcagctt acagtgagga gagggatcgg 1390

ccatacggcc tggtgtccat tgacacagtg actgtgctag atgcagaggg gccatgcacc 1450

tggccctgca gctgtgagga tgacggctac ccagccctgg acctggatgc tggcctggag 1510

cccagcccag gcctagagga cccactcttg gatgcaggga ccacagtcct gtcctgtggc 1570

tgtgtctcag ctggcagccc tgggctagga gggcccctgg gaagcctcct ggacagacta 1630

aagccacccc ttgcagatgg ggaggactgg gctgggggac tgccctgggg tggccggtca 1690

cctggagggg tctcagagag tgaggcgggc tcacccctgg ccggcctgga tatggacacg 1750

tttgacagtg gctttgtgtg ctctgactgc agcagccctg tggagtgtga cttcaccagc 1810

cccggggacg aaggaccccc ccggagctac ctccgccagt gggtggtcat tcctccgcca 1870

ctttcgagcc ctggacccca ggccagctaa tgaggctgac tggatgtcca gagctggcca 1930

ggccactggg ccctgagcca gaaaaaaaaa 1960

<210> 21
<211> 538
<212> PRT
<213> Mus musculus

<400> 21
Met Pro Arg Gly Trp Ala Ala Ser Leu Leu Leu Leu Leu Leu Gln Gly
1               5                   10                  15

Gly Trp Gly Cys Pro Asp Leu Val Cys Tyr Thr Asp Tyr Leu Gln Thr
            20                  25                  30

Val Ile Cys Ile Leu Glu Met Trp Asn Leu His Pro Ser Thr Leu Thr
        35                  40                  45

Leu Thr Trp Gln Asp Gln Tyr Glu Glu Leu Lys Asp Glu Ala Thr Ser
    50                  55                  60

Cys Ser Leu His Arg Ser Ala His Asn Ala Thr His Ala Thr Tyr Thr
65                  70                  75                  80

Ser His Met Asp Val Phe His Phe Met Ala Asp Asp Ile Phe Ser Val
                85                  90                  95

Asn Ile Thr Asp Gln Ser Gly Asn Tyr Phe Gln Glu Cys Gly Ser Phe
            100                 105                 110

Leu Arg Ala Glu Ser Ile Lys Pro Ala Pro Pro Phe Asn Val Thr Val
        115                 120                 125

Thr Phe Ser Gly Gln Tyr Asn Ile Ser Arg Arg Ser Asp Tyr Glu Asp
    130                 135                 140

Pro Ala Phe Tyr Met Leu Lys Gly Lys Leu Gln Tyr Glu Leu Gln Tyr
145                 150                 155                 160

Arg Asn Arg Gly Asp Pro Trp Ala Val Ser Pro Arg Arg Lys Leu Ile
                165                 170                 175

Ser Val Asp Ser Arg Ser Val Ser Leu Leu Pro Leu Glu Phe Arg Lys
            180                 185                 190

Asp Ser Ser Tyr Glu Leu Gln Val Arg Ala Gly Pro Met Pro Gly Ser
        195                 200                 205

Ser Tyr Gln Gly Thr Trp Ser Glu Trp Ser Asp Pro Val Ile Phe Gln
    210                 215                 220

Thr Gln Ser Glu Glu Leu Lys Glu Gly Trp Asn Pro His Leu Leu Leu
225                 230                 235                 240

Leu Leu Leu Leu Val Ile Val Phe Ile Pro Ala Phe Trp Ser Leu Lys
                245                 250                 255

Thr His Pro Leu Trp Arg Leu Trp Lys Lys Ile Trp Ala Val Pro Ser
            260                 265                 270

Pro Glu Arg Phe Phe Met Pro Leu Tyr Lys Gly Cys Ser Gly Asp Phe
        275                 280                 285

Lys Lys Trp Val Gly Ala Pro Phe Thr Gly Ser Ser Leu Glu Leu Gly
    290                 295                 300

Pro Trp Ser Pro Glu Val Pro Ser Thr Leu Glu Val Tyr Ser Cys His
305                 310                 315                 320

Pro Pro Arg Ser Pro Ala Lys Arg Leu Gln Leu Thr Glu Leu Gln Glu
                325                 330                 335

Pro Ala Glu Leu Val Glu Ser Asp Gly Val Pro Lys Pro Ser Phe Trp
            340                 345                 350

Pro Thr Ala Gln Asn Ser Gly Gly Ser Ala Tyr Ser Glu Glu Arg Asp
        355                 360                 365

Arg Pro Tyr Gly Leu Val Ser Ile Asp Thr Val Thr Val Leu Asp Ala
    370                 375                 380

Glu Gly Pro Cys Thr Trp Pro Cys Ser Cys Glu Asp Asp Gly Tyr Pro
385                 390                 395                 400

Ala Leu Asp Leu Asp Ala Gly Leu Glu Pro Ser Pro Gly Leu Glu Asp
                405                 410                 415

Pro Leu Leu Asp Ala Gly Thr Thr Val Leu Ser Cys Gly Cys Val Ser
            420                 425                 430

Ala Gly Ser Pro Gly Leu Gly Gly Pro Leu Gly Ser Leu Leu Asp Arg
        435                 440                 445

Leu Lys Pro Pro Leu Ala Asp Gly Glu Asp Trp Ala Gly Gly Leu Pro
    450                 455                 460

Trp Gly Gly Arg Ser Pro Gly Gly Val Ser Glu Ser Glu Ala Gly Ser

465                 470                 475                 480

Pro Leu Ala Gly Leu Asp Met Asp Thr Phe Asp Ser Gly Phe Val Cys
                485                 490                 495

Ser Asp Cys Ser Ser Pro Val Glu Cys Asp Phe Thr Ser Pro Gly Asp
            500                 505                 510

Glu Gly Pro Pro Arg Ser Tyr Leu Arg Gln Trp Val Val Ile Pro Pro
        515                 520                 525

Pro Leu Ser Ser Pro Gly Pro Gln Ala Ser
    530                 535

<210> 22
<211> 2115
<212> DNA
<213> Mus musculus

<400> 22
cagccagcgg cctcagacag acccactggc gtctctctgc tgagtgaccg taagctcggc 60

gtctggccct ctgcctgcct ctccctgagt gtggctgaca gccacgcagc tgtgtctgtc 120

tgtctgcggc ccgtgcatcc ctgctgcggc cgcctggtac cttccttgcc gtctctttcc 180

tctgtctgct gctctgtggg acacctgcct ggaggcccag ctgcccgtca tcagagtgac 240

aggtcttatg acagcctgat tggtgactcg ggctgggtgt ggattctcac cccaggcctc 300

tgcctgcttt ctcagaccct catcggtcac ccccacgctg aacccagctg ccacccccag 360

aagcccatca gactgccccc agcacacgga atggatttct gagaaagaag ccgaaacaga 420

aggcccgtgg gagtcagc atg ccg cgt ggc tgg gcc gcc tcc ttg ctc ctg   471
                    Met Pro Arg Gly Trp Ala Ala Ser Leu Leu Leu
                    1               5                   10

ctg ctg ctc cag gga ggc tgg ggc tgc ccc gac ctc gtc tgc tac acc   519
Leu Leu Leu Gln Gly Gly Trp Gly Cys Pro Asp Leu Val Cys Tyr Thr

            15                  20                  25

gat tac ctc cag acg gtc atc tgc atc ctg gaa atg tgg aac ctc cac 567
Asp Tyr Leu Gln Thr Val Ile Cys Ile Leu Glu Met Trp Asn Leu His
        30                  35                  40

ccc agc acg ctc acc ctt acc tgg caa gac cag tat gaa gag ctg aag 615
Pro Ser Thr Leu Thr Leu Thr Trp Gln Asp Gln Tyr Glu Glu Leu Lys
    45                  50                  55

gac gag gcc acc tcc tgc agc ctc cac agg tcg gcc cac aat gcc acg 663
Asp Glu Ala Thr Ser Cys Ser Leu His Arg Ser Ala His Asn Ala Thr
60                  65                  70                  75

cat gcc acc tac acc agc cac atg gat gta ttc cac ttc atg gcc gac 711
His Ala Thr Tyr Thr Ser His Met Asp Val Phe His Phe Met Ala Asp
                80                  85                  90

gac att ttc agt gtc aac atc aca gac cag tct ggc aac tac ttc cag 759
Asp Ile Phe Ser Val Asn Ile Thr Asp Gln Ser Gly Asn Tyr Phe Gln
            95                  100                 105

gag tgt ggc agc ttt ctc cgg gct gag agc atc aag ccg gct ccc cct 807
Glu Cys Gly Ser Phe Leu Arg Ala Glu Ser Ile Lys Pro Ala Pro Pro
        110                 115                 120

ttc aac gtg act gtg acc ttc tca gga cag tat aat atc tcc agg cgc 855
Phe Asn Val Thr Val Thr Phe Ser Gly Gln Tyr Asn Ile Ser Arg Arg
    125                 130                 135

tca gat tac gaa gac cct gcc ttc tac atg ctg aag ggc aag ctt cag 903
Ser Asp Tyr Glu Asp Pro Ala Phe Tyr Met Leu Lys Gly Lys Leu Gln
140                 145                 150                 155

tat gag ctg cag tac agg aac cgg gga gac ccc tgg gct gtg agt ccg 951
Tyr Glu Leu Gln Tyr Arg Asn Arg Gly Asp Pro Trp Ala Val Ser Pro
                160                 165                 170

agg aga aag ctg atc tca gtg gac tca aga agt gtc tcc ctc ctc ccc 999
Arg Arg Lys Leu Ile Ser Val Asp Ser Arg Ser Val Ser Leu Leu Pro
            175                 180                 185

ctg gag ttc cgc aaa gac tcg agc tat gag ctg cag gtg cgg gca ggg 1047
Leu Glu Phe Arg Lys Asp Ser Ser Tyr Glu Leu Gln Val Arg Ala Gly
        190                 195                 200

ccc atg cct ggc tcc tcc tac cag ggg acc tgg agt gaa tgg agt gac 1095
Pro Met Pro Gly Ser Ser Tyr Gln Gly Thr Trp Ser Glu Trp Ser Asp
    205                 210                 215

ccg gtc atc ttt cag acc cag tca gag gag tta aag gaa ggc tgg aac 1143
Pro Val Ile Phe Gln Thr Gln Ser Glu Glu Leu Lys Glu Gly Trp Asn
220                 225                 230                 235

cct cac ctg ctg ctt ctc ctc ctg ctt gtc ata gtc ttc att cct gcc 1191
Pro His Leu Leu Leu Leu Leu Leu Leu Val Ile Val Phe Ile Pro Ala
                240                 245                 250

ttc tgg agc ctg aag acc cat cca ttg tgg agg cta tgg aag aag ata 1239
Phe Trp Ser Leu Lys Thr His Pro Leu Trp Arg Leu Trp Lys Lys Ile
            255                 260                 265

tgg gcc gtc ccc agc cct gag cgg ttc ttc atg ccc ctg tac aag ggc 1287
Trp Ala Val Pro Ser Pro Glu Arg Phe Phe Met Pro Leu Tyr Lys Gly
        270                 275                 280

tgc agc gga gac ttc aag aaa tgg gtg ggt gca ccc ttc act ggc tcc 1335
Cys Ser Gly Asp Phe Lys Lys Trp Val Gly Ala Pro Phe Thr Gly Ser
    285                 290                 295

agc ctg gag ctg gga ccc tgg agc cca gag gtg ccc tcc acc ctg gag 1383
Ser Leu Glu Leu Gly Pro Trp Ser Pro Glu Val Pro Ser Thr Leu Glu
300                 305                 310                 315

gtg tac agc tgc cac cca cca cgg agc ccg gcc aag agg ctg cag ctc 1431
Val Tyr Ser Cys His Pro Pro Arg Ser Pro Ala Lys Arg Leu Gln Leu
                320                 325                 330

acg gag cta caa gaa cca gca gag ctg gtg gag tct gac ggt gtg ccc 1479
Thr Glu Leu Gln Glu Pro Ala Glu Leu Val Glu Ser Asp Gly Val Pro
            335                 340                 345

aag ccc agc ttc tgg ccg aca gcc cag aac tcg ggg ggc tca gct tac 1527
Lys Pro Ser Phe Trp Pro Thr Ala Gln Asn Ser Gly Gly Ser Ala Tyr
        350                 355                 360

agt gag gag agg gat cgg cca tac ggc ctg gtg tcc att gac aca gtg 1575
Ser Glu Glu Arg Asp Arg Pro Tyr Gly Leu Val Ser Ile Asp Thr Val
    365                 370                 375

act gtg cta gat gca gag ggg cca tgc acc tgg ccc tgc agc tgt gag 1623
Thr Val Leu Asp Ala Glu Gly Pro Cys Thr Trp Pro Cys Ser Cys Glu
380                 385                 390                 395

gat gac ggc tac cca gcc ctg gac ctg gat gct ggc ctg gag ccc agc 1671
Asp Asp Gly Tyr Pro Ala Leu Asp Leu Asp Ala Gly Leu Glu Pro Ser
                400                 405                 410

cca ggc cta gag gac cca ctc ttg gat gca ggg acc aca gtc ctg tcc 1719
Pro Gly Leu Glu Asp Pro Leu Leu Asp Ala Gly Thr Thr Val Leu Ser
            415                 420                 425

tgt ggc tgt gtc tca gct ggc agc cct ggg cta gga ggg ccc ctg gga 1767
Cys Gly Cys Val Ser Ala Gly Ser Pro Gly Leu Gly Gly Pro Leu Gly
        430                 435                 440

agc ctc ctg gac aga cta aag cca ccc ctt gca gat ggg gag gac tgg 1815
Ser Leu Leu Asp Arg Leu Lys Pro Pro Leu Ala Asp Gly Glu Asp Trp
    445                 450                 455

gct ggg gga ctg ccc tgg ggt ggc cgg tca cct gga ggg gtc tca gag 1863
Ala Gly Gly Leu Pro Trp Gly Gly Arg Ser Pro Gly Gly Val Ser Glu
460                 465                 470                 475

agt gag gcg ggc tca ccc ctg gcc ggc ctg gat atg gac acg ttt gac 1911
Ser Glu Ala Gly Ser Pro Leu Ala Gly Leu Asp Met Asp Thr Phe Asp
                480                 485                 490

agt ggc ttt gtg tgc tct gac tgc agc agc cct gtg gag tgt gac ttc 1959
Ser Gly Phe Val Cys Ser Asp Cys Ser Ser Pro Val Glu Cys Asp Phe
            495                 500                 505

acc agc ccc ggg gac gaa gga ccc ccc cgg agc tac ctc cgc cag tgg 2007

Thr Ser Pro Gly Asp Glu Gly Pro Pro Arg Ser Tyr Leu Arg Gln Trp
        510                 515                 520

gtg gtc att cct ccg cca ctt tcg agc cct gga ccc cag gcc agc      2052
Val Val Ile Pro Pro Pro Leu Ser Ser Pro Gly Pro Gln Ala Ser
    525                 530                 535

taatgaggct gactggatgt ccagagctgg ccaggccact gggccctgag ccagaaaaaa 2112

aaa                                                               2115

<210> 23
<211> 411
<212> DNA
<213> Homo sapiens

<220>
<221> 3'UTR
<222> (1)..(411)

<400> 23
taatgaggct gactggatgt ccagagctgg ccaggccact gggccctgag ccagagacaa 60

ggtcacctgg gctgtgatgt gaagacacct gcagcctttg gtctcctgga tgggcctttg 120

agcctgatgt ttacagtgtc tgtgtgtgtg tgcatatgtg tgtgtgtgca tatgcatgtg 180

tgtgtgtgtg tgtgtcttag gtgcgcagtg gcatgtccac gtgtgtgtga ttgcacgtgc 240

ctgtgggcct gggataatgc ccatggtact ccatgcattc acctgccctg tgcatgtctg 300

gactcacgga gctcacccat gtgcacaagt gtgcacagta aacgtgtttg tggtcaacag 360

aaaaaaaaaa aaaaaaaaaa aaaaaaaaaa aaaaaaaaaa aaaaaaaaaa a          411

<210> 24
<211> 877
<212> DNA
<213> Homo sapiens

<220>
<221> 3'UTR
<222> (1)..(877)

<400> 24
taatgaggct gactggatgt ccagagctgg ccaggccact gggccctgag ccagagacaa 60

ggtcacctgg gctgtgatgt gaagacacct gcagcctttg gtctcctgga tgggcctttg 120

agcctgatgt ttacagtgtc tgtgtgtgtg tgtgcatatg tgtgtgtgtg catatgcatg 180

tgtgtgtgtg tgtgtgtctt aggtgcgcag tggcatgtcc acgtgtgtgt gtgattgcac 240

gtgcctgtgg gcctgggata atgcccatgg tactccatgc attcacctgc cctgtgcatg 300

tctggactca cggagctcac ccatgtgcac aagtgtgcac agtaaacgtg tttgtggtca 360

acagatgaca acagccgtcc tccctcctag ggtcttgtgt tgcaagttgg tccacagcat 420

ctccggggct ttgtgggatc agggcattgc ctgtgactga ggcggagccc agccctccag 480

cgtctgcctc caggagctgc aagaagtcca tattgttcct tatcacctgc caacaggaag 540

cgaaagggga tggagtgagc ccatggtgac ctcgggaatg gcaatttttt gggcggcccc 600

tggacgaagg tctgaatccc gactctgata ccttctggct gtgctacctg agccaagtcg 660

cctcccctct ctgggctaga gtttccttat ccagacagtg gggaaggcat gacacacctg 720

ggggaaattg gcgatgtcac ccgtgtacgg tacgcagccc agagcagacc ctcaataaac 780

gtcagcttcc ttccttctgc ggccagagcc gaggcgggcg ggggtgagaa catcaatcgt 840

cagcgacaaa aaaaaaaaaa aaaaaaaaaa aaaaaaa 877

<210> 25
<211> 2791
<212> DNA
<213> Homo sapiens

<220>
<221> 3'UTR
<222> (1)..(2791)

<400> 25
taatgaggct gactggatgt ccagagctgg ccaggccact gggccctgag ccagagacaa 60

ggtcacctgg gctgtgatgt gaagacacct gcagcctttg gtctcctgga tgggcctttg 120

agcctgatgt ttacagtgtc tgtgtgtgtg tgtgcatatg tgtgtgtgtg catatgcatg 180

tgtgtgtgtg tgtgtgtctt aggtgcgcag tggcatgtcc acgtgtgtgt gtgattgcac 240

gtgcctgtgg gcctgggata atgcccatgg tactccatgc attcacctgc cctgtgcatg 300

tctggactca cggagctcac ccatgtgcac aagtgtgcac agtaaacgtg tttgtggtca 360

acagatgaca acagccgtcc tccctcctag ggtcttgtgt tgcaagttgg tccacagcat 420

ctccggggct ttgtgggatc agggcattgc ctgtgactga ggcggagccc agccctccag 480

cgtctgcctc caggagctgc aagaagtcca tattgttcct tatcacctgc caacaggaag 540

cgaaagggga tggagtgagc ccatggtgac ctcgggaatg gcaatttttt gggcggcccc 600

tggacgaagg tctgaatccc gactctgata ccttctggct gtgctacctg agccaagtcg 660

cctcccctct ctgggctaga gtttccttat ccagacagtg gggaaggcat gacacacctg 720

ggggaaattg gcgatgtcac ccgtgtacgg tacgcagccc agagcagacc ctcaataaac 780

gtcagcttcc ttccttctgc ggccagagcc gaggcgggcg ggggtgagaa catcaatcgt 840

cagcgacagc ctgggcaccc gcggggccgt cccgcctgca gagggccact cgggggggtt 900

tccaggctta aaatcagtcc gtttcgtctc ttggaaacag ctccccacca accaagattt 960

ctttttctaa cttctgctac taagttttta aaaattccct ttatgcaccc aagagatatt 1020

tattaaacac caattacgta gcaggccatg gctcatggga cccacccccc gtggcactca 1080

tggagggggc tgcaggttgg aactatgcag tgtgctccgg ccacacatcc tgctgggccc 1140

cctaccctgc cccaattcaa tcctgccaat aaatcctgtc ttatttgttc atcctggaga 1200

attgaaggga ggtcaagttg tttgtcaatg atttgtcaga gaacctgttg aaatgtgaat 1260

taagaagcta agaaaatatt tcttagcaac attttctttt tctttttttt tttttctttt 1320

tgagacagag tctcactctc gtcgcccagg ctggaatgca gtggtgcgat ctcggctctc 1380

tgcaacctct gtctcccggg ttcaagcgat ttcctgcgtc agccccagag tagctggaat 1440

tacaggcaca caccaccacg cctggctaat ttttgtattt ttagtagagc tggggccacc 1500

ctggcccggc cccgtcttcc tccccaaagg tcagactgca ggctgcaggg ctgtgctgga 1560

ggagccagct ctagctcacc catgcttttg caacagggtc gggttggaag tcagcacagg 1620

tcagtcctgc ggaaggttcc ttcgtgactc atctgtgaag tggggtggtt gggagaggta 1680

gctgagagaa tgcatgagag tcctcggtgc ctggcaggag gctggaaggt tctagaacac 1740

tgatggttat aagagtggga ctgtgagcct gggatcgggg ggtgtgagac ttggatggga 1800

gcacaagagt ggaaacacag cttctgcacg gagcaggcgc agccctcaac accccgtgca 1860

cctgcaccct agggactctt gggtccagat gtgctgtggt tttcacacct tcttgggggc 1920

aacaggttcc aggagccacc tgtgggtgcc acctgagcca caggctccca ggaaagcagc 1980

acagctctcc tgcacccaga gcttgctggg tggcggaggg gaacacagat ggttggggaa 2040

ggcctgaggc cagattgggg gactctggac tggggcagat gaggctcctc agaatcccac 2100

ctttgaaggg aactcagctt ataaacacag aggagcaaag ttggagggcc gggcgtagtg 2160

gctcacacct gtgatctcag cactttggga ggccaaggaa ggtggatcac ttgaggccag 2220

gagttcgaga ccagcctggg caacatagca aggccccatc tctacaaaaa ttattatttt 2280

ttaaaaaaat tagccaggtg tggtggtgct tgcctatagt cccagctact cgggaggcta 2340

aggtgggagg atcgctggag cccaggaatt tgaggctgca gtgagctgtg attacaccgt 2400

tgcactccag cctgggtcac agatcaagac cctgtctctt aaaaataaaa gttggagaca 2460

agagctggct cacctgaaag gagggattag taggtaggag ggtggatgga ggatggatgg 2520

atgtgtgggt ggataggaag atggtattaa gttggtgcaa aagtctttga tattactctt 2580

aatggcttta ataaaaagct tgaaggaaga atgattggtt ggatagacag agataaatgc 2640

atactggaaa caaagataaa gataaaacac aagttatacc aggccagcaa ctctattttg 2700

ttcactgcct ttagtcccag cctggcacat agtaggcact caataaagcc tgatttgtag 2760

caaaaaaaaa aaaaaaaaaa aaaaaaaaaa a 2791

<210> 26
<211> 907
<212> DNA
<213> Homo sapiens

<220>
<221> 3'UTR
<222> (1)..(907)

<400> 26
tgagctgcag gtgcgggcag ggcccatgcc tggctcctcc taccagggga cctggagtga 60

atggagtgac ccggtcatct ttcagaccca gtcagaggag ttaaaggaag gctggaaccc 120

tcacctgctg cttctcctcc tgcttgtcat agtcttcatt cctgccttct ggagcctgaa 180

gacccatcca ttgtggaggc tatggaagaa gatatgggcc gtccccagcc ctgagcggtt 240

cttcatgccc ctgtacaagg gctgcagcgg agacttcaag aaatgggtgg gtgcaccctt 300

cactggctcc agcctggagc tgggaccctg gagcccagag gtgccctcca ccctggaggt 360

gtacagctgc cacccaccca gcagccctgt ggagtgtgac ttcaccagcc ccggggacga 420

aggacccccc cggagctacc tccgccagtg ggtggtcatt cctccgccac tttcgagccc 480

tggaccccag gccagctaat gaggctgact ggatgtccag agctggccag gccactgggc 540

cctgagccag agacaaggtc acctgggctg tgatgtgaag acacctgcag cctttggtct 600

cctggatggg cctttgagcc tgatgtttac agtgtctgtg tgtgtgtgca tatgtgtgtg 660

tgtgcatatg catgtgtgtg tgtgtgtgtg tcttaggtgc gcagtggcat gtccacgtgt 720

gtgtgattgc acgtgcctgt gggcctggga taatgcccat ggtactccat gcattcacct 780

gccctgtgca tgtctggact cacggagctc acccatgtgc acaagtgtgc acagtaaacg 840

tgtttgtggt caacagaaaa aaaaaaaaaa aaaaaaaaaa aaaaaaaaaa aaaaaaaaaa 900

aaaaaaa 907

<210> 27
<211> 3818
<212> DNA
<213> Homo sapiens

<220>
<221> 3'UTR
<222> (1)..(3818)

<400> 27
   tgagctgcag gtgcgggcag ggcccatgcc tggctcctcc taccagggga cctggagtga 60
   atggagtgac ccggtcatct ttcagaccca gtcagaggag ttaaaggaag gctggaaccc 120
   tcacctgctg cttctcctcc tgcttgtcat agtcttcatt cctgccttct ggagcctgaa 180
   gacccatcca ttgtggaggc tatggaagaa gatatgggcc gtccccagcc ctgagcggtt 240
   cttcatgccc ctgtacaagg gctgcagcgg agacttcaag aaatgggtgg gtgcaccctt 300
   cactggctcc agcctggagc tgggaccctg gagcccagag gtgccctcca ccctggaggt 360
   gtacagctgc cacccaccac ggagcccggc caagaggctg cagctcacgg agctacaaga 420
   accagcagag ctggtggagt ctgacggtgt gcccaagccc agcttctggc cgacagccca 480
   gaactcgggg ggctcagctt acagtgagga gagggatcgg ccatacggcc tggtgtccat 540
   tgacacagtg actgtgctag atgcagaggg gccatgcacc tggccctgca gctgtgagga 600
   tgacggctac ccagccctgg acctggatgc tggcctggag cccagcccag gcctagagga 660
   cccactcttg gatgcaggga ccacagtcct gtcctgtggc tgtgtctcag ctggcagccc 720
   tgggctagga gggcccctgg gaagcctcct ggacagacta aagccacccc ttgcagatgg 780
   ggaggactgg gctgggggac tgccctgggg tggccggtca cctggagggg tctcagagag 840
   tgaggcgggc tcacccctgg ccggcctgga tatggacacg tttgacagtg gctttgtggg 900
   ctctgactgc agcagccctg tggagtgtga cttcaccagc cccggggacg aaggaccccc 960
   ccggagctac ctccgccagt gggtggtcat tcctccgcca ctttcgagcc ctggacccca 1020
   ggccagctaa tgaggctgac tggatgtcca gagctggcca ggccactggg ccctgagcca 1080
   gagacaaggt cacctgggct gtgatgtgaa gacacctgca gcctttggtc tcctggatgg 1140
   gcctttgagc ctgatgttta cagtgtctgt gtgtgtgtgt gcatatgtgt gtgtgtgcat 1200
   atgcatgtgt gtgtgtgtgt gtgtcttagg tgcgcagtgg catgtccacg tgtgtgtgtg 1260
   attgcacgtg cctgtgggcc tgggataatg cccatggtac tccatgcatt cacctgccct 1320
   gtgcatgtct ggactcacgg agctcaccca tgtgcacaag tgtgcacagt aaacgtgttt 1380
   gtggtcaaca gatgacaaca gccgtcctcc ctcctagggt cttgtgttgc aagttggtcc 1440
   acagcatctc cggggctttg tgggatcagg gcattgcctg tgactgaggc ggagcccagc 1500
   cctccagcgt ctgcctccag gagctgcaag aagtccatat tgttccttat cacctgccaa 1560
   caggaagcga aaggggatgg agtgagccca tggtgacctc gggaatggca attttttggg 1620
   cggcccctgg acgaaggtct gaatcccgac tctgatacct tctggctgtg ctacctgagc 1680
   caagtcgcct cccctctctg ggctagagtt tccttatcca gacagtgggg aaggcatgac 1740
   acacctgggg gaaattggcg atgtcacccg tgtacggtac gcagcccaga gcagaccctc 1800
   aataaacgtc agcttccttc cttctgcggc cagagccgag gcgggcgggg gtgagaacat 1860
   caatcgtcag cgacagcctg ggcacccgcg gggccgtccc gcctgcagag ggccactcgg 1920
   gggggtttcc aggcttaaaa tcagtccgtt tcgtctcttg gaaacagctc cccaccaacc 1980
   aagatttctt tttctaactt ctgctactaa gtttttaaaa attcccttta tgcacccaag 2040
   agatatttat taaacaccaa ttacgtagca ggccatggct catgggaccc accccccgtg 2100
   gcactcatgg agggggctgc aggttggaac tatgcagtgt gctccggcca cacatcctgc 2160
   tgggccccct accctgcccc aattcaatcc tgccaataaa tcctgtctta tttgttcatc 2220
   ctggagaatt gaagggaggt caagttgttt gtcaatgatt tgtcagagaa cctgttgaaa 2280
   tgtgaattaa gaagctaaga aaatatttct tagcaacatt ttctttttct tttttttttt 2340
   tttcttttga gacagagtct cactctcgtc gcccaggctg gaatgcagtg gtgcgatctc 2400
   ggctctctgc aacctctgtc tcccgggttc aagcgatttc ctgcgtcagc cccagagtag 2460
   ctggaattac aggcacacac caccacgcct ggctaatttt tgtattttta gtagagctgg 2520
   ggccaccctg gcccggcccc gtcttcctcc ccaaaggtca gactgcaggc tgcagggctg 2580
   tgctggagga gccagctcta gctcacccat gcttttgcaa cagggtcggg ttggaagtca 2640
   gcacaggtca gtcctgcgga aggttccttc gtgactcatc tgtgaagtgg ggtggttggg 2700
   agaggtagct gagagaatgc atgagagtcc tcggtgcctg gcaggaggct ggaaggttct 2760
   agaacactga tggttataag agtgggactg tgagcctggg atcggggggt gtgagacttg 2820
   gatgggagca caagagtgga aacacagctt ctgcacggag caggcgcagc cctcaacacc 2880
   ccgtgcacct gcaccctagg gactcttggg tccagatgtg ctgtggtttt cacaccttct 2940
   tgggggcaac aggttccagg agccacctgt gggtgccacc tgagccacag gctcccagga 3000
   aagcagcaca gctctcctgc acccagagct tgctgggtgg cggaggggaa cacagatggt 3060
   tggggaaggc ctgaggccag attgggggac tctggactgg ggcagatgag gctcctcaga 3120
   atcccacctt tgaagggaac tcagcttata aacacagagg agcaaagttg gagggccggg 3180
   cgtagtggct cacacctgtg atctcagcac tttgggaggc caaggaaggt ggatcacttg 3240
   aggccaggag ttcgagacca gcctgggcaa catagcaagg ccccatctct acaaaaatta 3300
   ttatttttta aaaaaattag ccaggtgtgg tggtgcttgc ctatagtccc agctactcgg 3360
   gaggctaagg tgggaggatc gctggagccc aggaatttga ggctgcagtg agctgtgatt 3420
   acaccgttgc actccagcct gggtcacaga tcaagaccct gtctcttaaa aataaaagtt 3480
   ggagacaaga gctggctcac ctgaaaggag ggattagtag gtaggagggt ggatggagga 3540
   tggatggatg tgtgggtgga taggaagatg gtattaagtt ggtgcaaaag tctttgatat 3600
   tactcttaat ggctttaata aaaagcttga aggaagaatg attggttgga tagacagaga 3660
   taaatgcata ctggaaacaa agataaagat aaaacacaag ttataccagg ccagcaactc 3720
   tattttgttc actgccttta gtcccagcct ggcacatagt aggcactcaa taaagcctga 3780
   tttgtagcaa aaaaaaaaaa aaaaaaaaaa aaaaaaaa 3818

<210> 28
<211> 330
<212> DNA
<213> Mus musculus

<220>
<223> primer sequence(1-30,301-330)
      mouse cDNA sequence(31-300)

<400> 28
ccggctcccc ctttcaacgt gactgtgacc ttctcaggac agtataatat ctccaggcgc 60

tcagattacg aagaccctgc cttctacatg ctgaagggca agcttcagta tgagctgcag 120

tacaggaacc ggggagaccc ctgggctgtg agtccgagga gaaagctgat ctcagtggac 180

tcaagaagtg tctccctcct ccccctggag ttccgcaaag actcgagcta tgagctgcag 240

gtgcgggcag ggcccatgcc tggctcctcc taccagggga cctggagtga atggagtgac 300

ccggtcatct ttcagaccca gtcagagggt                                  330


<210> 29
<211> 30
<212> DNA
<213> Artificial Sequence

<220>
<223> Artificially Synthesized Primer Sequence

<400> 29
tccaggcgct cagattacga agaccctgcc                                  30


<210> 30
<211> 30
<212> DNA
<213> Artificial Sequence

<220>
<223> Artificially Synthesized Primer Sequence

<400> 30
ACTCCAGGTC CCCTGGTAGG AGGAGCCAGG                                  30

## Description

### Technical Field

The present invention relates to novel hemopoietin receptor proteins, the encoding genes, and methods of production and uses thereof.

### Background Art

A large number of cytokines are known as humoral factors that are involved in the proliferation/differentiation of various cells, or activation of differentiated mature cells, and also cell death. These cytokines have their own specific receptors, which are categorized into several families based on their structural similarities (Hilton D.J., in "Guidebook to Cytokines and Their Receptors" edited by Nicola N.A. (A Sambrook & Tooze Publication at Oxford University Press), 1994, p8-16).

Compared to similarities between receptors, primary-structure homology is quite low between cytokines, and a significant amino acid homology cannot be seen even among cytokine members that belong to the same receptor family. This explains the functional specificity of each cytokine, as well as similarities of cellular reactions induced by each cytokine.

Representative examples of the above-mentioned receptor families are the tyrosine kinase receptor family, hemopoietin receptor family, tumor necrosis factor (TNF) receptor family, and transforming growth factor β (TGF β) receptor family. Different signal transduction pathways have been reported to be involved in each of these families. Among these receptor families, many receptors of especially the hemopoietin receptor family are expressed in blood cells and immunocytes, and their ligands, cytokines, are often termed as hemopoietic factors or interleukins. Some of these hemopoietic factors or interleukins exist within blood and are thought to be involved in a systemic humoral regulation of hemopoietic or immune functions.

This contrasts with the belief that cytokines belonging to other families are often involved in only topical regulations. Some of these hemopoietins can be taken as hormone-like factors, and conversely, representative peptide hormones such as the growth hormone, prolactin, or leptin receptors also belong to the hemopoietin receptor family. Because of these hormone-like systemic regulatory features, it is anticipated that hemopoietin administration would be applied in the treatment of various diseases.

Among the large number of cytokines, those that are actually being clinically applied are, erythropoietin, G-CSF, GM-CSF, and IL-2. Combined with IL-11, LIF, and IL-12 that are being considered for clinical trials, and the above-mentioned peptide hormones such as growth hormone and prolactin, it can be envisaged that by searching among the above-mentioned various receptor families for a novel cytokine that binds to hemopoietin receptors, it is possible to find a cytokine that can be clinically applied with a higher efficiency.

As mentioned above, cytokine receptors have structural similarities between the family members. Using these similarities, many investigations are being carried out aiming at finding novel receptors. Regarding the tyrosine kinase receptor especially, many receptors have already been cloned using its highly conserved sequence at the catalytic site (Matthews W. et al., Cell, 1991, 65 (7) p1143-52). Compared to this, hemopoietin receptors do not have a tyrosine kinase-like enzyme activity domain in their cytoplasmic regions, and their signal transductions are known to be mediated through associations with other tyrosine kinase proteins existing freely in the cytoplasm.

Though the binding site on receptors associating with these cytoplasmic tyrosine kinases (JAK kinases) is conserved between family members, the homology is not very high (Murakami M. et al., Proc. Natl. Acad. Sci. USA, 1991, 88, 11349-11353). On one hand, the sequence that characterizes these hemopoietin receptors most well exists in the extracellular region, and especially the five amino acid Trp-Ser-Xaa-Trp-Ser (where Xaa is an arbitrary amino acid) motif is conserved in almost all of the hemopoietin receptors. Therefore, novel receptors are expected to be obtained by searching novel family members using this sequence. In fact, this approach has already identified the IL-11 receptor (Robb, L. et al., J. Biol. Chem., 1996, 271 (23) 13754-13761), leptin receptor (Gainsford T. et al., Proc. Natl. Acad. Sci. USA, 1996, 93 (25) p14564-8) and the IL-13 receptor (Hilton D.J. et al., Proc. Natl. Acad. Sci. USA, 1996, 93 (1) p497-501).

### Disclosure of the Invention

The present invention provides a novel hemopoietin receptor protein, and the encoding DNA. The present invention also provides, a vector into which the DNA has been inserted, a transformant harboring the DNA, and a method of producing a recombinant protein using the transformant. It also provides a method of screening a compound that binds to the protein.

Until now, the inventors have been trying to search for a novel receptor using an oligonucleotide encoding the Trp-Ser-Xaa-Trp-Ser motif as a probe by plaque hybridization, RT-PCR method, and so on. However, because of reasons such as the oligonucleotide tggag (t/c) nnntggag (t/c) (where n is an arbitrary nucleotide) that encodes the motif being short having just 15 nucleotides, and the g/c being high, it was extremely difficult to strictly select only those in which the 15 nucleotides have completely hybridized under the usual hybridization conditions.

Also, a similar sequence is contained within cDNA encoding proteins other than hemopoietin receptors, starting with various collagens that are thought to be widely distributed and also have high expression amounts, which makes the screening by the above-mentioned plaque hybridization and RT-PCR highly inefficient.

To solve these problems, and to estimate how many different hemopoietic receptor genes actually exist on the human genome, the inventors computer-searched sequences that completely coincided with each probe using all capable oligonucleotide sequences encoding the above-mentioned Trp-Ser-Xaa-Trp-Ser motif as probes.

Next, among the clones identified by the above search, the nucleotide sequence around the probe sequence of human genome-derived clones (cosmid, BAC, PAC) was converted to the amino acid sequence and compared with the amino acid sequence of known hemopoietin receptors to select human genes thought to encode hemopoietin receptor family members.

From the above search, two clones thought to be hemopoietin receptor genes were identified. One of these was the known GM-CSFβ receptor gene (derived from the 22q12.3-13.2 region of chromosome no. 22), and the other (BAC clone AC002303 derived from the 16p12 region of chromosome no. 16) was presumed to encode a novel hemopoietin receptor protein, and this human gene was named "NR8."

Next, the cDNA thought to encode NR8 was found within the human fetal liver cell cDNA library by RT-PCR using a specific primer designed based on the obtained nucleotide sequence. Furthermore, using this cDNA library as the template, the full-length cDNA NR8α encoding a transmembrane receptor comprising 361 amino acids was ultimately obtained by 5'-RACE method and 3'-RACE method.

In the primary structure of NR8α, a cysteine residue and a proline rich motif conserved between other family members, were well conserved in the extracellular region, and in the intracellular region, the Box 1 motif thought to be involved in signal transduction was well conserved, and therefore, NR8α was thought to be a typical hemopoietin receptor.

Furthermore, the inventors revealed the presence of two genes named NR8β and NR8γ as selective splicing products of NR8α.

The inventors next attempted the isolation of the mouse gene corresponding to NR8 gene. First, using an oligonucleotide primer designed within human NR8 cDNA sequence and a mouse brain cDNA library as the template, xenogeneic cross PCR cloning was done to isolate the mouse partial nucleotide sequence of the above receptor. Furthermore, based on the obtained partial sequence, an oligonucleotide primer was designed, and using this, the inventors succeeded in isolating the full-length ORF of the mouse homologous gene corresponding to NR8 by the 5'-RACE method and 3'-RACE method. As a result of determining the whole nucleotide sequence of the obtained cDNA clone, alike NR8, the presence of mouse NR8γ encoding a transmembrane receptor protein comprising 538 amino acids, and mouse NR8β encoding a secretory, soluble receptor-like protein comprising 144 amino acids were confirmed by the difference of transcripts derived from the splice variant. When the amino acid sequences encoded by these receptor genes were compared between human and mouse, a high homology of 98.9% was observed for NR8γ, and on the other hand, a homology of 97.2% was seen for NR8β as well. Furthermore, the inventors succeeded in isolating the objective positive clones by plaque screening against a mouse genomic DNA library using the obtained mouse NR8β cDNA fragment as the probe.

Therefore, the present invention provides:
(1) a protein comprising the amino acid sequence from the 1^{st} amino acid Met to the 361^{st} amino acid Ser of SEQ ID NO: 1, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 361^{st} amino acid Ser of SEQ ID NO: 1;
(2) a protein comprising the amino acid sequence from the 1^{st} amino acid Met to the 144^{th} amino acid Leu of SEQ ID NO: 3, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 144^{th} amino acid Leu of SEQ ID NO: 3;
(3) a protein comprising the amino acid sequence from the 1^{st} amino acid Met to the 237^{th} amino acid Ser of SEQ ID NO: 5, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 237^{th} amino acid Ser of SEQ ID NO: 5;
(4) a protein comprising the amino acid sequence from the 1^{st} amino acid Met to the 538^{th} amino acid Ser of SEQ ID NO: 7, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 538^{th} amino acid Ser of SEQ ID NO: 7;
(5) a protein comprising the amino acid sequence from the 1^{st} amino acid Met to the 144^{th} amino acid Leu of SEQ ID NO: 19, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 144^{th} amino acid Leu of SEQ ID NO: 19;
(6) a protein comprising the amino acid sequence from the 1^{st} amino acid Met to the 538^{th} amino acid Ser of SEQ ID NO: 21, or a protein comprising a modified amino acid sequence of said amino acid sequence in which one or more amino acids have been deleted, added, and/or substituted with another amino acid, and being functionally equivalent to the protein comprising the amino acid sequence from the 1^{st} amino acid Met to the 583^{th} amino acid Ser of SEQ ID NO: 21;
(7) a protein encoded by a DNA hybridizing to a DNA comprising the nucleotide sequence of SEQ ID NO: 2, said protein being functionally equivalent to a protein comprising the amino acid sequence from the 1^{st} amino acid Met to the 361^{st} amino acid Ser of SEQ ID NO: 1;
(8) a protein encoded by a DNA hybridizing to a DNA comprising the nucleotide sequence of SEQ ID NO: 4, said protein being functionally equivalent to a protein comprising the amino acid sequence from the 1^{st} amino acid Met to the 144^{th} amino acid Leu of SEQ ID NO: 3;
(9) a protein encoded by a DNA hybridizing to a DNA comprising the nucleotide sequence of SEQ ID NO: 6, said protein being functionally equivalent to a protein comprising the amino acid sequence from the 1^{st} amino acid Met to the 237^{th} amino acid Ser of SEQ ID NO: 5;
(10) a protein encoded by a DNA hybridizing to a DNA comprising the nucleotide sequence of SEQ ID NO: 8, said protein being functionally equivalent to a protein comprising the amino acid sequence from the 1^{st} amino acid Met to the 538^{th} amino acid Ser of SEQ ID NO: 7;
(11) a protein encoded by a DNA hybridizing to a DNA comprising the nucleotide sequence of SEQ ID NO: 20, said protein being functionally equivalent to a protein comprising the amino acid sequence from the 1^{st} amino acid Met to the 144^{th} amino acid Leu of SEQ ID NO: 19;
(12) a protein encoded by a DNA hybridizing to a DNA comprising the nucleotide sequence of SEQ ID NO: 22, said protein being functionally equivalent to a protein comprising the amino acid sequence from the 1^{st} amino acid Met to the 538^{th} amino acid Ser of SEQ ID NO: 21;
(13) a fusion protein comprising the protein of any one of (1) to (12) and another peptide or polypeptide;
(14) a DNA encoding the protein of any one of (1) to (13);
(15) a vector comprising the DNA of (14);
(16) a transformant harboring the DNA of (14) in an expressible manner;
(17) a method of producing the protein of any one of (1) to (13), comprising the step of culturing the transformant of (16);
(18) a method of screening a compound that binds to the protein of any one of (1) to (13) comprising the steps of,
   (a) contacting a test sample with the protein of any one of (1) to (13), and
   (b) selecting a compound that comprises an activity to bind to the protein of any one of (1) to (13);
(19) an antibody that specifically binds to the protein of any one of (1) to (12);
(20) a method of detecting or measuring the protein of any one of (1) to (13) comprising the steps of contacting a test sample presumed to contain said protein with the antibody of (19), and detecting or measuring the formation of the immune complex between the antibody and the protein; and
(21) a DNA specifically hybridizing to a DNA comprising the nucleotide sequence of any one of SEQ ID NOs: 2, 4, 6, 8, 20, and 22 to 27, and comprising at least 15 nucleotides.

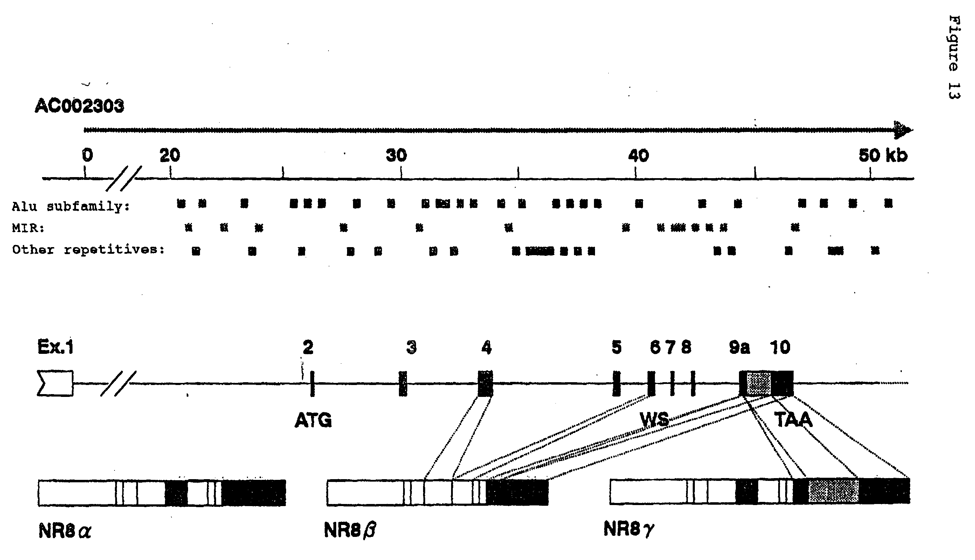

The present invention relates to the novel hemopoietin receptor "NR8." 5'-RACE and 3'-RACE analyses, NR8 genome sequence analysis, and plaque screening analysis revealed the presence of NR8α, NR8β, and NR8γ. The structures of these NR8 genes are shown in Fig. 13. Among the NR8 genes, NR8β is an alternative splicing product lacking the 5^{th} exon, and can encode two different proteins, a soluble protein in which the CDS ends with a stop codon on the 6^{th} exon that results from a frame shift following direct coupling to the 4^{th} exon, and a membrane-bound protein lacking the signal sequence starting from the ATG upon the 4^{th} exon.

Since the soluble protein comprises the same sequence as NR8α up to the 4^{th} exon, it may function as a soluble receptor. On the other hand, NR8γ encodes a protein containing a 177 amino acid insertion derived from the NR8 9^{th} intron close to the C terminus of the NR8α as a result of selective splicing.

Both NR8α and NR8γ encode transmembrane-type hemopoietin receptors. Among the sequences conserved between other hemopoietin receptors that are thought to be involved in signal transduction, a motif resembling Box 1 exists in the intracellular domain of NR8α and NR8γ adjacent to the cell membrane. Though low in the degree of conservation, a sequence that is similar to Box 2 also exists, and therefore, NR8 is thought to be a type of receptor in which the signal is transduced by a homodimer.

The amino acid sequences of the NR8 proteins included in the proteins of the present invention are shown in SEQ ID No: 1 (NR8α), SEQ ID NO: 3 (soluble NR8β), SEQ ID NO: 5 (membrane-bound NR8β), and SEQ ID NO: 7 (NR8γ), and the nucleotide sequences of cDNA encoding these proteins are shown in SEQ ID NO: 2, SEQ ID NO: 4, SEQ ID NO: 6, and SEQ ID NO: 8, respectively.

Northern blot analysis for the spleen, thymus, peripheral leucocytes, and lung showed two to three bands in the 5kb and 3 to 4kb regions. Similar sized bands were observed for cell lines HL60 and Raji also, but no expression was seen for other tumor cell lines (HeLa, SW480, A549, G361) and leukemia cell lines (K562, MOLT4).

The above results suggest that NR8 is specifically expressed on hemopoietic cell lines, especially on granulocytic lines, and B cell lines.

The above NR8 protein is expected to be applied in medicine. NR8 is expressed in fetal liver, spleen, thymus, and some leukemic cell lines, suggesting the possibility that it might be a receptor of an unknown hemopoietic factor. Therefore, NR8 protein would be a useful material for obtaining this unknown hemopoietic factor.

Furthermore, it is possible that NR8 is specifically expressed in limited cell populations within these hemopoietic tissues, and therefore, anti NR8 antibody may be useful as a means of separating these cell populations. Thus separated cell populations can be applied for cell transplant therapy. Anti NR8 antibody is also expected to be applied for the diagnosis and treatment of leukemic diseases represented by leukemia.

On the other hand, the soluble protein including the extracellular domain of NR8 protein, or NR8β, a splicing variant of NR8, may be applied as a decoy-type receptor that is an inhibitor of the NR8 ligand, and is anticipated to be applied in the treatment of diseases in which NR8 is involved, starting with leukemia.

The inventors also isolated mouse NR8 cDNA corresponding to the human-derived NR8 cDNA above-mentioned, by using the xenogeneic cross PCR cloning method. The amino acid sequences of the proteins named mouse NR8, which are included in the protein of the present invention are shown in SEQ ID NO: 19 (soluble mouse NR8β) and SEQ ID NO: 21 (mouse NR8γ), and the nucleotide sequences of the cDNA encoding these proteins are shown in SEQ ID NO: 20 and SEQ ID NO: 22, respectively.

As a result of structural analysis of the obtained mouse cDNA clones, alike human-derived NR8, the presence of mouse NR8γ encoding a transmembrane receptor protein comprising 538 amino acids and mouse NR8β encoding a secretory soluble receptor-like protein comprising 144 amino acids which were confirmed by the difference of transcripts derived the splice variant, was confirmed. When the amino acid sequences encoded by these receptor genes were compared between human and mouse, a high homology of 98.9% was observed for NR8γ, while a homology of 97.2% was seen for NR8β as well.

Northern blot analysis and RT-PCR analysis showed that although there were deviations in expression levels, mouse NR8 gene expression was seen in all organs analyzed, and seemed to be widely distributed compared to human NR8, for which a strong expression was seen only in immunocompetent and hemopoietic tissues. This also suggests the possibility that molecular functions of mouse NR8 may span a broad range of physiological regulatory mechanisms of the body.

The present invention also encompasses a protein that is functionally equivalent to the above-mentioned human or mouse NR8 protein. Herein "functionally equivalent" means having an equivalent biological activity to the above-mentioned NR8 proteins. Hemopoietic factor receptor protein activity can be given as an example of a biological activity. Such proteins can be obtained by the method of introducing a mutation to the amino acid sequence of a protein. For example, site-specific mutagenesis using a synthetic oligonucleotide primer, can be used to introduce a desired mutation into the amino acid sequence of a protein (Kramer, W. and Fritz, H.J., Methods in Enzymol., 1987, 154, 350-367). This could also be done by a PCR-mediated site-specific mutagenesis system (GIBCO-BRL). Using these methods, the amino acid sequence of SEQ ID NO: 1, SEQ ID NO: 3, SEQ ID NO: 5, SEQ ID NO: 7, SEQ ID NO: 19, or SEQ ID NO: 21 can be modified to obtain a protein functionally equivalent to the NR8 protein,in which one or more amino acids in the amino acid sequence of the protein have been deleted, added, and/or substituted by another amino acid without affecting the biological activity of the protein.

As a protein functionally equivalent to the NR8 protein of the invention, the following are given: one in which one or two or more, preferably, two to 30, more preferably, two to ten amino acids are deleted in any one of the amino acid sequences of SEQ ID NO: 1, SEQ ID NO: 3, SEQ ID NO: 5, SEQ ID NO: 7, SEQ ID NO: 19, or SEQ ID NO: 21; one in which one or two or more, preferably, two to 30, more preferably, two to ten amino acids have been added into any one of the amino acid sequences of SEQ ID NO: 1, SEQ ID NO: 3, SEQ ID NO: 5, or SEQ ID NO: 7; or one in which one or two or more, preferably, two to 30, more preferably, two to ten amino acids have been substituted with other amino acids in any one of the amino acid sequences of SEQ ID NO: 1, SEQ ID NO: 3, SEQ ID NO: 5, or SEQ ID NO: 7.

It is already known that a protein comprising a modified amino acid sequence of a certain amino acid sequence in which one or more amino acid residues have been deleted, added, and/or substituted with another amino acid, still maintains its biological activity (Mark, D. F. et al., Proc. Natl. Acad. Sci. USA, 1984, 81, 5662-5666; Zoller, M. J. & Smith, M., Nucleic Acids Research, 1982, 10, 6487-6500; Wang, A. et al., Science, 224, 1431-1433; Dalbadie-McFarland, G. et al., Proc. Natl. Acad. Sci. USA, 1982, 79, 6409-6413).

For example, a fusion protein can be given as a protein in which one or more amino acid residues have been added to the NR8 protein of the present invention. A fusion protein is made by fusing the NR8 protein of the present invention with another peptide or protein and is encompassed in the present invention. A fusion protein can be prepared by ligating DNA encoding the NR8 protein of the present invention with DNA encoding another peptide or protein so as the frames match, introducing this into an expression vector, and expressing the fusion gene in a host. Methods commonly known can be used for preparing such a fusion gene. There is no restriction as to the other peptide or protein that is fused to the protein of this invention.

For example, FLAG (Hopp, T.P. et al., Biotechnology, 1988, 6, 1204-1210), 6x His constituting six histidine (His) residues, 10x His, Influenza agglutinin (HA), human c-myc fragment, VSV-GP fragment, p18HIV fragment, T7-tag, HSV-tag, E-tag, SV40T antigen fragment, lck tag, α-tubulin fragment, B-tag, Protein C fragment, and such well-known peptides can be used. Examples of proteins are, glutathione-S-transferase (GST), Influenza agglutinin (HA), immunoglobulin constant region, β-galactosidase, maltose-binding protein (MBP), etc. Commercially available DNAs encoding these may also be used to prepare fusion proteins.

The protein of the invention can also be encoded by a DNA that hybridizes under stringent conditions to a DNA comprising any one of the nucleotide sequences of SEQ ID NO: 2, SEQ ID NO: 4, SEQ ID NO: 6, SEQ ID NO: 8, SEQ ID NO: 20, and SEQ ID NO: 22 to 27. Such a protein also includes a protein functionally equivalent to the above-mentioned NR8 protein. Stringent conditions can be suitably selected by one skilled in the art, and for example, low-stringent conditions can be given. Low-stringent conditions are, for example, 42°C, 2x SSC, and 0.1% SDS, and preferably, 50°C, 2x SSC, and 0.1% SDS. More preferable are highly stringent conditions, for example, 65°C, 2x SSC, and 0.1% SDS. Under these conditions, the higher the temperature is raised, the higher the homology of the obtained DNA will be.

The present invention also includes a protein that is functionally equivalent to the above NR8 protein, which has also a homology with a protein comprising any one of the amino acid sequences of SEQ ID NO: 1, SEQ ID NO: 3, SEQ ID NO: 5, SEQ ID NO: 7, SEQ ID NO: 19, or SEQ ID NO: 21. A protein having a homology means, a protein having at least 70%, preferably at least 80%, more preferably at least 90%, even more preferably, at least 95% homology to any one of the amino acid sequences of SEQ ID NO: 1, SEQ ID NO: 3, SEQ ID NO: 5, and SEQ ID NO: 7. The homology of a protein can be determined by the algorithm in "Wilbur, W.J. and Lipman, D.J. Proc. Natl. Acad. Sci. USA, 1983, 80, 726-730."

In the protein of the invention, the amino acid sequence, molecular weight, isoelectric point, the presence or absence of the sugar chain, and its form differ according to the producing cells, host, or purification method described below. However, as long as the obtained, protein comprises a hemopoietic factor receptor protein activity, it is included in the present invention.

For example, if the protein of the present invention is expressed in prokaryotic cells such as *E. coli*, a methionine residue is added at the N-terminus of the amino acid sequence of the expressed protein. If the protein of the present invention is expressed in eukaryotic cells such as mammalian cells, the N-terminal signal sequence is removed. The protein of the present invention includes these proteins.

For example, as a result of analyzing the protein of the invention based on the method in "Von Heijne, G., Nucleic Acids Research, 1986, 14, 4683-4690," it was presumed that the signal sequence is from the 1^{st} Met to the 19^{th} Gly in the amino acid sequence of SEQ ID NO: 1. Therefore, the present invention encompasses a protein comprising the sequence from the 20^{th} Cys to 361^{st} Ser in the amino acid sequence of SEQ ID NO: 1.

To produce the protein of the invention, the obtained DNA is incorporated into an expression vector in a manner that the DNA is expressible under the regulation of an expression regulatory region, for example, an enhancer or promoter. Next, host cells are transformed by this expression vector to express the protein.

Specifically, the protein can be produced as follows. When mammalian cells are used, DNA comprising a commonly used useful promoter/enhancer, DNA encoding the protein of the invention, and the poly A signal that is functionally bound to the 3' side downstream of the protein-encoding DNA, or a vector containing it, is constructed. For example, as the promoter/enhancer, human cytomegalovirus immediate early promoter/enhancer can be given.

Also, as other promoters/enhancers that can be used for protein expression, viral promoters/enhancers of retroviruses, polyomaviruses, adenoviruses, simian virus 40 (SV40), and such, and promoters/enhancers derived from mammalian cells, such as that of human elongation factor 1α (HEF1α) can be used.

For example, a protein can be easily expressed by following the method of Mulligan et al. (Nature, 1979, 277, 108) when using the SV40 promoter/enhancer, and the method of Mizushima et al. (Nucleic Acids Res., 1990, 18, 5322) when using the HEF1α promoter/enhancer.

When using *E. coli*, well-used useful promotors, the signal sequence for polypeptide secretion, and genes to be expressed, may be functionally bound to express the desired gene. For example, lacZ promoter and araB promoter may be used as promotors. When using the lacZ promoter, the method of Ward et al. (Nature, 1098, 341, 544-546; FASEB J., 1992, 6, 2422-2427), and when using the araB promoter, the method of Better et al. (Science, 1988, 240, 1041-1043) may be followed.

When producing the protein into the periplasm of *E. coli*, the pelB (Lei, S. P. et al., J. Bacteriol., 1987, 169, 4379) signal sequence may be used as a protein secretion signal.

A replication origin derived from SV40, polyomavirus, adenovirus, bovine papilomavirus (BPV), and such may be used. To amplify gene copies in host cell lines, the expression vector may include an aminoglycoside transferase (APH) gene, thymidine kinase (TK) gene, *E.coli* xanthine guanine phosphoribosyl transferase (Ecogpt) gene, dihydrofolate reductase (dhfr) gene, and such as a selective marker.

The expression vector used to produce the protein of the invention may be any, as long as it's an expression vector that is suitably used for the present invention. Mammalian expression vectors, for example, pEF and pCDM8; insect-derived expression vectors, for example, pBacPAK8; plant-derived expression vectors, for example, pMH1 and pMH2; animal virus-derived expression vectors, for example, pHSV, pMV, and pAdexLcw; retrovirus-derived expression vectors, for example, pZIpneo; yeast-derived expression vectors, for example, pNV11 and SP-Q01; *Bacillus subtilis*-derived expression vectors, for example, pPL608 and pKTH50; *E. coli*-derived expression vectors, for example, pQE, pGEAPP, pGEMEAPP, and pMALp2 can be given as expression vectors of this invention.

Not only vectors that produce the protein of the invention *in vivo* and *in vitro*, but also those that are used for gene therapy of mammals, for example humans, are also included as vectors of the present invention.

When introducing the expression vector of the present invention constructed above into a host cell, well-known methods, for example the calcium phosphate method (Virology, 1973, 52, 456-467), electroporation (EMBO J., 1982, 1, 841-845), and such may be used.

In the present invention, an arbitrary production system may be used to produce the protein. *In vitro* and *in vivo* production systems are known as production systems for producing proteins. Production systems using eukaryotic cells and prokaryotic cells may be used as *in vitro* production systems.

When using eukaryotic cells, production systems using, for example, animal cells, plant cells, and fungal cells are known. As animal cells used, for example, mammalian cells such as CHO (J. Exp. Med., 1995, 108, 945), COS, myeloma, baby hamster kidney (BHK), HeLa, or Vero, amphibian cells such as *Xenopus* oocytes (Valle, et al., Nature, 1981, 291, 358-340), insect cells such as sf9, sf21, or Tn5, are known. As CHO cells, especially DHFR gene-deficient CHO cell, dhfr-CHO (Proc. Natl. Acad. Sci. USA, 1980, 77, 4216-4220), and CHO K-1 (Proc. Natl. Acad. Sci. USA, 1968, 60, 1275) can be suitably used.

*Nicotiana tabacum*-derived cells are well known as plant cells, and these can be callus cultured. As fungal cells, yeasts such as the *Saccharomyces* genus, for example, *Saccharomyces cerevisiae*, filamentous bacteria such as the *Aspergillus* genus, for example, *Aspergillus niger* are known.

Bacterial cells may be used as prokaryotic production systems. As bacterial cells, *E. coli* and *Bacillus subtilis* are known.

Proteins can be obtained by transforming these cells with the objective DNA, and culturing the transformed cells *in vitro* according to well-known methods. For example, DMEM, MEM, RPMI1640, and IMDM can be used as culture media. At that instance, fetal calf serum (FCS) and such serum supplements may be added in the above media, or a serum-free culture medium may be used. The pH is preferably about 6 to 8. Culture is usually done at about 30°C to 40°C, for about 15 to 200 hr, and medium changes, aeration, and stirring are done as necessary.

On the other hand, production systems using animals and plants may be given as *in vivo* production systems. The objective gene is introduced into the plant or animal, and the protein is produced within the plant or animal, and recovered. "Host" as used in the present invention encompasses such animals and plants as well.

When using animals, mammalian and insect production systems can be used. As mammals, goats, pigs, sheep, mice, and cattle may be used (Vicki Glaser, SPECTRUM Biotechnology Applications, 1993). Transgenic animals may also be used when using mammals.

For example, the objective DNA is inserted within a gene encoding a protein produced intrinsically into milk, such as goat β casein, to prepare a fusion gene. The DNA fragment containing the fusion gene is injected into a goat's embryo, and this embryo is implanted in a female goat. The protein is collected from the milk of the transgenic goats produced from the goat that received the embryo, and descendents thereof. To increase the amount of protein-containing milk produced from the transgenic goat, a suitable hormone/hormones may be given to the transgenic goats (Ebert, K.M. et al., Bio/Technology, 1994, 12, 699-702).

Silk worms may be used as insects. When using the silk worm, it is infected with a baculovirus to which the objective DNA has been inserted, and the desired protein is obtained from the body fluids of the silk worm (Susumu, M. et al., Nature, 1985, 315, 592-594).

When using plants, for example, tobacco can be used. In the case of tobacco, the objective DNA is inserted into a plant expression vector, for example pMON 530, and this vector is introduced into a bacterium such as *Agrobacterium tumefaciens*. This bacterium is infected to tobacco, for example *Nicotiana tabacum*, to obtain the desired polypeptide from tobacco leaves (Julian, K.-C. Ma et al., Eur. J. Immunol., 1994, 24, 131-138).

The thus-obtained protein of the invention is isolated from within and without cells, or from hosts, and can be purified as a substantially pure homogenous protein. The separation and purification of the protein is not limited to any specific method and can be done using ordinary separation and purification methods used to purify proteins. For example, chromatography, filtration, ultrafiltration, salting out, solvent precipitation, solvent extraction, distillation, immunoprecipitation, SDS-polyacrylamide gel electrophoresis, isoelectric focusing, dialysis, recrystalization, and such may be suitably selected, or combined to separate/purify the protein.

As chromatographies, for example, affinity chromatography, ion exchange chromatography, hydrophobic chromatography, gel filtration, reversed-phase chromatography, adsorption chromatography, and such can be exemplified (Strategies for Protein Purification and Characterization: A Laboratory Course Manual. Ed Daniel R. Marshak et al., Cold Spring Harbor Laboratory Press, 1996). These chromatographies can be done by liquid chromatography such as HPLC, FPLC, and the like. The present invention encompasses proteins highly purified by using such purification methods.

Proteins can be arbitrarily modified, or peptides may be partially excised by treating the proteins with appropriate modification enzymes prior to or after the purification. Trypsin, chymotrypsin, lysyl endopeptidase, protein kinase, glucosidase, and such are used as protein modification enzymes.

The present invention includes a partial peptide comprising the active center of a protein comprising any one of the amino acid sequences of SEQ ID NO: 1, SEQ ID NO: 3, SEQ ID NO: 5, SEQ ID NO: 7, SEQ ID NO: 19, and SEQ ID NO: 21. A partial peptide of the protein of the present invention is, for example, a partial peptide of the molecules of the protein, which contains one or more regions of the hydrophilic region and hydrophobic region presumed by hydrophobicity plot analysis. These partial peptides may contain the whole hydrophilic region or a part of it, and may contain the whole hydrophobic region or a part of it. For example, soluble proteins and proteins comprising extracellular regions of the protein of the invention, are also encompassed in the invention.

The partial peptides of the protein of the invention may be produced by genetic engineering techniques, well-known peptide synthesizing methods, or by excising the protein of the invention by a suitable peptidase. As peptide synthesizing methods, the solid-phase synthesizing method, and the liquid-phase synthesizing method may be used.

The present invention also relates to a DNA encoding the protein of the invention. A cDNA encoding the protein of the invention may be obtained by, for example, screening a human cDNA library using the probe described herein.

Using the obtained cDNA or cDNA fragment as a probe, cDNA can also be obtained from other cells, tissues, organs, or species by further screening cDNA libraries. cDNA libraries may be prepared by, for example, the method of Sambrook, J. et al., Molecular Cloning, Cold Spring Harbor Laboratory Press (1989), or commercially available cDNA libraries may be used.

By determining the nucleotide sequence of the obtained cDNA, the translation region encoded by it can be determined, and the amino acid sequence of the protein of the present invention can be obtained. Furthermore, genomic DNA can be isolated by screening the genomic DNA library using the obtained cDNA as a probe.

Specifically, this can be done as follows. First, mRNA is isolated from cells, tissues, and organs expressing the protein of the invention. For this mRNA isolation, whole RNA is prepared using well-known methods, for example, guanidine ultracentrifugation method (Chirgwin, J.M. et al., Biochemistry, 1979, 18, 5294-5299), the AGPC method (Chomczynski, P. and Sacchi, N., Anal. Biochem., 1987, 162, 156-159), and such, and purified using the mRNA Purification Kit (Pharmacia), etc. mRNA may be directly prepared using the QuickPrep mRNA Purification Kit (Pharmacia).

cDNA is synthesized using reverse transcriptase from the obtained mRNA. cDNA can be synthesized using the AMV Reverse Transcriptase First-strand cDNA Synthesis Kit (SEIKAGAKU CORPORATION), etc. Also, cDNA synthesis and amplification may also be done using the probe described herein by following the 5'-RACE method (Frohman, M.A. et al., Proc. Natl. Acad. Sci. U.S.A., 1988, 85, 8998-9002; Belyavsky, A. et al., Nucleic Acids Res., 1989, 17, 2919-2932) using the polymerase chain reaction (PCR) and the 5'-Ampli FINDER RACE KIT (Clontech).

The objective DNA fragment is prepared from the obtained PCR product and ligated with vector DNA. Thus, a recombination vector is created, introduced into *E.coli*, etc. and colonies are selected to prepare the desired recombination vector. The nucleotide sequence of the objective DNA may be verified by known methods, for example, the dideoxy nucleotide chain termination method.

In the DNA of the invention, a sequence with a higher expression efficiency can be designed by considering the codon usage frequency of hosts used for the expression (Grantham, R. et al., Nucleic Acids Research, 1981, 9, p43-p74). The DNA of the invention may also be modified using commercially available kits and known methods. For example, digestion by restriction enzymes, insertion of synthetic oligonucleotides and suitable DNA fragments, addition of linkers, insertion of a start codon (ATG) and/or stop codon (ATT, TGA, or TAG), and such can be given.

The DNA of the present invention encompasses DNA comprising the nucleotide sequence from the 441^{st} nucleotide A to the 1523^{rd} nucleotide C in the nucleotide sequence of SEQ ID NO: 2, DNA comprising the nucleotide sequence from the 441^{st} nucleotide A to the 872^{nd} nucleotide A in the nucleotide sequence of SEQ ID NO: 4, DNA comprising the nucleotide sequence from the 659^{th} nucleotide A to the 1368^{th} nucleotide C in the nucleotide sequence of SEQ ID NO: 6, DNA comprising the nucleotide sequence from the 441^{st} nucleotide A to the 2054^{th} nucleotide C in the nucleotide sequence of SEQ ID NO: 8, DNA comprising the nucleotide sequence from the 439^{th} nucleotide A to the 870^{th} nucleotide A in the nucleotide sequence of SEQ ID NO: 20, and DNA comprising the nucleotide sequence from the 439^{th} nucleotide A to the 2052^{nd} nucleotide C in the nucleotide sequence of SEQ ID NO: 22.

The DNA of the present invention encompasses DNA that hybridizes under stringent conditions to the DNA comprising any one of the nucleotide sequences of SEQ ID NO: 2, SEQ ID NO: 4, SEQ ID NO: 6, SEQ ID NO: 8, SEQ ID NO: 20, and SEQ ID NO: 22 to 27, which also includes a DNA encoding a protein functionally equivalent to the NR8 protein.

Stringent conditions can be suitably selected by one skilled in the art, and for example, low-stringent conditions can be given. Low-stringent conditions are, for example, 42°C, 2x SSC, and 0.1% SDS, and preferably 50°C, 2x SSC, and 0.1% SDS. More preferable are highly stringent conditions, for example, 65°C, 2x SSC, and 0.1% SDS. Under these conditions, the higher the temperature is raised, the higher the homology of the obtained DNA will be. The above DNA is preferably natural DNA such as cDNA and chromosomal DNA.

As shown in Examples, the mRNA of the gene hybridizing to cDNA encoding the protein of the invention was distributed in various human tissues. Therefore, the above-mentioned natural DNA may be, for example, genomic DNA and cDNA derived from tissues in which the mRNA that hybridizes to the cDNA encoding the protein of the invention is detected in Examples. The DNA encoding the protein of the invention may be cDNA, genomic DNA, or synthetic DNA.

The protein of the invention is useful in screening a compound that binds to it. Namely, the protein of the invention is used in the screening method that comprises the steps of contacting a test sample expected to contain a compound that binds to the protein of the invention with the protein of the invention, and selecting the compound that comprises an activity to bind to the protein of the invention.

As methods for screening a compound that comprises an activity to bind to the protein of the invention, numerous methods usually used by those skilled in the art can be employed. The protein of the invention that is used for the screening of the invention may be a recombinant, natural, or partial peptide. A compound comprising an activity to bind to the protein of the invention may be a protein comprising a binding activity, or it may be a chemically synthesized compound having a binding activity.

As a test sample that is used in the screening method of the present invention, for example, peptides, purified or crudely purified proteins, non-peptide compounds, synthetic compounds, microbial fermentation products, extracts of marine organisms, plant extracts, cell extracts, animal tissue extracts, and such can be given. These test samples may be novel compounds, or well-known compounds.

A protein that binds to the protein of the invention can be screened by, for example, using the West-western blotting method (Skolnik, E.Y. et al., Cell, 1991, 65, 83-90). cDNA is isolated from cells, tissues, and organs presumed to express the protein binding to the protein of the invention, this is inserted into phage vectors, for example, λgt11, ZAPII, and such, to make a cDNA library, expressed on a plate containing a culture medium, the proteins expressed are fixed on a filter, this filter is reacted with the labeled, purified protein of the invention, and plaques expressing the protein bound to the protein of the invention are detected by the labels. As methods to label the protein of the invention, the method that uses the binding ability of avidin and biotin, the method of using an antibody that specifically binds to the protein of the invention or the peptide or polypeptide fused to the protein of the invention, the method of using radioisotopes, or fluorescence, and such can be given.

A ligand that binds specifically to the protein of the invention can be screened by, preparing a chimeric receptor by ligating the extracellular domain of the protein of the invention with the intracellular domain containing the transmembrane domain of a hemopoietin receptor protein comprising a known signal transduction ability, expressing this chimeric receptor on the cell surface of a suitable cell line, preferably, a cell line that can survive and proliferate under the presence of a suitable growth factor (a growth factor-dependent cell line), and culturing the cell line by adding a material that is expected to contain various growth factors, cytokines, or hemopoietic factors. This method uses the fact that the above-mentioned growth factor-dependent cell line survives and proliferates only when a ligand that specifically binds to the extracellular domain of the protein of the invention exists within the test material. Known hemopoietic receptors are, for example, the thrombopoietin receptor, erythropoietin receptor, G-CSF receptor, gp130, etc. However, the partner of the chimeric receptor used in the screening of the invention is not limited to these known hemopoietic receptors, and any may be used as long as a structure needed for the signal transduction activity is contained in the cytoplasmic domain. Growth factor-dependent cell lines are for example, IL-3-dependent cell lines starting with BaF3 and FDC-P1.

As a ligand that specifically binds to the protein of the invention, the possibility of not only soluble proteins, but also cell membrane-binding proteins can be envisaged, though rare. In such cases, screening can be done by labeling the protein containing only the extracellular domain of the protein of the invention, or a fusion protein in which the partial sequence of another soluble protein has been added to this extracellular domain, and measuring the binding with cells expected to express the ligand. As examples of proteins containing only the extracellular domain of the protein of the invention, for example, a soluble receptor protein artificially made by inserting a stop codon to the N terminal side of the transmembrane domain, or NR8β soluble protein may be used. On the other hand, as a fusion protein in which the partial sequence of another soluble protein has been added to the extracellular domain of the protein of the invention, for example, proteins prepared by adding immunoglobulin Fc site, FLAG peptide, etc. to the C terminus of the extracellular domain can be used. These soluble labeled proteins can be used in the detection in the above-described West-western blotting method.

A protein that binds to the protein of the invention can be screened by using the two-hybrid system (Fields, S. and Sternglanz, R., Trends. Genet., 1994, 10, 286-292).

In the two-hybrid system, an expression vector containing DNA encoding the fusion protein between the protein of the invention and one subunit of a heterodimeric transcriptional regulatory factor, and an expression vector containing DNA made by ligating DNA encoding the other subunit of the heterodimeric transcriptional regulatory factor and a desired cDNA used as a test sample are introduced into cells and expressed. If the protein encoded by the cDNA binds with the protein of the invention and the transcriptional regulatory factor forms a heterodimer, a reporter gene constructed in the cell beforehand will be expressed. Therefore, a protein binding to the protein of the invention can be selected by detecting or measuring the expression level of the reporter gene.

Specifically, the DNA encoding the protein of the invention and the gene encoding the DNA binding domain of LexA are ligated so as the frames match to prepare an expression vector. Next, the desired cDNA and the gene encoding GAL4 transcription activation domain are ligated to prepare an expression vector.

Cells into which the HIS3 gene has been incorporated (the transcription of HIS3 gene is regulated by the promoter having a LexA binding motif) are transformed by the above two-hybrid system expression plasmids, and then incubated on a histidine-free synthetic culture medium. Herein, cells only grow when a protein interaction is present. Thus, the increase in reporter gene expression can be examined by the growth rate of the transformant.

Other than the HIS3 gene, for example, the luciferase gene, plasminogen activator inhibitor type1 (PAI-1) gene, ADE2 gene, LacZ gene, CDC25H gene, and such can be used as reporter genes.

The two-hybrid system may be constructed according to the usual methods, or a commercially available kit may be used. As commercially available two-hybrid system kits, the MATCHMARKER Two-Hybrid System, Mammalian MATCHMARKER Two-Hybrid Assay Kit (both by CLONTEC), HybriZAP Two-Hybrid Vector System (Stratagene), and CytoTrap two-hybrid system (Stratagene) can be given.

A protein binding to the protein of the invention can be screened by affinity chromatography. Namely, the protein of the invention is immobilized onto a carrier of an affinity column, and a test sample presumed to express a protein binding to the protein of the invention is applied to the column. As this test sample, a cell culture supernatant, cell extract, cell lysate, and such may be used. After applying the test sample, the column is washed to obtain the protein binding to the protein of the invention.

The compound isolated by the screening method of the invention is a candidate drug for promoting or inhibiting the activity of the protein of the invention. The compound obtained by using the screening method of the invention encompasses a compound resulting from modifying the compound having an activity to bind to the protein of the invention by adding, deleting, and/or replacing a part of the structure.

When using the compound obtained by the screening method of the invention as drugs for humans and other mammals such as, mice, rats, guinea pigs, rabbits, chicken, cats, dogs, sheep, pigs, cattle, monkeys, sacred baboons, and chimpanzees, the drug may be administered using ordinary means.

For example, according to the need, the drugs can be taken orally as sugar-coated tablets, capsules, elixirs, and microcapsules, or parenterally in the form of injections of sterile solutions or suspensions with water or any other pharmaceutically acceptable liquid. For example, the compounds comprising the activity to bind to the protein of the invention can be mixed with physiologically acceptable carriers, flavoring agents, excipients, vehicles, preservatives, stabilizers, and binders, in a unit dose form required for generally accepted drug implementation. The amount of active ingredients in these preparations makes a suitable dosage within the indicated range acquirable.

Examples of additives that can be mixed to tablets and capsules are, binders such as gelatin, corn starch, tragacanth gum, and arabic gum; excipients such as crystalline cellulose; swelling agents such as cornstarch, gelatin, and alginic acid; lubricants such as magnesium stearate; sweeteners such as sucrose, lactose, or saccharin; and flavoring agents such as peppermint, Gaultheria adenothrix oil, and cherry. When the unit dosage form is a capsule, a liquid carrier, such as oil, can also be included in the above additives. Sterile compositions for injections can be formulated following usual drug implementations using vehicles such as distilled water used for injections.

For example, physiological saline and isotonic liquids including glucose or other adjuvants, such as D-sorbitol, D-mannose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil or soy-bean oil can be used as a oleaginous liquid and may be used in conjunction with benzyl benzoate or benzyl alcohol as a solubilizer; may be formulated with a buffer such as phosphate buffer and sodium acetate buffer; a pain-killer such as procaine hydrochloride; a stabilizer such as benzyl alcohol and phenol; and an anti-oxidant. The prepared injection is usually filled into a suitable ampule.

Although the dosage of the compound that has the activity to bind to the protein of the invention varies according to symptoms, the daily dose is generally about 0.1 to about 100 mg, preferably about 1.0 to about 50 mg, and more preferably about 1.0 to about 20 mg, when administered orally to an adult (body weight 60 kg).

When given parenterally, the dose differs according to the patient, target organ, symptoms, and method of administration, but the daily dose is usually about 0.01 to about 30 mg, preferably about 0.1 to about 20 mg and more preferably about 0.1 to about 10 mg for an adult (body weight 60 kg) when given as an intravenous injection. Also, in the case of other animals too, it is possible to administer an amount converted to 60 kg of body-weight.

The antibody of the present invention can be obtained as a monoclonal antibody or a polyclonal antibody using well-known methods.

The antibody that specifically binds to the protein of the invention can be prepared by using the protein of the invention as a sensitizing antigen for immunization according to usual immunizing methods, fusing the obtained immunized cells with known parent cells by ordinary cell fusion methods, and screening for antibody producing cells using the usual screening techniques.

Specifically, a monoclonal or polyclonal antibody that binds to the proteins of the invention may be prepared as follows.

For example, the protein of the invention that is used as a sensitizing antigen for obtaining the antibody is not restricted by the animal species from which it is derived, but is preferably a protein derived from mammals, for example, humans, mice, or rats, especially from humans. Proteins of human origin can be obtained by using the nucleotide sequence or amino acid sequence disclosed herein.

The protein that is used as a sensitizing antigen in the present invention can be a protein that comprises the biological activity of all the proteins described herein. Partial peptides of the proteins may also be used. As partial peptides of the proteins, for example, the amino (N) terminal fragment of the protein, and the carboxy (C) terminal fragment can be given. "Antibody" as used herein means an antibody that specifically reacts with the full-length or fragment of the protein.

A gene encoding the protein of the invention or a fragment thereof is inserted into a well-known expression vector, and after transforming the host cells described herein, the objective protein or a fragment thereof is obtained from within and without the host cell, or from the host using well-known methods, and this protein can be used as a sensitizing antigen. Also, cells expressing the protein, cell lysates, or chemically synthesized protein of the invention may be used as a sensitizing antigen.

The mammals that are immunized by the sensitizing antigen are not restricted, but it is preferable to select the animal by considering the adaptability with the parent cells used in cell fusion. Generally, an animal belonging to Rodentia, Lagomorpha, or Primates is used.

As animals belonging to Rodentia, for example, mice, rats, hamsters, and such are used. As animals belonging to Lagomorpha, for example rabbits, as Primates, for example monkeys, are used. As monkeys, monkeys of the infraorder Catarrhini (Old World Monkeys), for example, cynomolgus monkeys, rhesus monkeys, sacred baboons, chimpanzees, etc., are used.

To immunize animals with the sensitizing antigen, well-known methods may be used. For example, the sensitizing antigen is generally injected into mammals intraperitoneally or subcutaneously. Specifically, the sensitizing antigen is suitably diluted, suspended in physiological saline or phosphate-buffered saline (PBS), mixed with a suitable amount of a general adjuvant if desired, for example, with Freund's complete adjuvant, emulsified and injected into the mammal. Thereafter, the sensitizing antigen suitably mixed with Freund's incomplete adjuvant is preferably given several times every four to 21 days. A suitable carrier can also be used when immunizing an animal with the sensitizing antigen. After the immunization, the elevation in the serum antibody level is detected by usual methods.

Polyclonal antibodies against the protein of the invention can be obtained as follows. After verifying that the desired serum antibody level has been reached, blood is withdrawn from the mammal sensitized with the antigen. Serum is isolated from this blood using well-known methods. The serum containing the polyclonal antibody may be used as the polyclonal antibody, or according to needs, the polyclonal antibody-containing fraction may be further isolated from the serum.

To obtain monoclonal antibodies, after verifying that the desired serum antibody level has been reached in the mammal sensitized with the above-described antigen, immunocytes are taken from the mammal and used for cell fusion. At this instance, immunocytes that are preferably used for cell fusion are splenocytes. As parent cells fused with the above immunocytes, preferable are mammalian myeloma cells, more preferable are, myeloma cells that have attained the feature of distinguishing fusion cells by agents.

For the cell fusion between the above immunocytes and myeloma cells, for example, the method of Milstein et al. (Galfre, G. and Milstein, C., Methods Enzymol., 1981, 73, 3-46) is basically well known.

The hybridoma obtained from cell fusion is selected by culturing in a usual selective culture medium, for example, HAT culture medium (hypoxanthine, aminopterin, thymidine-containing culture medium). The culture in this HAT medium is continued for a period sufficient enough for cells (non-fusion cells)other than the objective hybridoma to perish, usually from a few days to a few weeks. Next, the usual limiting dilution method is carried out, and the hybridoma producing the objective antibody is screened and cloned.

Other than the above method of obtaining a hybridoma by immunizing an animal other than humans with the antigen, a hybridoma producing the objective human antibodies comprising the activity to bind to proteins can be obtained by the method of sensitizing human lymphocytes, for example, human lymphocytes infected with the EB virus, with proteins, protein-expressing cells, or lysates thereof *in vitro*, fusing the sensitized lymphocytes with myeloma cells derived from human, for example U266, having the capacity of permanent cell division (Unexamined Published Japanese Patent Application (JP-A) No. Sho 63-17688).

Moreover, human antibody against the protein can be obtained using a hybridoma made by fusing myeloma cells with antibody-producing cells obtained by immunizing a transgenic animal comprising a repertoire of human antibody genes with an antigen such as a protein, protein-expressing cells, or a cell lysate thereof WO92/03918, WO93/2227, WO94/02602, WO94/25585, WO96/33735, and WO96/34096).

Other than producing antibodies by using hybridoma, antibody-producing immunocytes such as sensitized lymphocytes that are immortalized by oncogenes may also be used.

Such monoclonal antibodies can also be obtained as recombinant antibodies produced by using the gene engineering technique (for example, Borrebaeck, C.A.K. and Larrick, J.W., THERAPEUTIC MONOCLONAL ANTIBODIES, Published in the United Kingdom by MACMILLAN PUBLISHERS LTD, 1990). Recombinant antibodies are produced by cloning the encoding DNA from immunocytes such as hybridoma or antibody-producing sensitized lymphocytes, incorporating this into a suitable vector, and introducing this vector into a host to produce the antibody. The present invention encompasses such recombinant antibodies as well.

The antibody of the present invention may be an antibody fragment or a modified-antibody as long as it binds to the protein of the invention. For example, Fab, F(ab')₂, Fv, or single chain Fv in which the H chain Fv and the L chain Fv are suitably linked by a linker (scFv, Huston, J.S. et al., Proc. Natl. Acad. Sci. U.S.A., 1988, 85, 5879-5883) can be given as antibody fragments. Specifically, antibody fragments are produced by treating an antibody with an enzyme, for example, papain, pepsin, etc. or by constructing a gene encoding an antibody fragment, introducing this into an expression vector, and expressing this vector on suitable host cells (for example, Co, M.S. et al., J. Immunol., 1994, 152, 2968-2976; Better, M. and Horwitz, A.H., Methods Enzymol., 1989, 178, 476-496; Pluckthun, A. and Skerra, A., Methods Enzymol., 1989, 178, 497-515; Lamoyi, E., Methods Enzymol., 1986, 121, 652-663; Rousseaux, J. et al., Methods Enzymol., 1986, 121, 663-669; Bird, R.E. and Walker, B.W., Trends Biotechnol., 1991, 9, 132-137).

As a modified antibody, an antibody bound to various molecules such as polyethylene glycol (PEG) can be used. The present antibody encompasses such modified antibodies as well. To obtain such a modified antibody, chemical modifications are done to the obtained antibody. These methods are already established in the field.

The antibody of the invention may be obtained as a chimeric antibody comprising non-human antibody-derived variable region and a human antibody-derived constant region, or as a humanized antibody comprising non-human antibody-derived complementarity determining region (CDR), and human antibody-derived framework region (FR) and a constant region.

Antibodies thus obtained can be purified till uniform. The separation and purification methods for separating and purifying the antibody used in the present invention may be any method usually used for proteins, and is not in the least limited. Antibody concentration of the above mentioned antibody can be assayed by measuring the absorbance, or by the enzyme-linked immunosorbent assay (ELISA), etc.

Also, as methods that assay the antigen-binding activity of the antibody of the invention, ELISA, enzyme immunoassay (EIA), radio immunoassay (RIA), or fluorescent antibody method can be given. For example, when using ELISA, the protein of the invention is added to a plate coated with the antibody of the invention, and next, the objective antibody sample, for example, culture supernatants of antibody-producing cells, or purified antibodies are added. Then, secondary antibody recognizing the antibody, which is labeled by alkaline phosphatase and such enzymes, is added, the plate is incubated and washed, and absorbance is measured to evaluate the antigen-binding activity after adding an enzyme substrate such as p-nitrophenyl phosphate. As the protein, a protein fragment, for example, a fragment comprising a C terminus, or a fragment comprising an N terminus may be used. To evaluate the activity of the antibody of the invention, BIAcore (Pharmacia) may be used.

By using these methods, the antibody of the invention and a sample presumed to contain the protein of the invention are contacted, and the protein of the invention is detected or assayed by detecting or assaying the immune complex of the above-mentioned antibody and protein.

A method of detecting or assaying the protein of the invention is useful in various experiments using proteins as it can specifically detect or assay the proteins.

The present invention also encompasses a DNA specifically hybridizing to a DNA comprising a nucleotide sequence of any one of SEQ ID NOs: 2, 4, 6, 8, 20, and 22 to 27 or its complementary DNA, and comprising at least 15 nucleotides. Namely, a probe that can selectively hybridize to the DNA encoding the protein of the invention, or a DNA complementary to the above DNA, a nucleotide or nucleotide derivative, for example, antisense oligonucleotide, ribozyme, and such are included.

The present invention also encompasses an antisense oligonucleotide that hybridizes to any portion of any one of the nucleotide sequences shown in, for example, SEQ ID NOs: 2, 4, 6, 8, 20, and 22 to 27. This antisense oligonucleotide is preferably one against at least 15 continuous nucleotides in any one of the nucleotide sequences of SEQ ID NOs: 2, 4, 6, 8, 20, and 22 to 27. More preferable is the above-mentioned antisense oligonucleotide against the above-mentioned at least 15 continuous nucleotides containing a translation start codon.

Derivatives or modified products of antisense oligonucleotides can be used as antisense oligonucleotides. As such modified products, for example, lower alkyl phosphonate modifications such as methyl-phosphonate-type or ethyl-phosphonate-type, phosphorothioate or phosphoroamidate-modified products, etc. may be used.

The term "antisense oligonucleotide(s)" as used herein means, not only those in which the nucleotides corresponding to those constituting a specified region of a DNA or mRNA are entirely complementary, but also those having a mismatch of one or more nucleotides, as long as the DNA or mRNA and the oligonucleotide can selectively and stably hybridize with the nucleotide sequence of SEQ ID NO: 1.

"Selectively and stably hybridize" means that significant cross hybridization with DNA encoding other proteins does not occur under usual hybridization conditions, preferably under stringent hybridization conditions. Such DNAs are indicated as those having, in the "at least 15 continuous nucleotide" sequence region, a homology of at least 70% or higher, preferably 80% or higher, more preferably 90% or higher, even more preferably 95% or higher nucleotide sequence homology. The algorithm stated herein can be used to determine homology. Such DNA is useful as a probe for detecting or isolating DNA encoding the protein of the invention, or as a primer for amplification as described in Examples below.

The antisense oligonucleotide derivative of the present invention acts upon cells producing the protein of the invention by binding to the DNA or mRNA encoding the protein to inhibit its transcription or translation, and to promote the degradation of mRNA, and has an effect of suppressing the function of the protein of the invention by suppressing the expression of the protein.

The antisense oligonucleotide derivative of the present invention can be made into an external preparation such as a liniment and a poultice by mixing with a suitable base material, which is inactive against the derivatives.

Also, as needed, the derivatives can be formulated into tablets, powders, granules, capsules, liposome capsules, injections, solutions, nose-drops, and freeze-dried agents by adding excipients, isotonic agents, solubilizers, stabilizers, preservatives, pain-killers, etc. These can be prepared using the usual methods.

The antisense oligonucleotide derivative is given to the patient by directly applying onto the ailing site, by injecting into a blood vessel, etc. so that it will reach the ailing site. An antisense-mounting material can also be used to increase durability and membrane-permeability. Examples are, liposome, poly-L lysine, lipid, cholesterol, lipofectin, or derivatives of these.

The dosage of the antisense oligonucleotide derivative of the present invention can be adjusted suitably according to the patient's condition and used in desired amounts. For example, a dose range of 0.1 to 100 mg/kg, preferably 0.1 to 50 mg/kg can be administered.

The antisense oligonucleotide derivative of the present invention is useful in inhibiting the expression of the protein of the invention, and therefore is useful in suppressing the biological activity of the protein of the invention. Also, expression-inhibitors comprising the antisense oligonucleotide derivative of the present invention are useful because of their capability to suppress the biological activity of the protein of the invention.

### Brief Description of the Drawings

Figure 1 is a schematic diagram showing the results of BlastX search where the query was 180 nucleotides of 40861-41040 including 40952-40966, the only probe sequence within the AC002303. "#": For only NR8 the number was indicated by the nucleotide number. The underline of the NR8 sequence shows the portion corresponding to the exon. Other underlined sequences show identical amino acids.
Figure 2 is a schematic diagram showing the results of BlastX scanning of 180 nucleotides in both the 5' and 3' directions, where the search centered on the 180 nucleotides of 40861-41040 containing 40952-40966, the only probe sequence within the AC002303.
Figure 3 shows the electrophoresis results of the amplification done by the RT-PCR method for the combinations of SN1/AS1, SN1/AS2, SN2/AS1, and SN2/AS2 primers using human fetal liver and skeletal muscle cDNA as templates.
Figure 4 shows the electrophoretic results of the 5'-RACE method and 3'-RACE method using human fetal liver cDNA as the template.
Figure 5 shows the nucleotide sequence and the amino acid sequence of NR8α cDNA. The arrows show the positions of primers used for RT-PCR. They are, SN1 (798-827), SN2 (894-923), AS2 (1055-1026), and AS1 (1127-1098) from the 5' side, in their order. For two bases at the 5' end of AS1, AC, which is derived from the genomic sequence, was used in place of CT.
Figure 6 is the continuation of Fig. 5 showing the nucleotide sequence and the amino acid sequence of NR8α cDNA.

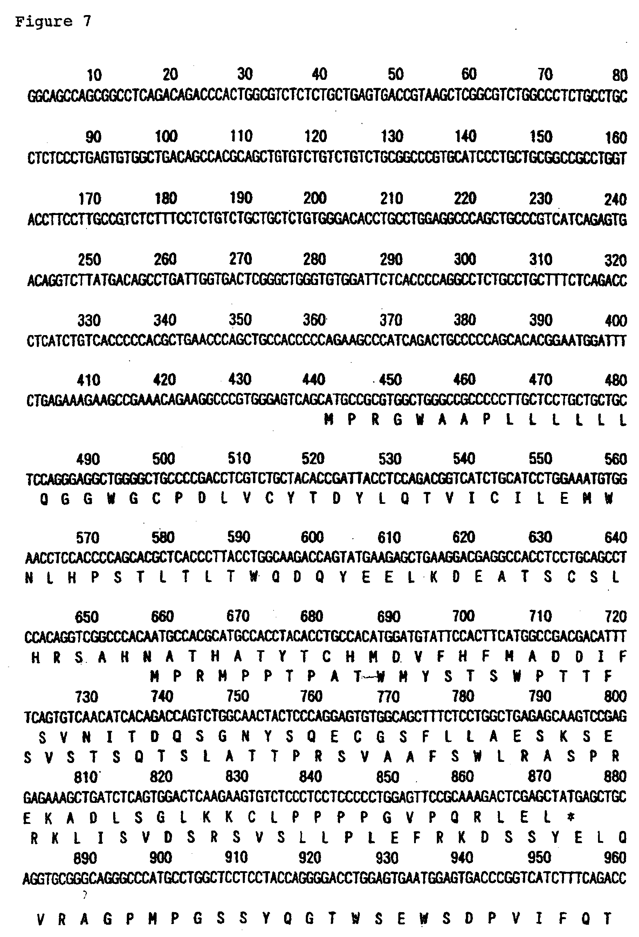

Figure 7 shows the nucleotide sequence and the amino acid sequence of NR8β cDNA. Two possible open reading frames (ORF) are shown.

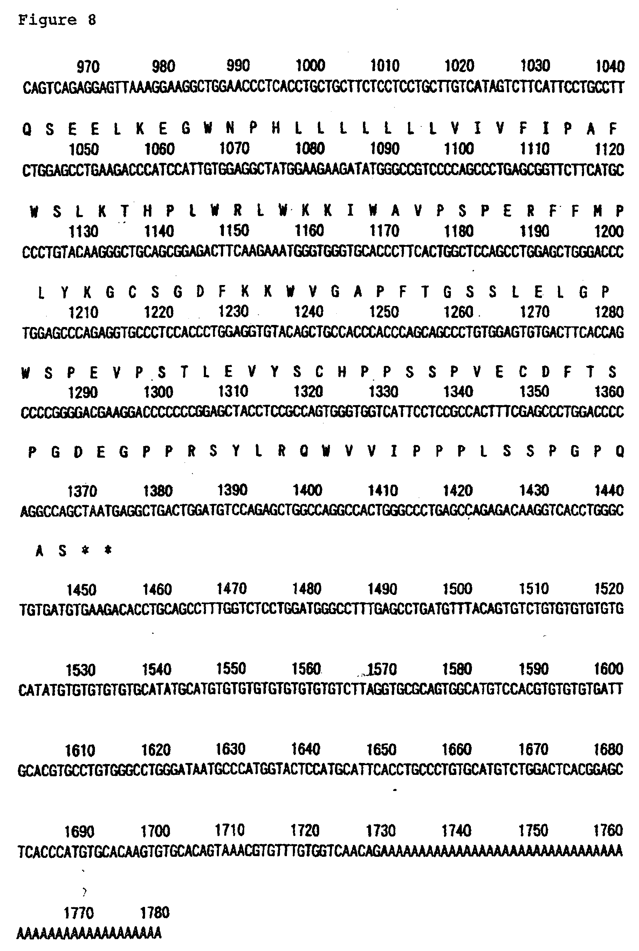

Figure 8 is the continuation of Fig. 7 showing the nucleotide sequence and the amino acid sequence of NR8β cDNA.

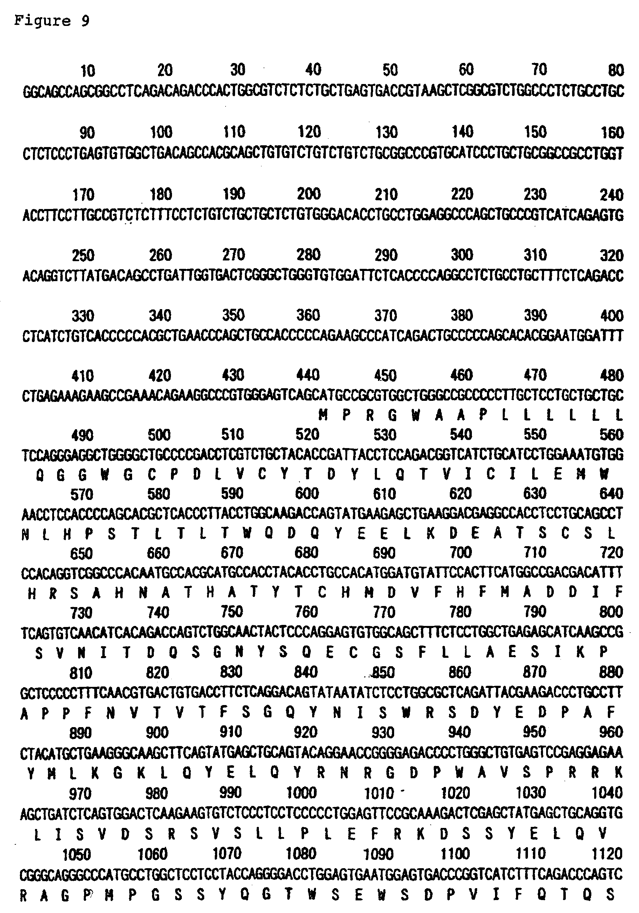

Figure 9 shows the nucleotide sequence and the amino acid sequence of NR8γ cDNA. The 177 amino acids inserted by selective splicing are underlined.

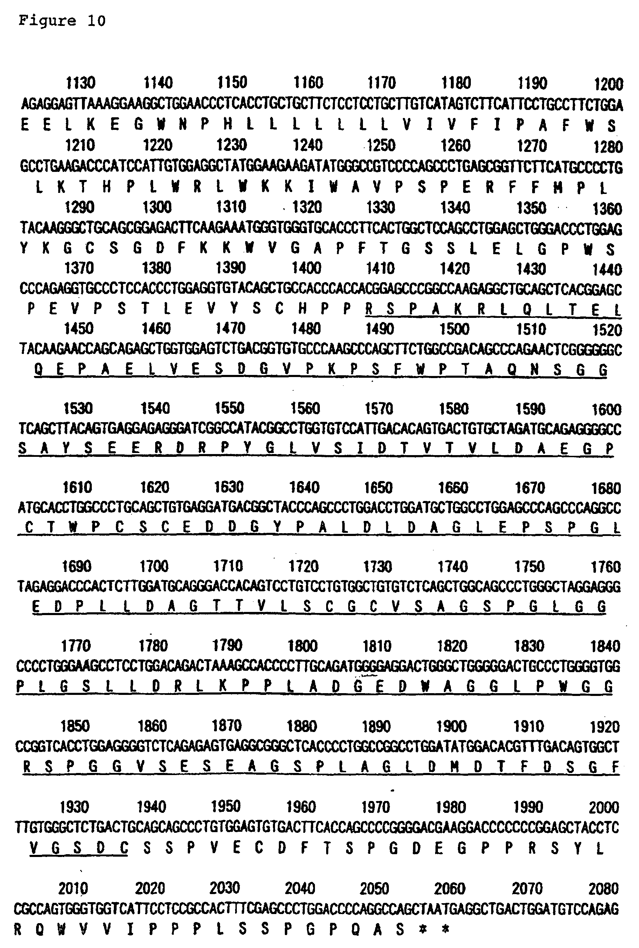

Figure 10 is the continuation of Fig. 9 showing the nucleotide sequence and the amino acid sequence of NR8γ cDNA. The 177 amino acids inserted by selective splicing are underlined.

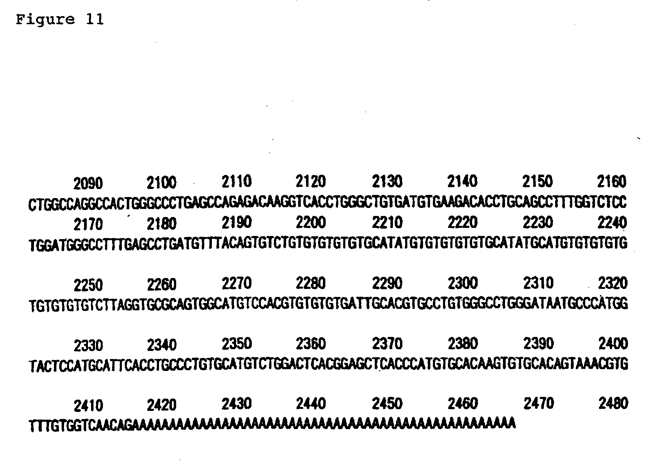

Figure 11 is the continuation of Fig. 10 showing the nucleotide sequence and the amino acid sequence of NR8γ cDNA.
Figure 12 shows the results of Northern blot analysis of NR8 expression in each organ.
Figure 13 is a schematic diagram showing the structure of the NR8 gene. Other repetitives include, (CA)n, (CAGA)n, (TGGA)n, (CATA)n, (TA)n, (GA)n, (GGAA)n, (CATG)n, (GAAA)n, MSTA, AT-rich, MLT1A1, LINE2, FLAM_C, MER63A, and MSTB.
Figure 14 is a schematic diagram showing the structure of expressible proteins constructed in the expression vector.
Figure 15 shows the results of cross PCR, in which the human NR8 primer set was used against a mouse cDNA library. As the size marker, 100 bp DNA Ladder (NEB#323-1L) was used.
Figure 16 shows a comparison between amino acid sequences of human and mouse NR8β. The amino acid sequences where the two coincide are shadowed. Also, cysteine residues conserved in other hemopoietin receptors are displayed in boldface type within the sequence.
Figure 17 shows a comparison between amino acid sequences of human and mouse NR8γ. The amino acid sequences where the two coincide are shadowed. Also, cysteine residues conserved in other hemopoietin receptors and the WSXWS-Box are displayed in boldface type within the sequence.
Figure 18 shows the results of NR8 gene expression analysis in each mouse organ using the RT-PCR method. The size marker, 100 bp DNA Ladder (NEB#323-1L), is shown on the either sides of the lane. A 320 bp target gene has been detected in all organs.
Figure 19 shows the results of NR8 gene expression analysis in each mouse organ using the Northern blotting method (left). An approximately 4.2 kb transcript was intensely detected in the testis only. Mouse β-actin was detected in the same blot as a positive control (right).

### Best Mode for Carrying Out the Invention

The present invention shall be described in detail below with reference to examples, but is not be construed as being limited thereto.

### Example 1: Two step Blast Search

Probe sequences (256 types) comprising the tggag(t/c)nnntggag(t/c) (where n is an arbitrary nucleotide) as the oligonucleotide encoding the Trp-Ser-Xaa-Trp-Ser motif were designed. These sequences enable the detection of almost all known hemopoietin receptors, except for the EPO receptor, TPO receptor, and the mouse IL6 receptor. Using each sequence as the query, the GenBank nr database was searched using the BlastN (Advanced BlastN 2.0.4) program. Default values (Descriptions=100, Alignments=100) were used as parameters for the search, except for making the expectation value 100.

Since approximately 500 clones that completely matched the probe sequences were obtained as a result of the primary search, among these, a 180-residue nucleotide sequence of human genome-derived clones (cosmid, BAC, and PAC) containing the probe sequence in approximately the center was excised. Next, using this 180-residue nucleotide sequence as the query, the nr database was searched again using the BlastX (Advanced BlastX 2.0.4) program to search the homology of the amino acid sequence around the probe sequence with known hemopoietin receptors.

Default values were used as parameters for the search, except for making the expectation value 100. However, when extremely large number of hits were obtained (caused by the Alu sub family that is a high repetitive sequence), it was often difficult to observe hits for known hemopoietic receptors. Therefore, to maximize the sensitivity in such cases, a value of "Expect=1000, Descriptions=500, Alignments=500" was used.

As a result of the secondary search by BlastX, 28 clones hit one or more known hemopoietin receptors (Table 1 to Table 8).

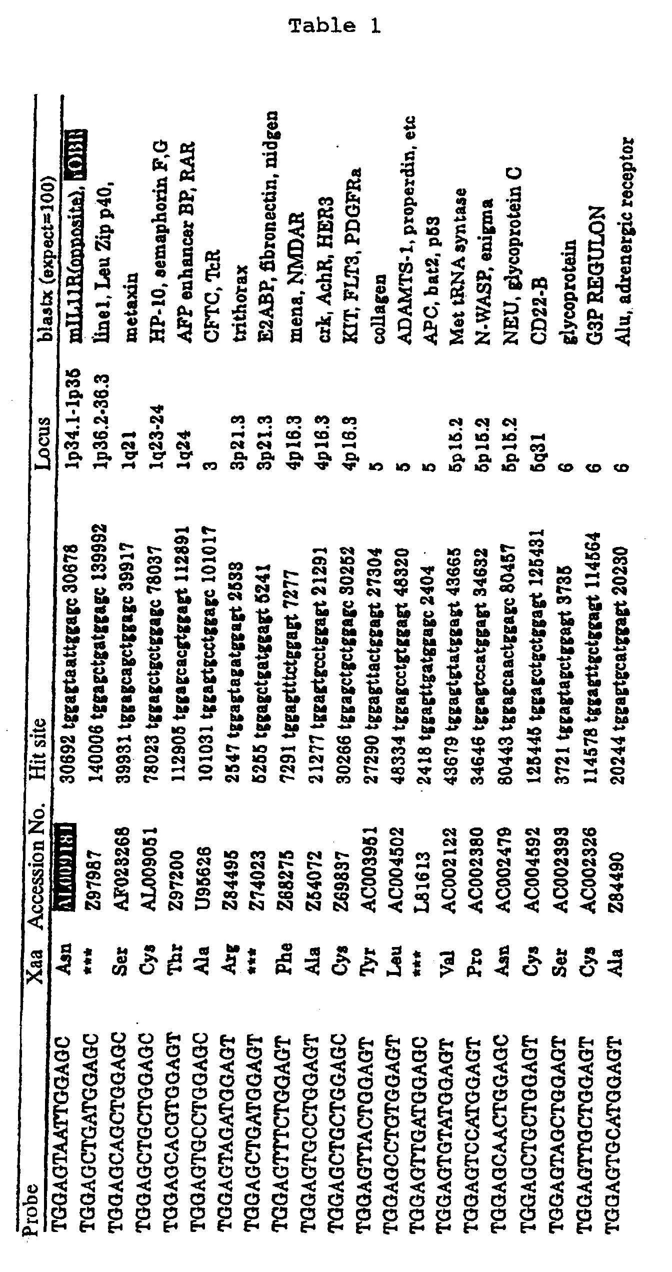

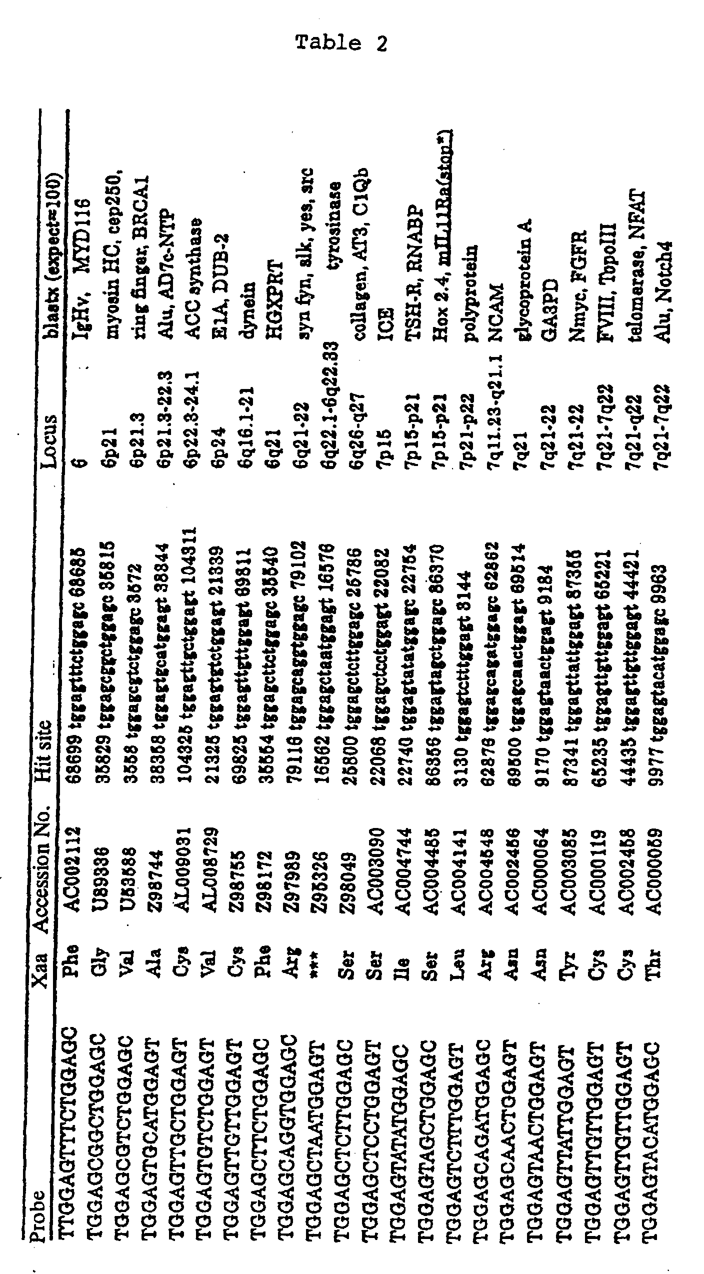

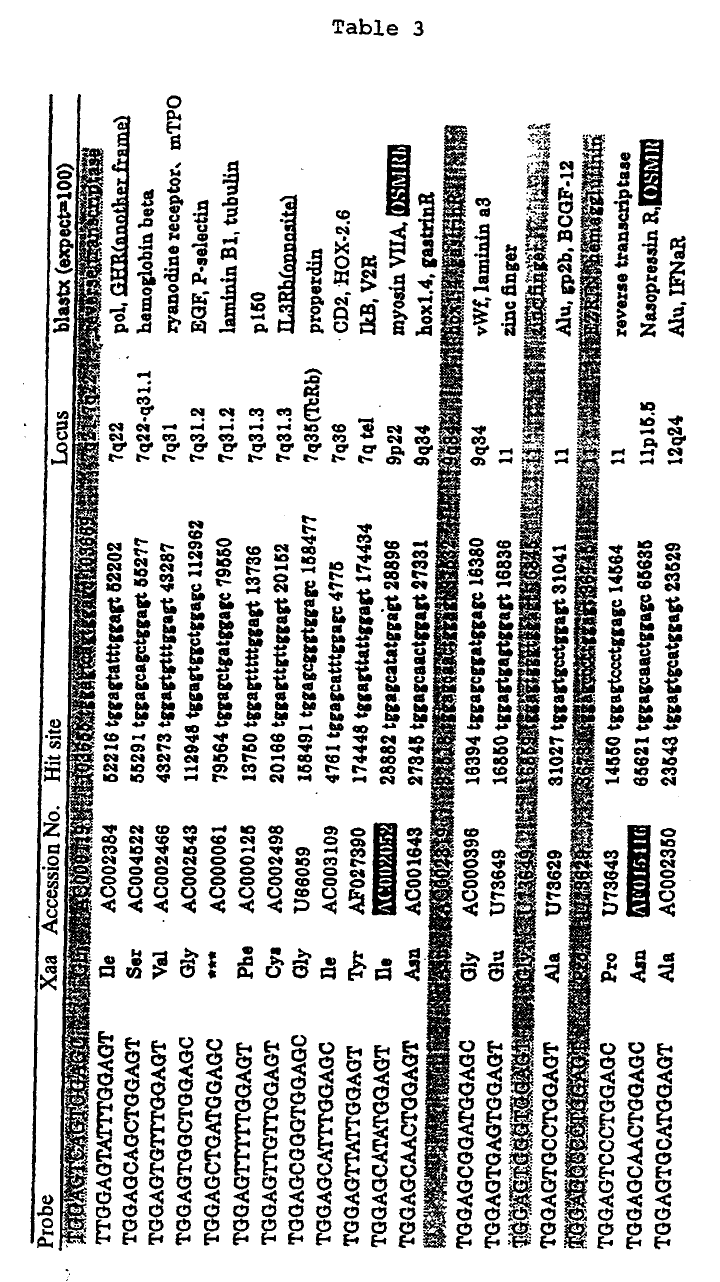

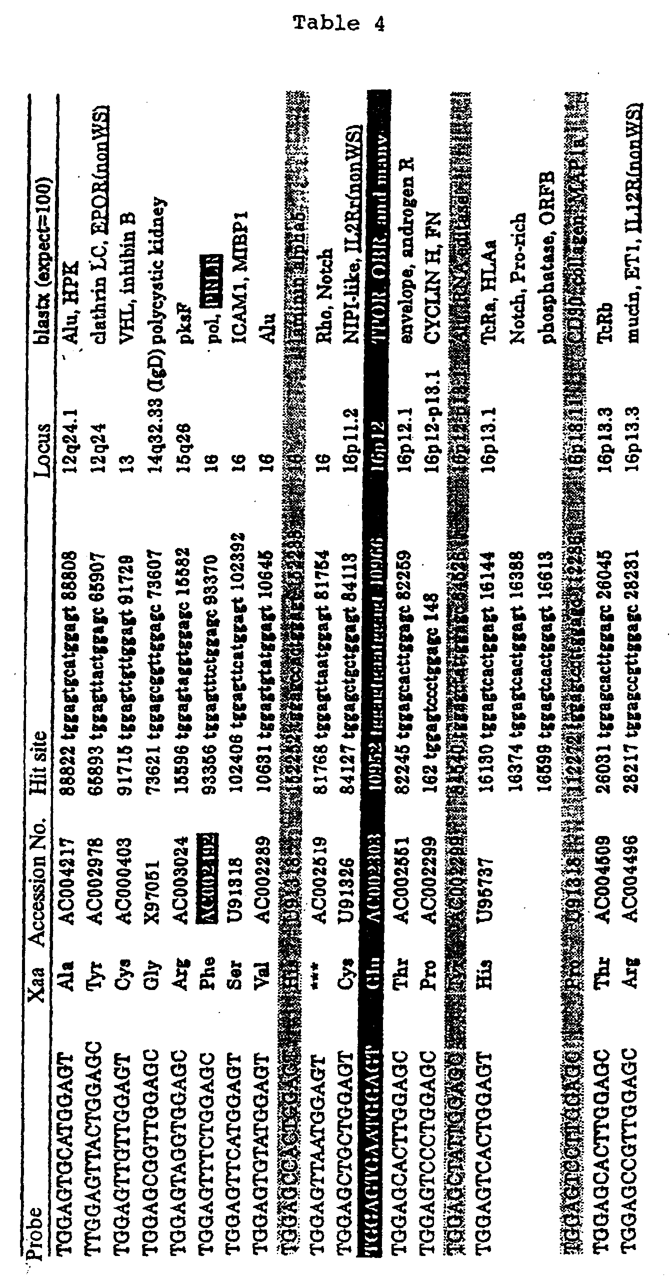

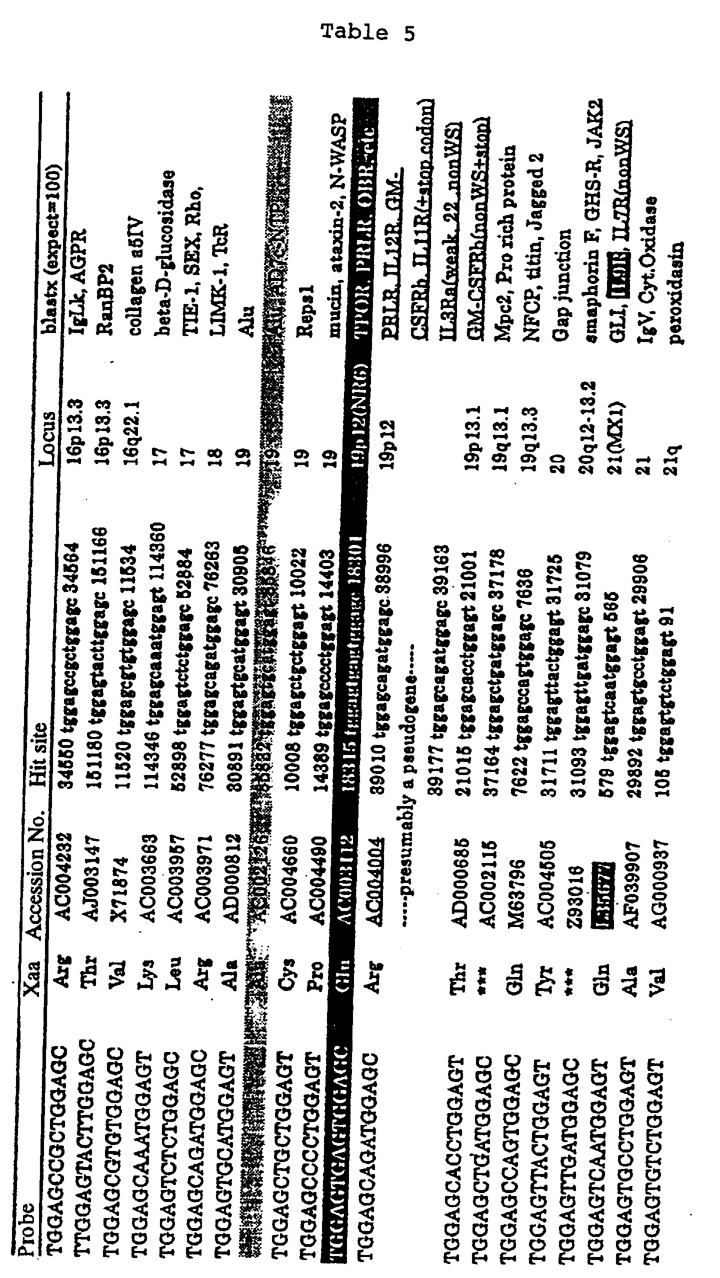

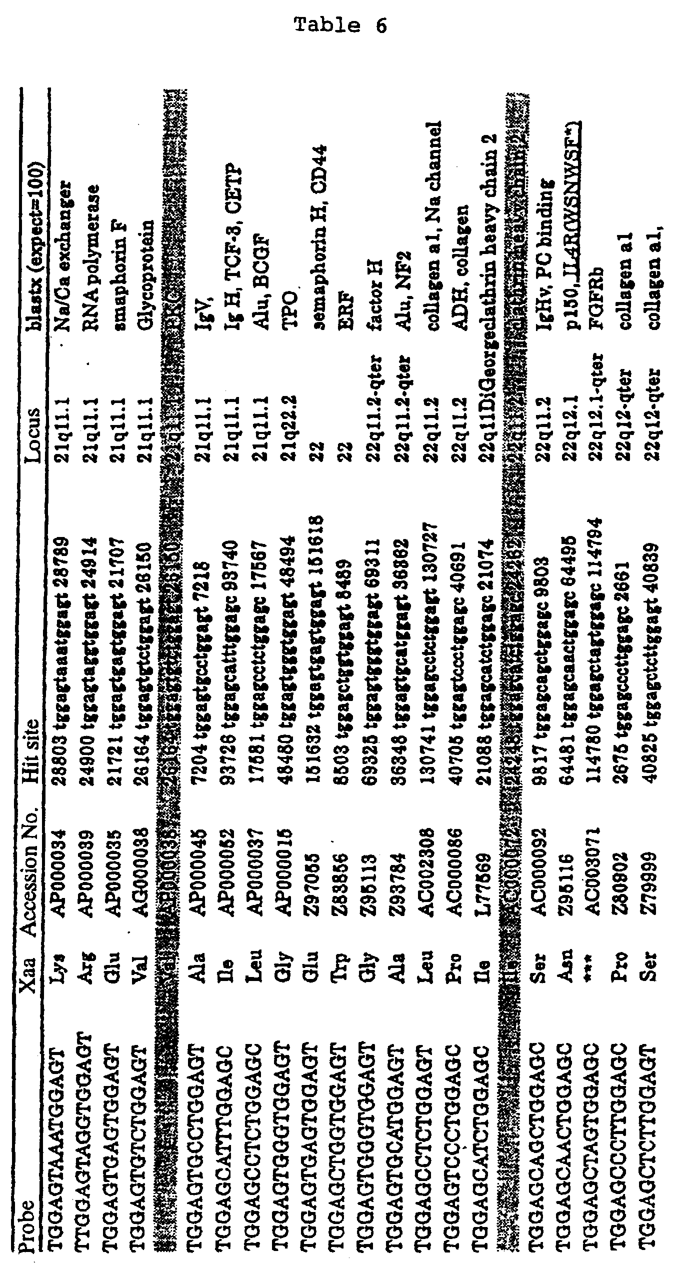

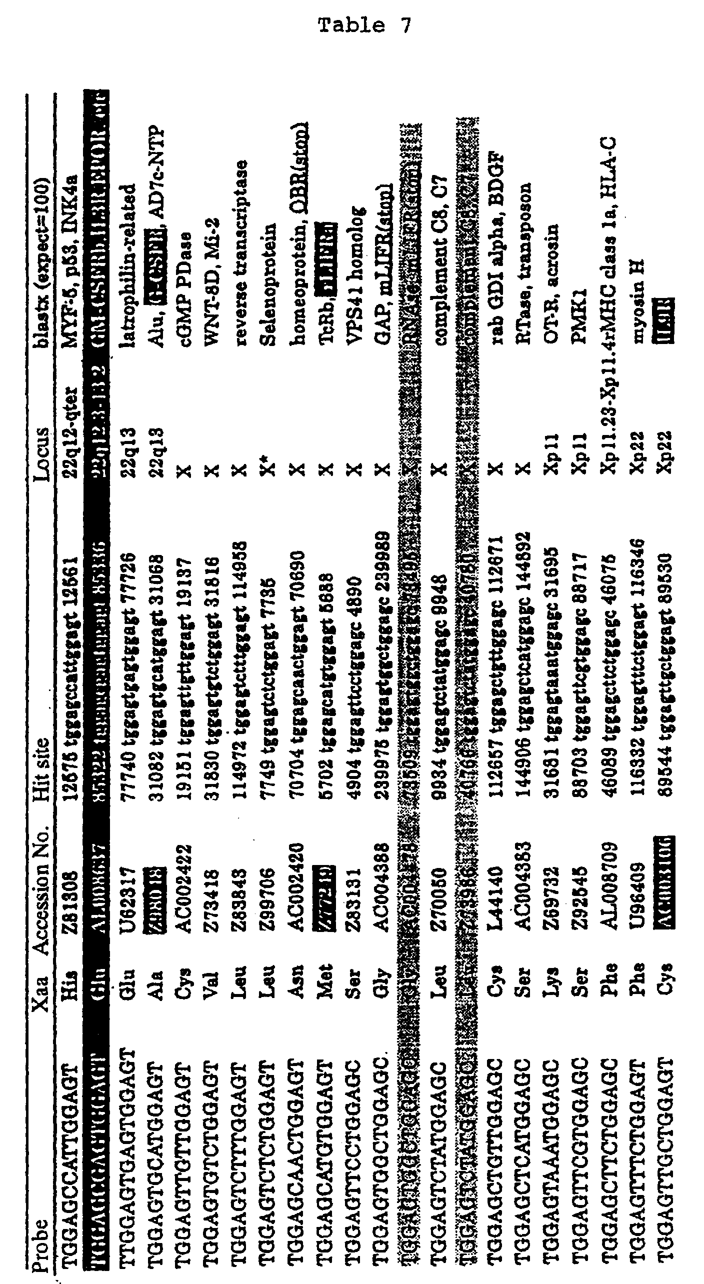

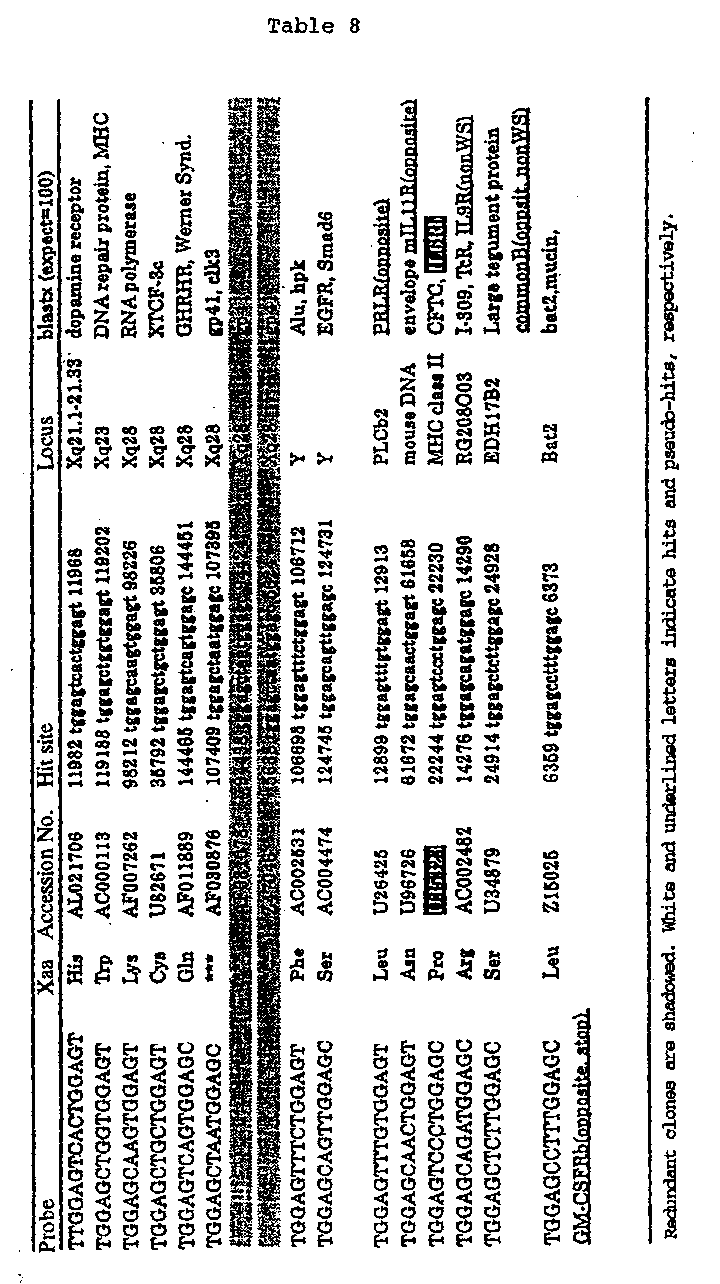

Four clones out of these 28 clones (AC002303, AC003112, AL008637, and AC004004) hit several known hemopoietin receptors, however, AC004004 was excluded as it has a stop codon downstream three amino acids of the Trp-Ser-Xaa-Trp-Ser motif. Among the three remaining clones, AL008637 was thought to be a known receptor, GM-CSF receptor β. AC002303 is the BAC clone CIT987-SKA-670B5 derived from the 16p12 region of human chromosome no. 16 registered by TIGR group on June 19, 1997 and comprises the full-length of 131530 base pairs (Lamerdin, J.E., et al., GenBank Report on AC003112, 1997).

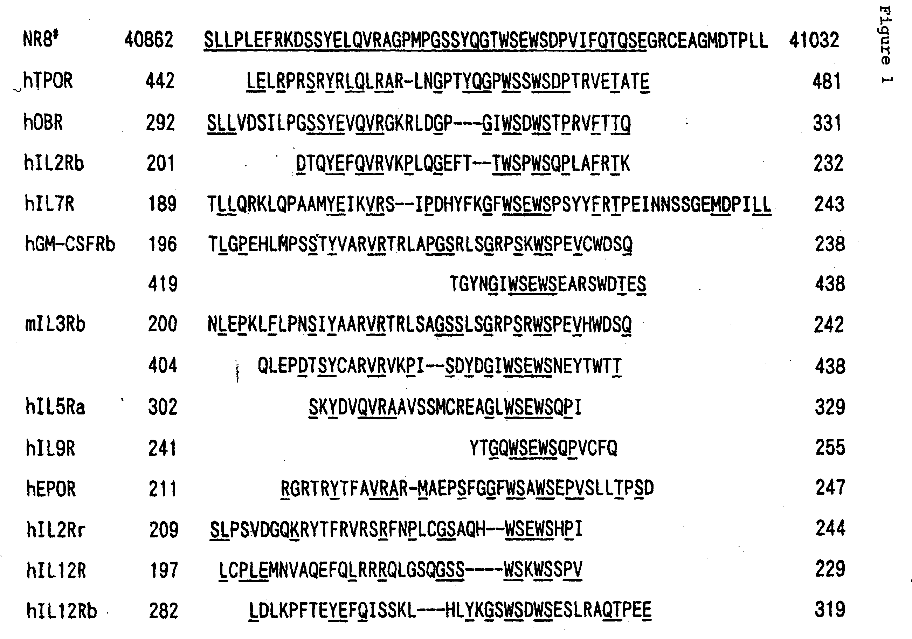

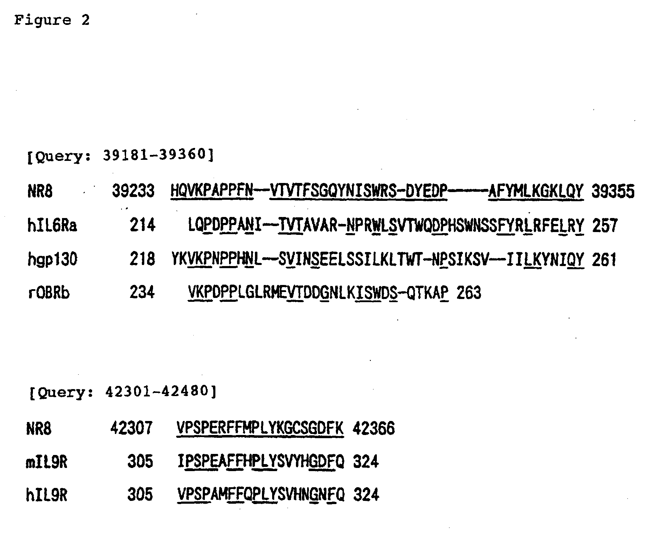

As shown in Fig. 1, a BlastX search (query: 180 nucleotides of 40861-41040 including tggagtgaatggagt (40952-40966), the only probe sequence within the AC002303) revealed that numerous hemopoietin receptors starting with the TPO receptor and leptin receptor show an evident homology, however, there were no known, database-registered hemopoietin receptors that completely matched the query sequence. Also, a BlastX scanning was done under the above conditions, by excising a sequential 180-residue nucleotide sequence in both the 5' and 3' directions, centering on the 180-residue nucleotide sequence mentioned above, and when this was used as a query, two sequences having a homology to known hemopoietin receptors were found in the regions 39181-39360 and 42301-42480, and were thought to be other exons of the same gene (Fig. 2).

A Pro-rich motif PAPPF was conserved in the 39181-39360 site, and a Box 1 motif in the 42301-42480 site. The 3' side exon adjacent to the exon containing the Trp-Ser-Xaa-Trp-Ser motif has a transmembrane domain, and this domain has a low homology with other hemopoietin receptors, and was not detected by the BlastX scan. These results suggested the possibility of a novel hemopoietin receptor gene existing in the above-described BAC clone CIT987-SKA-670B5.

### Example 2: Search for NR8 expressing tissues using RT-PCR

Pseudogenes have been reported to exist in several hemopoietin receptors (Kermouni, A. et al., Genomics, 1995, 29 (2) 371-382; Fukunaga, R. and Nagata, S., Eur. J. Biochem., 1994, 220, 881-891). To verify that NR8 is not a pseudogene, and with the objective of identifying NR8 expressing tissues, transcripts of the NR8 gene were searched by RT-PCR method.

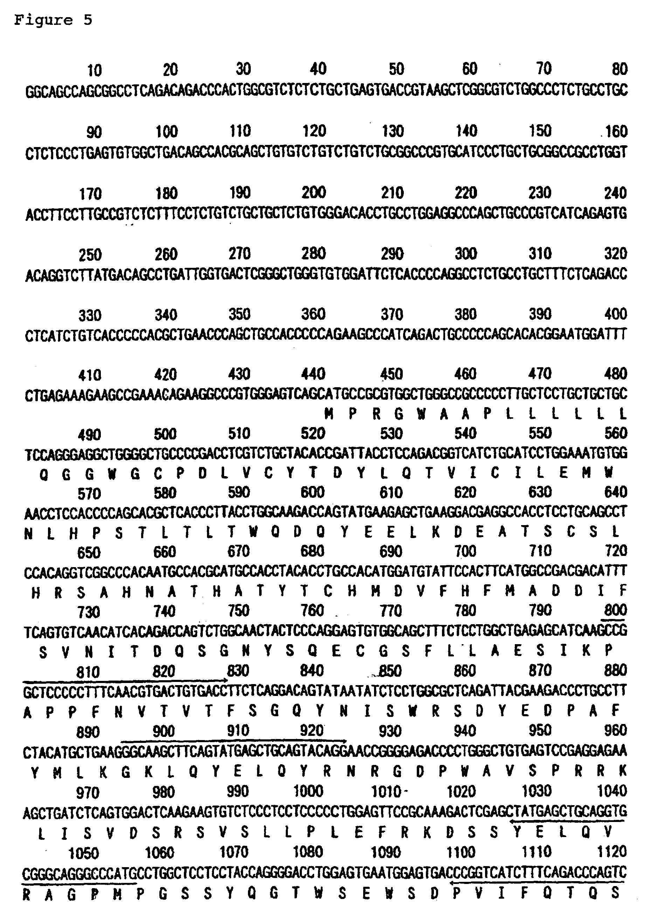

In the AC002303 sequence of the above-described BAC clone, several exon regions widely conserved at the amino acid translation level in known cytokine receptors were surmised, and on the sequence of the surmised exon region, the following primers were synthesized. (See Fig. 5 for the location of each primer.)
NR8-SN1; 5'- CCG GCT CCC CCT TTC AAC GTG ACT GTG ACC -3' (SEQ ID NO: 9)
NR8-SN2; 5'- GGC AAG CTT CAG TAT GAG CTG CAG TAC AGG -3' (SEQ ID NO: 10)
NR8-AS1; 5'- ACC CTC TGA CTG GGT CTG AAA GAT GAC CGG -3' (SEQ ID NO: 11)
NR8-AS2; 5'- CAT GGG CCC TGC CCG CAC CTG CAG CTC ATA -3' (SEQ ID NO: 12)

Using the Human Fetal Multiple Tissue cDNA Panel (Clontech #K1425-1) as the template, RT-PCR was attempted using combinations of the above primers. Advantage cDNA Polymerase Mix (Clontech #8417-1) was used for the PCR, which was conducted under the conditions below using the Perkin Elmer Gene Amp PCR System 2400 Thermalcycler.

Namely, the PCR conditions were, 94°C for 4 min, 5 cycles of "94°C for 20 sec, 72°C for 3 min," 5 cycles of "94°C for 20 sec,70°C for 3 min," 28 cycles of "94°C for 20 sec, 68°C for 3 min," 72°C for 4 min, and completed at 4°C.

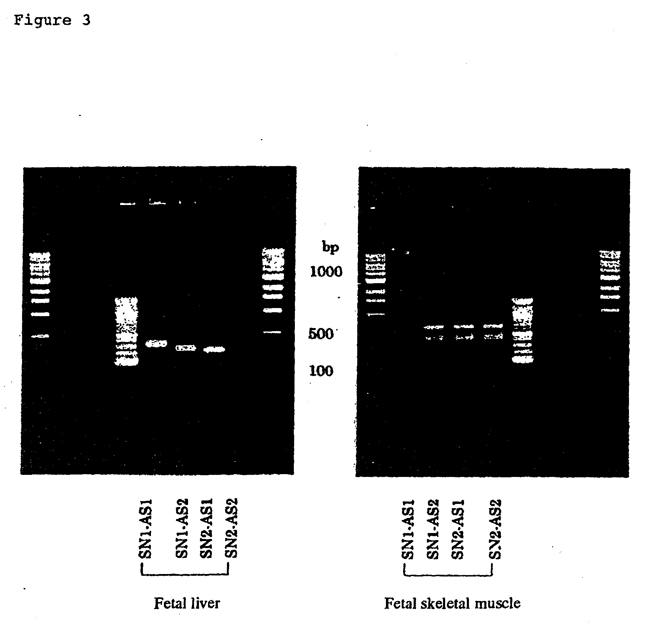

From the primer locations shown in Fig. 5, amplifications of bands sized 330 bp, 258 bp, 234 bp, and 162 bp can be expected from the combinations of SN1/AS1, SN1/AS2, SN2/AS1, and SN2/AS2. When evaluated using human fetal liver, brain, and skeletal muscle cDNA as the template, clear bands having the anticipated sizes were obtained in the fetal liver only with the respective primer combinations (Fig. 3).

An amplification was not seen at all for fetal brain cDNA, and a band of about 650 bp and a broad band of 400 to 500 bp were observed for fetal skeletal muscle cDNA. However, since the band sizes for skeletal muscle cDNA remained constant even when different combinations of primers were used, it is thought that these bands were non-specific amplifications due to some reason.

The obtained PCR product was subcloned to pGEM-T Easy vector (Promega #A1360), and the nucleotide sequence was determined. The recombination of PCR products to the pGEM-T Easy vector was done by T4 DNA Ligase (Promega #A1360) reacted at 4°C for 12 hr. The genetic recombinant between the PCR product and pGEM-T Easy vector was obtained by transforming *E.coli* strain DH5α (Toyobo #DNA-903).

For the selection of the genetic recombinant, Insert Check Ready (Toyobo #PIK-101) was used. The dRhodamine Terminator Cycle Sequencing Kit (ABI/Perkin Elmer #4303141) was used for determining the nucleotide sequence, and analysis was done using the ABI PRISM 377 DNA Sequencer. As a result of determining the nucleotide sequences of all inserts of the 10 independent clones of genetic recombinants, all clones were found to comprise a single nucleotide sequence. These obtained sequences were verified to be partial nucleotide sequences of NR8.

### Example 3: Full-length cDNA cloning by the 5' and 3'-RACE methods

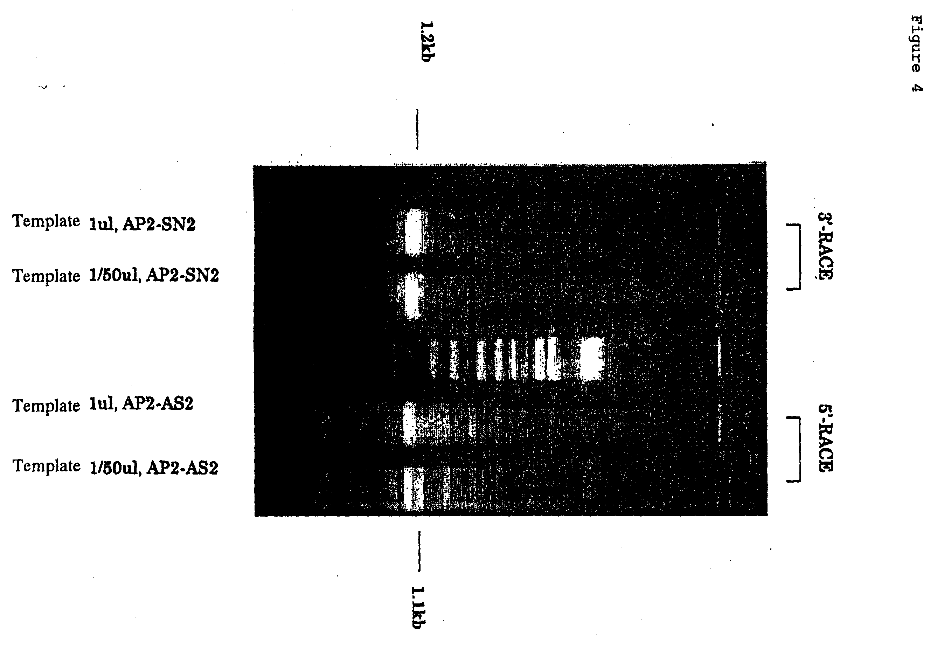

Using the thus-obtained fetal liver-derived cDNA, 5' and 3'-RACE methods were conducted to obtain full-length cDNA (Fig. 4).

### 3-1) 5'-RACE method

5'-RACE PCR was performed using the above-mentioned NR8-AS1 primer for primary PCR, and NR8-AS2 primer for secondary PCR. Human Fetal Liver Marathon-Ready cDNA Library (Clontech #7403-1) was used as the template and Advantage cDNA Polymerase Mix for the PCR experiment. As a result of PCR under the following conditions using the Perkin Elmer Gene Amp PCR System 2400 Thermalcycler, two types of PCR products were obtained, which have different sizes through selective splicing.

Primary PCR conditions were 94°C for 4 min, 5 cycles of "94°C for 20 sec, 72°C for 4 min," 5 cycles of "94°C for 20 sec, 70°C for 4 min," 28 cycles of "94°C for 20 sec, 68°C for 4 min," 72°C for 4 min, and completed at 4°C.

Secondary PCR conditions were 94°C for 4 min, 5 cycles of "94°C for 20 sec, 70°C for 3 min 30 sec," 28 cycles of "94°C for 20 sec, 68°C for 3 min 30 sec," 72°C for 4 min, and completed at 4°C.

Both types of PCR products obtained were subcloned to pGEM-T Easy vector as mentioned earlier, and the nucleotide sequences of all inserts were determined for the 16 independent clones of genetic transformants. As before, the dRhodamine Terminator Cycle Sequencing Kit was used for determining the nucleotide sequence, and analysis was done using the ABI PRISM 377 DNA Sequencer. As a result, the clones can be divided into two groups, one having 14 clones, and the other having 2 clones, by the length of the base pairs and the differences in sequence (though described later, the differences lie in the products due to selective splicing, and the group of 14 independent clones comprises the sequence corresponding to exon 5 in the genomic sequence, and the remaining group of two independent clones does not have this sequence).

### 3-2) 3'-RACE method

3'-RACE PCR was performed using the above-mentioned NR8-SN1 primer for primary PCR, and NR8-SN2 primer for secondary PCR. Human Fetal Liver Marathon-Ready cDNA Library was used as the template similar to 5'-RACE PCR, and Advantage cDNA Polymerase Mix for the PCR experiment. As a result of conducting PCR under the conditions shown in 3-1), a single band PCR product was obtained.

The obtained PCR product was subcloned to pGEM-T Easy vector as above, and the nucleotide sequences of all inserts of the 12 independent clones of genetic recombinants were determined. As before, the dRhodamine Terminator Cycle Sequencing Kit was used for determining the nucleotide sequence, and the sequences determined were analyzed using the ABI PRISM 377 DNA Sequencer. As a result, all 12 independent clones showed a single nucleotide sequence.

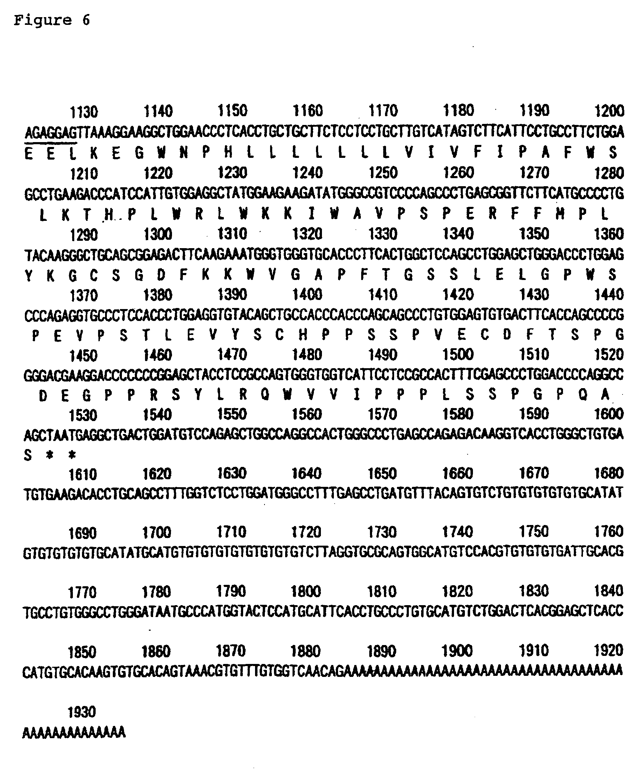

As a result of analyzing the nucleotide sequence of the fragments (approximately 1.1 kb and 1.2 kb) amplified by 5'-RACE and 3'-RACE, respectively, it was conceived that the approximately 260 bp of each fragment overlap and extend to the 5' side and 3' side, and contain almost the full-length of NR8 mRNA. These were joined to make a full-length cDNA (NR8α) (Fig. 5 and Fig. 6). The plasmid containing the NR8α cDNA (SEQ ID NO: 2) was named pGEM-NR8α, and *E.coli* containing the plasmid has been internationally deposited at the National Institute of BioScience and Human-Technology, Agency of Industrial Science and Technology (1-3, Higashi 1-chome, Tsukuba-shi, Ibaraki-ken, Japan) under the accession number FERM BP-6543 since October 9, 1998 according to the Budapest Treaty.

As shown in Fig. 5 and Fig. 6, in the ORF of NR8α cDNA, the Met starting from nucleotide no. 441 is thought to be the start codon due to the presence of an inframe stop codon 39 bp upstream, and completes with two stop codons starting from nucleotide no. 1524. It has the features of, from the N terminus in order, a typical secretion signal sequence, a domain thought to be the ligand binding site containing a Cys residue conserved in other hemopoietic receptor members, a Pro-rich motif, Trp-Ser-Xaa-Trp-Ser motif, a transmembrane domain, a Box 1 motif thought to be involved in signal transduction, and such features of hemopoietin receptors. From the above results, the NR8 gene was thought to encode a novel hemopoietin receptor.

Analysis of fragments amplified by the RACE method suggested the presence of a splice variant. As a result of nucleotide sequence analysis, this variant was revealed to be lacking approximately 150 bp including the above-described Pro-rich motif of NR8α. Moreover, as a result of comparing AC002303 sequence with NR8α, and carrying out analogy of exons/introns (Table 9), the above-described variant was thought to be deficient of the 5^{th} exon due to selective splicing.

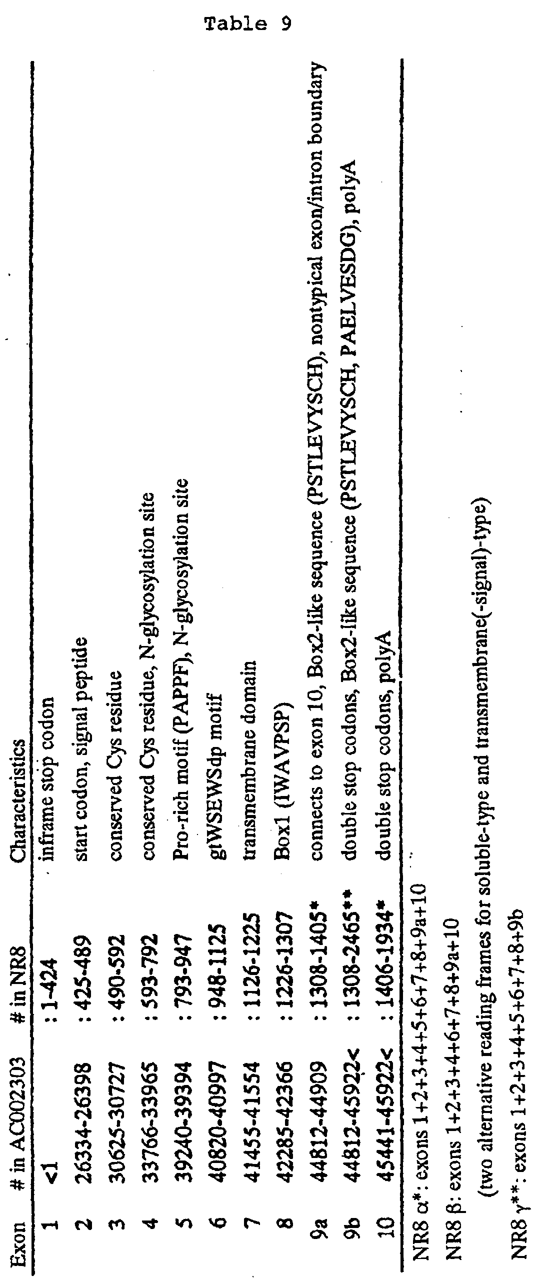

This variant (NR8β) can encode a soluble receptor in the truncated form by the joining of the 6^{th} exon directly to the 4^{th} exon and causing a frame shift. The boundary between the exons and the introns takes a consensus sequence in most cases, but the boundary between the 9^{th} exon (Exon 9a) and the 9^{th} intron is the only boundary that takes a different sequence from the consensus sequence (nag/gtgagt, etc.), being acc/acggag. The plasmid comprising NR8β cDNA (SEQ ID NO: 4) was named pGEM-NR8β, and *E.coli* comprising the plasmid has been internationally deposited at the National Institute of BioScience and Human-Technology, Agency of Industrial Science and Technology (1-3, Higashi 1-chome, Tsukuba-shi, Ibaraki-ken, Japan ) under the accession number FERM BP-6544 since October 9, 1998 according to the Budapest Treaty.

### Example 4: Northern blotting

In order to analyze the distribution and mode of NR8 gene expression in each human organ and human cancer cell lines, Northern blot analysis was done using the cDNA encoding the full-length NR8α protein prepared based on all the cDNA fragments obtained in Example 3 as a probe. The probe was prepared using Mega Prime Kit (Amersham, cat#RPN1607) by radiolabeling it with [α-³²P] dCTP (Amersham, cat#AA0005).

As Northern blots, Human Multiple Tissue Northern (MTN) Blot (Clontech #7760-1), Human MTN Blot IV (Clontech #7766-1), and Human Cancer Cell Line MTN Blot (Clontech #7757-1) were used. Express Hyb Hybridization Solution (Clontech #8015-2) was used for hybridization.

Hybridization conditions were: a prehybridization at 68°C for 30 min, followed by hybridization at 68°C for 14 hr. After washing under the following conditions, the blots were exposed to Imaging Plate (FUJI#BAS-III), and the gene expression of NR8 mRNA was detected by the Image Analyzer (FUJIX, BAS-2000 II). Washing conditions were: (1) 1x SSC/0.1% SDS, at room temperature for 5 min; (2) 1x SSC/0.1% SDS, at 50°C 30 min; and (3) 0.1x SSC/0.1% SDS, at 50°C 30 min.

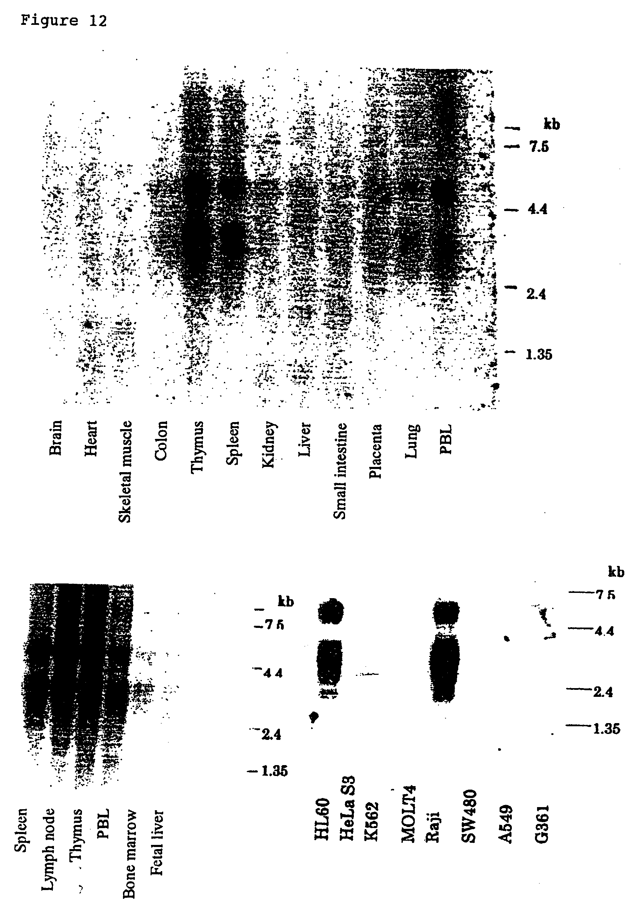

Fig. 12 shows the results of Northern blot analysis of NR8 expression in each organ. A total of three different-sized mRNA, one 5kb-sized and two 3 to 4kb sized, were detected in human adult lung, spleen, thymus, skeletal muscle, pancreas, small intestines, peripheral leucocytes, and uterus. A similar examination of various cell lines including hemopoietic cell lines showed similar sized bands in two cell lines, the promyeloid leukemic cell line HL60 and Burkett's lymphoma-derived Raji.

### Example 5: Plaque screening

Northern blot analysis of NR8 gene expression detected at least three types of specific mRNA bands with different sizes in each human organ and in each human cancer cell line for which NR8 gene expression was seen. However, the inventors had succeeded in isolating only two types of selective splicing variants, namely NR8α and NR8β genes, in the above-described Examples. Therefore, the inventors performed plaque screening with the objective of isolating the gene of the third selective splicing variant. Human Lymph Node (Clontech, cat#HL5000a) that showed a strong NR8 gene expression in the above-mentioned Northern analysis results, was used as the cDNA library. The probe used was NR8α cDNA fragment, which was radio-labeled by [α-³²P] dCTP (Amersham, cat#AA0005) using the Mega Prime Kit (Amersham, cat#RPN1607). Approximately 7.2 x 10⁵ plagues of Human Lymph Node cDNA Library were blotted onto a Hybond N (+) (Amersham, cat#RPN303B) charged nylon membrane to conduct primary screening. Rapid Hybridization Buffer (Amersham, cat#RPN1636) was used for the hybridization. Hybridization conditions were: a prehybridization at 65°C for 1 hr, followed by hybridization at 65°C for 14 hr. After washing under the conditions, (1) 1x SSC/0.1% SDS, at room temperature for 15 min; (2) 1x SSC/0.1% SDS, at 58°C 30 min; and (3) 0.1x SSC/0.1% SDS, at 58°C 30 min, the membrane was exposed to an X-ray film (Kodak, cat#165-1512) to detect NR8 positive plaques.

As a result, positive or pseudo-positive 16 independent clones were obtained. A similar secondary screening was done for the 16 clones obtained from the primary screening to successfully isolate plaques of NR8 positive 15 independent clones. The inserts of these 15 clones were amplified by PCR through a pair of primers located in both ends of the λgt10 vector cloning site. Advantage cDNA polymerase Mix (Clontech #8417-1) was used for the PCR reaction conducted using the Perkin Elmer Gene Amp PCR System 2400 Thermalcycler, under the following experiment conditions. Namely, 94°C for 4 min, 5 cycles of "94°C for 20 sec, 70°C for 4 min," 30 cycles of "94°C for 20 sec, 68°C for 4 min," 72°C for 4 min, and completed at 4°C.

Similar to above, the obtained PCR products were subcloned to pGEM-T Easy vector, and the nucleotide sequence of the inserts were determined using the BigDye Terminator Cycle Sequencing SF Ready Reaction Kit (ABI/Perkin Elmer#4303150), and analyzed by the ABI PRISM 377 DNA Sequencer. As a result, among the 15 clones obtained, at least two clones showed an insertion of 177 amino acids flanking the NR8α C terminus, and since this portion derives from the 9^{th} intron of the NR8 gene and is removed by splicing in NR8α, this 3^{rd} selective splicing variant was named NR8γ. The plasmid containing the NR8γ cDNA (SEQ ID NO: 8) was named pGEM-NR8γ, and *E.coli* containing the plasmid has been internationally deposited at the National Institute of BioScience and Human-Technology, Agency of Industrial Science and Technology (1-3, Higashi 1-chome, Tsukuba-shi, Ibaraki-ken, Japan) under the accession number FERM BP-6545 since October 9, 1998 according to the Budapest Treaty.

Among the 15 clones obtained here, four clones other than the two mentioned above were further selected, and their nucleotide sequences were analyzed. As a result, among the six clones selected, two clones had the NR8β nucleotide sequence, and all the remaining four clones had the NR8γ nucleotide sequence. Therefore, the six clones for which the nucleotide sequence was analyzed did not contain the NR8α sequence. The NR8γ cDNA clones for which the nucleotide sequences were determined included those having 3'-UTR (3UTR-2) in which a poly-A tail is added to the site elongated 483 bp from the 3'-UTR of NR8α obtained by the 3'-RACE method (3UTR-1), and those having 3'-UTR (3UTR-3) in which a poly-A tail is added to the site elongated 2397 bp from the 3'-UTR of NR8α. On the other hand, the two clones of NR8β for which the nucleotide sequence was decided above, both contained the nucleotide sequence of 3UTR-3. In Table 10 below, the 3' end non-translation region sequences contained in the cDNA clones thus far obtained are summarized. Also, the nucleotide sequences of 3UTR-1, 3UTR-2, and 3UTR-3 following the translation stop codon of NR8γ cDNA sequence are shown in SEQ ID NO: 23, SEQ ID NO: 24, and SEQ ID NO: 25, respectively.

Moreover, the nucleotide sequences of 3UTR-B1 and 3UTR-B3 following the translation stop codon of NR8β cDNA sequence are shown in SEQ ID NO: 26 and SEQ ID NO: 27, respectively.

**Table 10**

| NR8 cDNA clone | 3'-UTR sequence |
|---|---|
| NR8α | 3UTR-1 |
| NR8β | 3UTR-B1, 3UTR-B3 |
| NR8γ | 3UTR-1, 3UTR-2, 3UTR-3 |

The nucleotide sequences thus obtained revealed that the gene transcripts of NR8 can encode various different sizes not only due to the differences in selective splicing, but also due to the length of the 3' end non-translation region sequence. This may adequately explain the presence of various-sized transcripts detected by Northern blot analysis.

### Example 6: Ligand screening

### 6-1) Construction of NR8 chimeric receptor

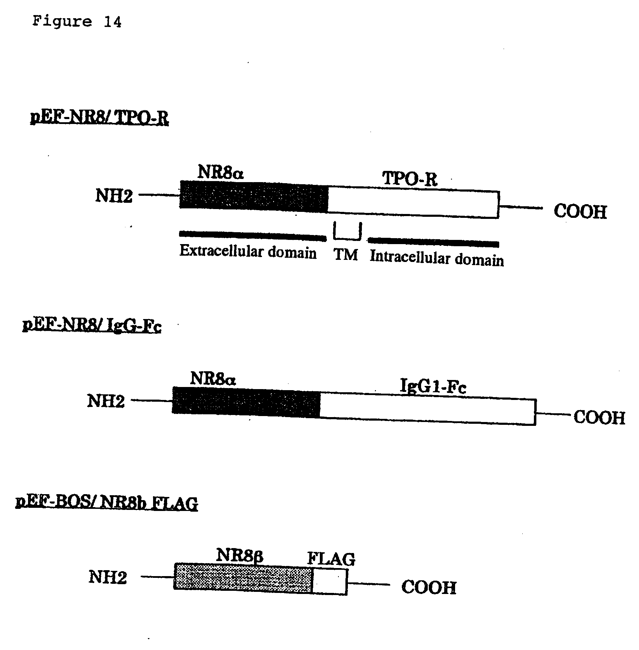

A screening system was constructed for searching a ligand that can specifically bind to NR8, namely, a novel hemopoietin. First, the cDNA sequence encoding the extracellular region of NR8α (the amino acid sequence of SEQ ID NO: 1; from the 1^{st} Met to the 228^{th} Glu) was amplified by PCR, and this DNA fragment was bound to DNA fragments encoding the transmembrane region and the intracellular region of a known hemopoietin receptor to prepare a fusion sequence encoding a chimeric receptor. As described above, there were several candidates for the partner, the known hemopoietin receptor, and among them, the human TPO receptor (Human MPL-P) was selected. Namely, after amplifying the DNA sequence encoding the intracellular region that includes the transmembrane region of the human TPO receptor by PCR, this sequence was bound to the cDNA sequence encoding the extracellular region of NR8α in frame, and inserted into a plasmid vector expressible in mammalian cells. The expression vector constructed was named pEF-NR8/TPO-R. A schematic diagram of the structure of the constructed NR8/TPO-R chimeric receptor is shown in Fig. 14, and the nucleotide sequence of the chimeric receptor and the expressible amino acid sequence encoded by it are shown in SEQ ID NOs: 13 and 14, respectively. Together with an expression vector pSV2bsr (Kaken Pharmaceutical Co., Ltd.) containing Blastcidin S resistant gene, the NR8/TPO-R chimeric receptor-expressing vector was introduced into the growth factor-dependent cell line Ba/F3, and forcedly expressed. Gene-introduced cells were selected by culturing with 8 µg/ml of Blastcidin S hydrochloride (Kaken Pharmaceutical Co., Ltd.) and IL-3. By transferring the obtained chimeric receptor-introduced cells to an IL-3-free medium, adding a material expected to contain a target ligand, and culturing, it is possible to conduct a screening that uses the fact that survival/proliferation will be possible only when a ligand that specifically binds to NR8 is present.

### 6-2) Preparation of NR8/IgG1-Fc soluble fusion protein

NR8/IgG1-Fc soluble fusion protein was prepared to be used for searching cell membrane-bound ligands, or the detection of soluble ligands through BIAcore (Pharmacia) and West-western blotting. A fusion sequence encoding the soluble fusion protein was prepared by binding a DNA fragment encoding the extracellular region of NR8α (amino acid sequence; from the 1^{st} Met to the 228^{th} Glu) prepared in 5-1) with the DNA fragment encoding the Fc region of human immunoglobulin IgG1 in frame. A schematic diagram of the structure of the soluble fusion protein encoding the NR8/IgG1-Fc is shown in Fig. 14, and the nucleotide sequence and the expressible amino acid sequence encoded by it in SEQ ID NOs: 15 and 16, respectively. This fusion gene fragment was inserted into a plasmid vector expressible in mammalian cells, and the constructed expression vector was named pEF-NR8/IgG1-Fc. If this pEF-NR8/IgG1-Fc is forcedly expressed in mammalian cells, and after selecting stable gene-introduced cells, the recombinant protein secreted into the culture supernatant can be purified by immunoprecipitation using anti-human IgG1-Fc antibody, or by affinity columns, etc.

### 6-3) Construction of an expression system of NR8β and purification of recombinant NR8β protein

The recombinant NR8β protein was prepared to be used for searching cell membrane-bound ligands, or the detection of soluble ligands using BIAcore (Pharmacia) or West-western-blotting. Using the amino acid coding sequence of NR8β cDNA, the stop codon was replaced by point mutation to a nucleotide sequence encoding an arbitrary amino acid residue, and then, was bound to the nucleotide sequence encoding the FLAG peptide in frame. This bound fragment was inserted into a plasmid vector expressible within mammalian cells, and the constructed expression vector was named pEF-BOS/NR8β FLAG. Fig. 14 shows a schematic diagram of the structure of the insert NR8β FLAG within the constructed expression vector. Moreover, the nucleotide sequence of NR8β FLAG and the expressible amino acid sequence encoded by it are shown in SEQ ID NOs: 17 and 18, respectively. If this pEF-BOS/NR8β FLAG is forcedly expressed in mammalian cells, and after selecting stable gene-introduced cells, the recombinant protein secreted into the culture supernatant can be immunoprecipitated using anti-FLAG peptide antibody, or may be purified by affinity columns, etc.

### Example 7: Isolation of mouse NR8 (mNR8) gene

### 7-1) The mouse homologous gene using human NR8 primers

Xenogeneic cross PCR cloning was isolated using the oligonucleotide primers, NR8-SN1 and NR8-SN2 (SEQ ID NOs: 9 and 10) at the sense side (downstream direction) and NR8-AS1 and NR8-AS2 (SEQ ID NOs: 11 and 12) at the antisense side (upstream direction), which were used for isolating full-length cDNA of human NR8. By combining the above-mentioned human NR8 primers, four types of primer sets can be constructed. Namely, using the combinations of "NR8-SN1 vs. NR8-AS1," "NR8-SN1 vs. NR8-AS2," "NR8-SN2 vs. NR8-AS1," and "NR8-SN2 vs. NR8-AS2," and a mouse brain cDNA library (Clontech #7450-1) and a mouse testis cDNA library (Clontech #7455-1) as templates, amplification of cross PCR products was expected. Advantage cDNA Polymerase Mix (Clontech #8417-1) was used for the PCR that was conducted under the conditions below using the Perkin Elmer Gene Amp PCR System 2400 Thermalcycler to amplify partial nucleotide sequence that could encode a mouse homologous gene of this receptor.

Namely, the cross PCR conditions were 94°C for 4 min, 5 cycles of "94°C for 20 sec, 72°C for 1 min," 5 cycles of "94°C for 20 sec,70°C for 1 min," 28 cycles of "94°C for 20 sec,68°C for 1 min," 72°C for 4 min, and completed at 4°C.

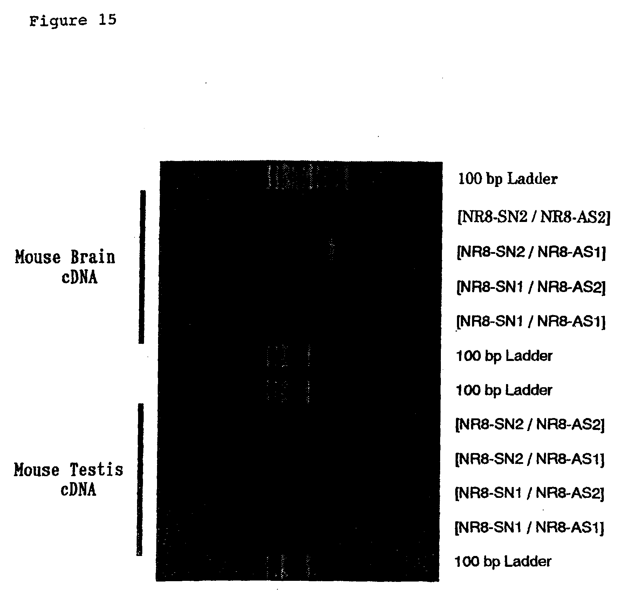

As a result, as shown in Fig. 15, an amplification of the cross PCR product was seen when any primer set was used. Also, a much clearer amplification product can be obtained when mouse brain cDNA was used as the template than when mouse testis cDNA was used.

### 7-2) Determination of the partial nucleotide sequence of the mouse homologous gene corresponding to NR8

Among the amplification products obtained in 7-1), mouse brain cDNA-derived product was subcloned to pGEM-T Easy vector (Promega #A1360), and the nucleotide sequence was determined. Namely, the PCR product was recombined into pGEM-T Easy vector by using T4 DNA ligase (Promega #A1360) at 4°C for 12 hr, and the resulting product was transfected into *E.coli* strain DH5α (Toyobo #DNA-903) to obtain the genetic recombinants of the PCR product and pGEM-T Easy vector. For the selection of genetic recombinant, Insert Check Ready Blue (Toyobo #PIK-201) was used. The nucleotide sequence was determined by using the BigDye Terminator Cycle Sequencing Ready Reaction Kit (ABI/Perkin Elmer #4303154), and sequence analysis was done by the ABI PRISM 377 DNA Sequencer. As a result of determining the nucleotide sequence of all inserts of eight independent clones of genetic recombinants, nucleotide sequences derived from the same transcript were obtained, and they were verified to be partial nucleotide sequences of mNR8. The obtained partial nucleotide sequence is shown in SEQ ID NO: 28.

### 7-3) Design of oligonucleotide primers specific to the mouse NR8 gene

Based on the partial nucleotide sequence of mNR8 obtained in 7-2), oligonucleotide primers specific to the mouse NR8 were designed. As shown in the sequence given below, mNR8-SN3 was synthesized in the sense side (downstream direction), and, mNR8-AS3 was synthesized in the antisense side (upstream direction). ABI's 394 DNA/RNA Synthesizer was used for primer synthesis, which was done under 5'-end trityl residue addition conditions. After that, the complete length of the synthesized product was purified by using an OPC column (ABI #400771). These primers contributed towards the 5'-RACE method and the 3'-RACE method described later on.
mNR8-SN3; 5'- TCC AGG CGC TCA GAT TAC GAA GAC CCT GCC -3' (SEQ ID NO: 29)
mNR8-AS3; 5'- ACT CCA GGT CCC CTG GTA GGA GGA GCC AGG -3' (SEQ ID NO: 30)

### 7-4) Cloning of cDNA corresponding to N terminus by the 5'-RACE method

To isolate full-length cDNA of mNR8, 5'-RACE PCR was performed using the NR8-AS2 primer (SEQ ID NO: 12) for the primary PCR, and the above-mentioned mNR8-AS3 primer (SEQ ID NO: 30) for secondary PCR. Mouse Brain Marathon-Ready cDNA Library (Clontech #7450-1) was used as the template, and Advantage cDNA Polymerase Mix for PCR experiment. As a result of conducting PCR under the following conditions using the Perkin Elmer Gene Amp PCR System 2400 Thermalcycler, PCR products of two different sizes were obtained.

Primary PCR conditions were 94°C for 4 min, 5 cycles of "94°C for 20 sec, 72°C for 100 sec," 5 cycles of "94°C for 20 sec,70°C for 100 sec," 28 cycles of "94°C for 20 sec,68°C for 100 sec," 72°C for 3 min, and completed at 4°C.

Secondary PCR conditions were 94°C for 4 min, 5 cycles of "94°C for 20 sec, 70°C for 100 sec," 25 cycles of "94°C for 20 sec,68°C for 100 sec," 72°C for 3 min, and completed at 4°C.

Both types of PCR products obtained were subcloned to pGEM-T Easy vector as described above, and the nucleotide sequences were determined. Namely, the PCR products were recombined into the pGEM-T Easy vector with T4 DNA ligase at 4°C for 12 hr, and the resulting product was transfected into *E.coli* strain DH5α to obtain the genetic recombinant between the PCR product and pGEM-T Easy vector. Also, as mentioned earlier, Insert Check Ready Blue was used for the selection of the genetic recombinant. For the determination of the nucleotide sequence, the BigDye Terminator Cycle Sequencing Ready Reaction Kit was used, and the nucleotide sequence was analyzed by the ABI PRISM 377 DNA Sequencer. The result of determining the nucleotide sequences of all inserts of eight independent clones of genetic recombinants suggests that they could be divided into two groups of four clones each by the base pair length and differences in the sequence. This difference of the products was caused by selective splicing, and both of the obtained sequences were verified to contain the sequence of full-length mNR8 cDNA clone corresponding to the N terminal sequence. The cDNA clone comprising the long ORF containing the exon encoding the Pro-rich region was named mNR8γ, and the cDNA clone encoding the short ORF that does not have the Pro-rich region was named mNR8β. These clones correspond to xenogeneic homologous genes of human NR8γ and human NR8β, respectively.

### 7-5) Cloning of cDNA corresponding to C terminus using the 3'-RACE method

To isolate full-length cDNA of mNR8, 3'-RACE PCR was performed using the NR8-SN1 primer (SEQ ID NO: 9) for the primary PCR, and the mNR8-SN3 primer (SEQ ID NO: 29) for secondary PCR. Mouse Brain Marathon-Ready cDNA Library was used as the template, and Advantage cDNA Polymerase Mix for PCR experiment. As a result of conducting PCR under the above-mentioned conditions using the Perkin Elmer Gene Amp PCR System 2400 Thermalcycler, a PCR product of a single size was obtained. The PCR product obtained was subcloned to pGEM-T Easy vector as before according to 7-2), and the nucleotide sequence was determined. As a result of determining the nucleotide sequences of all inserts of four independent clones of genetic recombinants, it was found to contain the sequence of full-length mNR8 cDNA corresponding to the C terminal sequence. By combining the resulting nucleotide sequence determined through this 3'-RACE PCR, and the nucleotide sequence of 5'-RACE PCR products determined in 7-4), the complete nucleotide sequences of the full-length of mNR8γ and mNR8β cDNA were finally determined. The determined mNR8γ cDNA nucleotide sequence and the amino acid sequence encoded by it are shown in SEQ ID NOs: 22 and 21, respectively. The determined mNR8β cDNA nucleotide sequence and the amino acid sequence encoded by it are shown in SEQ ID NOs: 20 and 19, respectively.

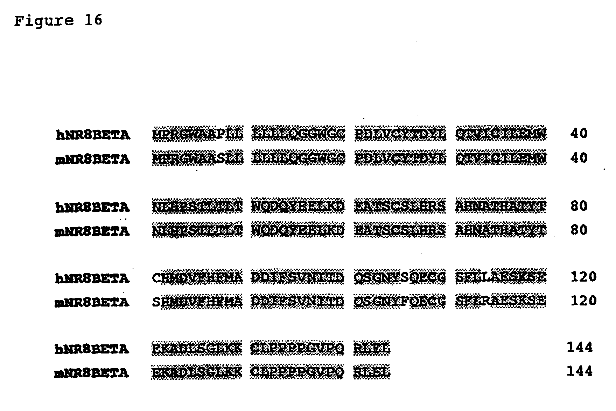

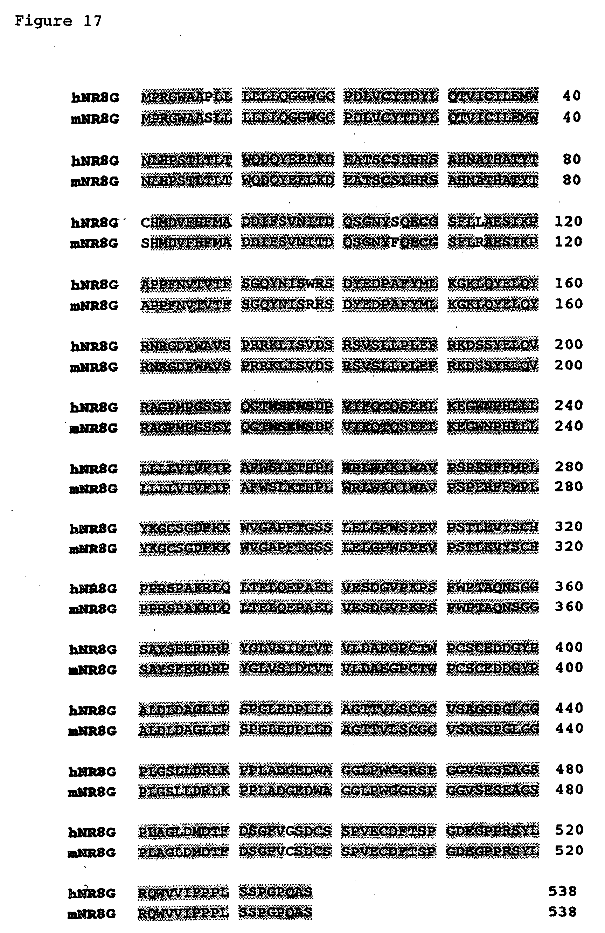

When the human and mouse NR8 amino acid sequences were compared, a high homology of 98.9% was seen for NR8γ, and the homology was 97.2% even for NR8β. This result strongly suggests the possibility that the same receptor gene has a vital functional responsibility that exceeds species. Fig. 16 shows a comparison between human and mouse NR8β amino acid sequences. Fig. 17 shows a comparison between human and mouse NR8γ amino acid sequences.

Both the full-length cDNAs of mNR8γ and mNR8β finally isolated were able to encode the transmembrane receptor protein comprising 538 amino acids, and the soluble receptor-like protein comprising 144 amino acids, respectively, through a selective splicing similar to human NR8. The structure below shows the characteristics of mNR8γ. First, it is presumed that from amino acid no. 1 Met to amino acid no. 19 Gly is a typical secretion signal sequence. Here, since an inframe stop codon exists in the minus 13 position from the 1^{st} Met, this Met residue is presumed to be the translation start codon. Next, from the 25^{th} Cys to the 35^{th} Cys residue is a typical ligand binding site sequence, and the 65^{th} and 109^{th} Cys residues also show the repetitive Cys residue structure conserved in other hemopoietin receptors as well. Next, the Pro-rich region is conserved by the Pro residues repeating at the 120^{th}, 122^{nd} and 123^{rd} positions. From the 214^{th} Trp to 218^{th} Ser residue is a typical WSXWS-Box (WS motif). Following these structural characteristics in the extracellular region, a typical transmembrane domain is seen in the 23 amino acids from the 233^{rd} Gly to the 255^{th} Leu. In the intracellular region that follows, the 271^{st} and 273^{rd} Pro residues are Box-1 consensus sequence (PXP motif) conserved in other hemopoietin receptor members, and these are thought to be deeply involved in signal transduction. Thus, mNR8γ adequately satisfies the characteristics of hemopoietin receptor members.

On the other hand, for mNR8β, among the structural characteristics for the above-mentioned extracellular region, the exon sequence encoding the Pro-rich region has been skipped by selective splicing, and directly joins the next exon encoding the WS motif. However, the WSXWS-Box sequence has been excluded from the reading frame by frame shift, and after coding up to 144^{th}Leu, the translation frame completed the next stop codon. Thus, a soluble hemopoietin receptor-like protein that does not have a transmembrane domain is encoded.

### Example 8: Expression analysis of mouse NR8 gene

### 8-1) Analysis of mouse NR8 gene expression by the RT-PCR method

To analyze the distribution and mode of NR8 gene expression in each mouse organ, the mRNA was detected by RT-PCR analysis. As primers for this RT-PCR analysis, NR8-SN1 primer (SEQ ID NO: 9) was used as the sense side (downstream direction) primer, and NR8-AS1 primer was used as the antisense side (upstream direction) primer. Mouse Multiple Tissue cDNA Panel (Clontech #K1423-1) was used as the template. Advantage cDNA Polymerase Mix (Clontech #8417-1) and the Perkin Elmer Gene Amp PCR System 2400 Thermalcycler were used for PCR. The target genes were amplified by the PCR reaction under the cycle condition given below.

PCR conditions were 94°C for 4 min, 5 cycles of "94°C for 20 sec, 72°C for 1 min," 5 cycles of "94°C for 20 sec, 70°C for 1 min," 24 cycles of "94°C for 20 sec, 68°C for 1 min," 72°C for 3 min, and completed at 4°C.

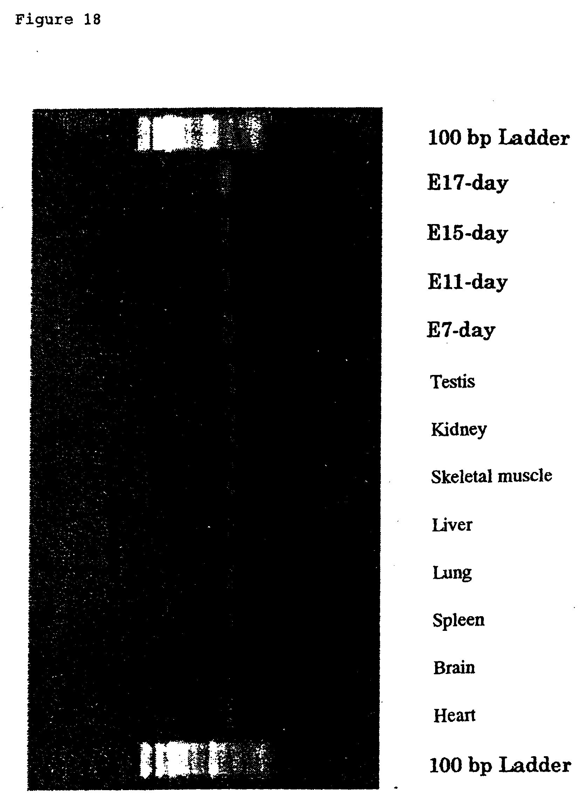

The results of RT-PCR are shown in Fig. 18. The NR8 gene was strongly detected in the testis and day 17 embryo, and a constitutive gene expression was seen in all mouse organs and in all mouse tissue-derived mRNA analyzed. By detecting the expression of the house keeping gene G3PDH under the above-mentioned PCR conditions using the mouse G3PDH primer for all the templates used in the analysis, it has been verified beforehand that the number of copies of template mRNA has been normalized (standardized) between samples. The detected RT-PCR product size herein was 320 bp, and this coincides with the size calculated by the determined nucleotide sequence. Therefore, it was thought to be the product of the mouse NR8 specific PCR amplification reaction. To further verify this, the PCR product amplified in the day 17 embryo was subcloned to pGEM-T Easy vector according to 7-2), and the nucleotide sequence was analyzed. The result verified that the PCR product could be a partial nucleotide sequence of mouse NR8, and the possibility that it might be the product of a non-specific PCR amplification was denied.

### 8-2) Analysis of mouse NR8 gene expression by Northern blotting

In order to analyze NR8 gene expression in each mouse organ, and with the objective of identifying the NR8 transcription size, gene expression analysis by the Northern blotting method was conducted. Mouse Multiple Tissue Northern Blot (Clontech #7762-1) was used as the blot. Among the 5'-RACE products obtained in 7-4), the mNR8β cDNA fragment was used as the probe. The probe was radiolabeled with [α-³²P] dCTP (Amersham, cat#AA0005) using Mega Prime Kit (Amersham, cat#RPN1607). Express Hyb Hybridization Solution (Clontech #8015-2) was used for hybridization. After a prehybridization at 68°C for 30 min, the heat-denatured labeled probe was added, and hybridization was conducted at 68°C for 16 hr. After washing under the following conditions, the blot was exposed to Imaging Plate (FUJI #BAS-III), and a mouse NR8 specific signal was detected by the Image Analyzer (FUJIX, BAS-2000 II).

Washing conditions were: (1) 1x SSC/0.1% SDS, at room temperature for 5 min; (2) 1x SSC/0.1% SDS, at 50°C 30 min; and (3) 0.5x SSC/0.1% SDS, at 50°C 30 min.

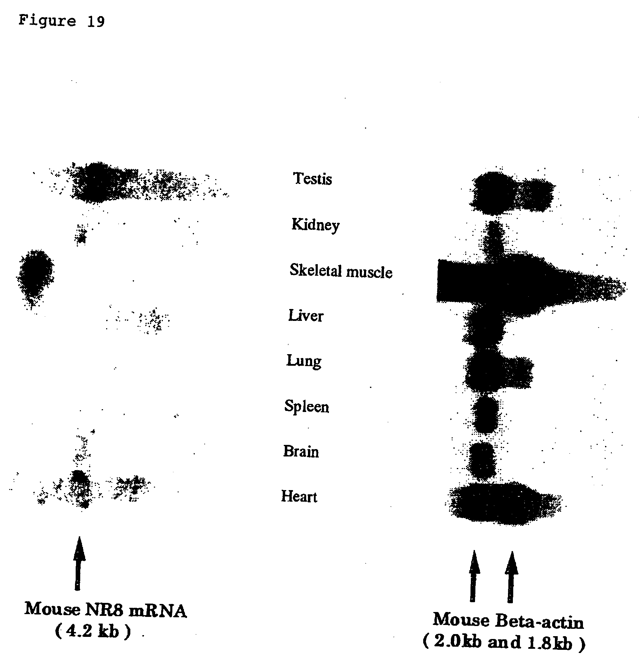

As a result, as shown in Fig. 19, a strong expression was seen in the mouse testis only, and no gene expression of the same gene was detected in other organs. Here, there is a difference between the results of RT-PCR analysis and Northern blot analysis. Since the detection sensitivity of the Northern method is much lower than RT-PCR, it is thought that mRNA with low expression levels could not be detected. However, results of both analyses coincide in the point that a strong gene expression was detected in the testis. Also, the size of the detected transcript was about 4.2 kb.

Although there was a deviation of the expression levels in each mouse organ analyzed by the Northern method and RT-PCR, the gene expression was widely distributed, being detectable in all the organs analyzed especially when using RT-PCR. This result contrasts with the human NR8 gene in which the expression was strong only in immunocompetent tissues, hemopoietic tissues, and specific leukemic cell lines, and the significance of this expression is extremely interesting. This means namely the possibilities that in mouse, the NR8 molecule not only is involved in systemic hemopoietic functions, or in immunological responses, and hemopoiesis, but also may be involved in various physiological regulatory mechanisms of the body. Namely, its ligand may be able to function as a hormone-like factor.

### Example 9: Isolation of the NR8 mouse genomic gene by plaque screening

The present inventors analyzed the genomic structure of mouse NR8 gene and performed plaque hybridization against the mouse genomic DNA library. 129SVJ strain Genomic DNA (Stratagene #946313) constructed in Lambda FIX II was used as the library. This genomic library of approximately 5.0 x 10⁵ plagues was developed and blotted to a Hybond N(+)(Amersham #RPN303B) charged nylon membrane to perform primary screening. NR8β cDNA fragment of 5'-RACE products obtained in 7-4) was used as the probe. The probe was radiolabeled with [α-³²P] dCTP prepared as above-mentioned in 8-2) using the Mega Prime Kit. Express Hyb Hybridization Solution was used for hybridization, and after a prehybridization at 65°C for 30 min, a heat-denatured labeled probe was added, and hybridization was done at 65°C for 16 hr. After washing under the following conditions, the membrane was exposed to an X-ray film (Kodak, cat#165-1512) to detect mouse NR8 positive plaques.

Washing conditions were: (1) 1x SSC/0.1% SDS, at room temperature for 5 min; (2) 1x SSC/0.1% SDS, at 58°C 30 min; and (3) 0.5x SSC/0.1% SDS, at 58°C 30 min.

As a result, positive, or pseudo-positive 16 independent clones were obtained. When a secondary screening was similarly conducted against these 16 clones obtained by the primary screening, the inventors succeeded in isolating NR8 positive, nine independent plaque clones.

### Industrial Applicability

The present invention provides a novel hemopoietin receptor protein "NR8," and the encoding DNA. The present invention also provides, a vector into which the DNA has been inserted, a transformant harboring the DNA, and a method of producing a recombinant protein using the transformant. It also provides a method of screening a compound or a natural ligand that binds to the protein. The NR8 protein of the invention is thought to be related to hemopoiesis, and therefore, is useful in analyzing hemopoietic functions. The protein would also be applied in the diagnosis and treatment of hemopoiesis-associated diseases.

Since the expression of mouse NR8 gene was widely distributed in mouse organs, mouse NR8 protein would be involved in various physiological regulatory mechanisms of the body, including the above-mentioned hemopoiesis. Furthermore, by using mouse NR8 protein, it is possible to isolate first the mouse NR8 ligand, and next, the human homologue of the NR8 ligand using the conserved structure of the mouse NR8 ligand. Specifically, after determining the nucleotide sequence of mouse NR8 ligand cDNA, an oligonucleotide primer is designed on this sequence, and using this to conduct cross PCR using the human-derived cDNA library as the template, human NR8 ligand cDNA can be obtained. Alternatively, human NR8 ligand cDNA can be obtained by conducting cross hybridization against human-derived cDNA library using mouse NR8 ligand cDNA as the probe. It is also possible to analyze biological function of the NR8 receptor protein by creating a mouse NR8 gene-deficient mouse using the mouse NR8 gene.

## Claims

1. A protein comprising the amino acid sequence from the 1^{st} amino acid Met to the 361^{st} amino acid Ser of SEQ ID NO: 1, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 361^{st} amino acid Ser of SEQ ID NO: 1.

2. A protein comprising the amino acid sequence from the 1^{st} amino acid Met to the 144^{th} amino acid Leu of SEQ ID NO: 3, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 144^{th} amino acid Leu of SEQ ID NO: 3.

3. A protein comprising the amino acid sequence from the 1^{st} amino acid Met to the 237^{th} amino acid Ser of SEQ ID NO: 5, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 237^{th} amino acid Ser of SEQ ID NO: 5.

4. A protein comprising the amino acid sequence from the 1^{st} amino acid Met to the 538^{th} amino acid Ser of SEQ ID NO: 7, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 538^{th} amino acid Ser of SEQ ID NO: 7.

5. A protein comprising the amino acid sequence from the 1^{st} amino acid Met to the 144^{th} amino acid Leu of SEQ ID NO: 19, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 144^{th} amino acid Leu of SEQ ID NO: 19.

6. A protein comprising the amino acid sequence from the 1^{st} amino acid Met to the 538^{th} amino acid Ser of SEQ ID NO: 21, or a protein comprising a modified amino acid sequence of said amino acid sequence in which one or more amino acids have been deleted, added and/or substituted with another amino acid, and being functionally equivalent to the protein comprising the amino acid sequence from the 1^{st} amino acid Met to the 538^{th} amino acid Ser of SEQ ID NO: 21.

7. A protein encoded by a DNA hybridizing to a DNA comprising the nucleotide sequence of SEQ ID NO: 2, said protein being functionally equivalent to a protein comprising the amino acid sequence from the 1^{st} amino acid Met to the 361^{st} amino acid Ser of SEQ ID NO: 1.

8. A protein encoded by a DNA hybridizing to a DNA comprising the nucleotide sequence of SEQ ID NO: 4, said protein being functionally equivalent to a protein comprising the amino acid sequence from the 1^{st} amino acid Met to the 144^{th} amino acid Leu of SEQ ID NO: 3.

9. A protein encoded by a DNA hybridizing to a DNA comprising the nucleotide sequence of SEQ ID NO: 6, which is functionally equivalent to a protein comprising the amino acid sequence from the 1^{st} amino acid Met to the 237^{th} amino acid Ser of SEQ ID NO: 5.

10. A protein encoded by a DNA hybridizing to a DNA comprising the nucleotide sequence of SEQ ID NO: 8, said protein being functionally equivalent to a protein comprising the amino acid sequence from the 1^{st} amino acid Met to the 538^{th} amino acid Ser of SEQ ID NO: 7.

11. A protein encoded by a DNA hybridizing to a DNA comprising the nucleotide sequence of SEQ ID NO: 20, said protein being functionally equivalent to a protein comprising the amino acid sequence from the 1^{st} amino acid Met to the 144^{th} amino acid Leu of SEQ ID NO: 19.

12. A protein encoded by a DNA hybridizing to a DNA comprising the nucleotide sequence of SEQ ID NO: 22, said protein being functionally equivalent to a protein comprising the amino acid sequence from the 1^{st} amino acid Met to the 538^{th} amino acid Ser of SEQ ID NO: 21.

13. A fusion protein comprising the protein of any one of claims 1 to 12 and another peptide or polypeptide.

14. A DNA encoding the protein of any one of claims 1 to 13.

15. A vector comprising the DNA of claim 14.

16. A transformant harboring the DNA of claim 14 in an expressible manner.

17. A method of producing the protein of any one of claims 1 to 13, comprising the step of culturing the transformant of claim 16.

18. A method of screening a compound that binds to the protein of any one of claims 1 to 13 comprising the steps of:
(a) contacting a test sample with the protein of any one of claims 1 to 13; and
(b) selecting a compound that comprises an activity to bind to the protein of any one of claims 1 to 13.

19. An antibody that specifically binds to the protein of any one of claims 1 to 12.

20. A method of detecting or measuring the protein of any one of claims 1 to 13 comprising the steps of contacting a test sample presumed to contain said protein with the antibody of claim 19, and detecting or measuring the formation of the immune complex between the antibody and the protein.

21. A DNA specifically hybridizing to a DNA comprising a nucleotide sequence of any one of SEQ ID NOs: 2, 4, 6, 8, 20, and 22 to 27 comprising at least 15 nucleotides, and comprising at least 15 nucleotides.
